# EUROPÄISCHE PATENTSCHRIFT

(11) **EP 1 817 806 B1**
(45) Veröffentlichungstag und Bekanntmachung des Hinweises auf die Patenterteilung: **30.07.2014**
(21) Anmeldenummer: 05810164.3
(22) Anmeldetag: 25.11.2005
(51) Int. Cl.: C07D 417/04, H01L 51/30

(54) **PHENOTHIAZINE-S-OXIDE UND S,S-DIOXIDE SOWIE PHENOXAZINE ALS Loch- und Excitonblocker in OLEDs**
PHENOTHAZINES-S-OXIDES AND S,S-DIOXIDES AS hole and excitonblockers for OLEDs
UTILISATION DE PHENOTHAZINE-S-OXYDE ET S,S-DIOXIDE COMME BLOQUEURS DE trou et d'exciton DE DIODES OLEDS

(30) Priorität: 25.11.2004 DE 102004057086
(43) Veröffentlichungstag der Anmeldung: 15.08.2007
(73) Patentinhaber: BASF SE, 67056 Ludwigshafen (DE)
(72) Erfinder: GESSNER, Thomas, 69120 Heidelberg (DE); SCHMIDT, Hans-Werner, 95444 Bayreuth (DE); THELAKKAT, Mukundan, 95445 Bayreuth (DE); BÄTE, Markus, 95508 Kulmain (DE)
(86) Internationale Anmeldenummer: PCT/EP2005/012647
(87) Internationale Veröffentlichungsnummer: WO 2006/056465

(56) Entgegenhaltungen:
- EP-A- 0 535 672
- EP-A- 1 400 578
- DE-A1- 10 143 249
- DE-A1- 19 846 767
- DE-A1-102004 020 046
- GB-A- 2 083 488
- JP-B- 7 120 056
- US-A- 5 942 615
- US-A1- 2004 127 666
- PATENT ABSTRACTS OF JAPAN Bd. 2003, Nr. 05, 12. Mai 2003 (2003-05-12) -& JP 2003 007466 A (KONICA CORP), 10. Januar 2003 (2003-01-10)
- PATENT ABSTRACTS OF JAPAN Bd. 1995, Nr. 07, 31. August 1995 (1995-08-31) -& JP 07 109449 A (TOYO INK MFG CO LTD), 25. April 1995 (1995-04-25)

## Beschreibung

Die vorliegende Erfindung betrifft die Verwendung von Phenothiazin-S-oxid- oder Phenothiazin-S,S-dioxid-Derivaten als Emittersubstanzen oder Loch- und Excitonenblocker in organischen Leuchtdioden, eine organische Leuchtdiode umfassend eine Licht-emittierende Schicht, wobei die Licht-emittierende Schicht mindestens ein Phenoxazin-, Phenothiazin-, Phenothiazin-S-oxid- oder Phenothiazin-S,S-dioxid-Derivat als Emittersubstanz enthält, sowie eine Licht-emittierende Schicht enthaltend oder bestehend aus dem vorstehend genannten Phenoxazin-, Phenothiazin-, Phenothiazin-S-oxid- oder Phenothiazin-S,S-dioxid-Derivat als Emittersubstanz eine Loch- und Excitonenblockschicht enthaltend oder bestehend aus dem vorstehend genannten Phenoxazin-, Phenothiazin-, Phenothiazin-S-oxid- oder Phenothiazin-S,S-dioxid-Derivat, sowie eine Vorrichtung, die eine erfindungsgemäße organische Leuchtdiode enthält. Des Weiteren betrifft die vorliegende Erfindung spezielle Phenothiazin-S,S-dioxid-Derivate, Phenothiazin-S-oxid-Derivate und Phenothiazin-Derivate und deren Herstellungsverfahren, sowie deren Verwendung in organischen Leuchtdioden.

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigen Stromverbrauchs eignen sich Vorrichtungen enthaltend OLEDs insbesondere für mobile Anwendungen, zum Beispiel für Anwendungen in Handys, Laptops, usw.

Es wurden zahlreiche Materialien vorgeschlagen, die bei Anregung durch elektrischen Strom Licht emittieren.

Phenoxazin- und Phenothiazin-Derivate werden gemäß dem Stand der Technik im Allgemeinen als Ladungstransportmaterialien eingesetzt.

So betrifft EP-A 0 517 542 aromatische Aminoverbindungen, die sich durch eine gute Hitzestabilität auszeichnen und unter anderem eine Phenothiazin-Einheit aufweisen können. Diese aromatischen Aminoverbindungen werden als Lochtransportmaterialien in OLEDs eingesetzt.

EP-A 0 562 883 betrifft ebenfalls Lochtransportmaterialien, die in OLEDs eingesetzt werden und die eine hohe Hitzestabilität aufweisen. Als Lochtransportmaterialien werden tris-Phenothiazinyl-triphenylamin-Derivate oder tris-Phenoxazinyl-triphenylamin-Derivate eingesetzt.

DE-A 101 43 249 betrifft ein Verfahren zur Herstellung von konjugierten Oligo- und Polyphenothiazinen und deren Verwendung als Lochleiter in organischen Leuchtdioden und Feldeffekttransistoren. Die Oligo- und Polyphenothiazine werden mittels Kreuzkupplung von funktionalisierten Phenothiazin-Derivaten hergestellt.

In EP-A 0 535 672 ist ein elektrofotografischer Fotorezeptor offenbart, der in seiner fotosensitiven Schicht ein organisches leitendes Material enthält. Als organisches leitendes Material sind unter anderem Verbindungen geeignet, die Phenothiazin-Struktureinheiten aufweisen.

US 5,942,615 sowie JP-A 11-158165 betreffen Phenothiazin und Phenoxazin-Derivate, ein Ladungstransportmaterial, das diese Derivate enthält, sowie einen elektrofotografischen Fotorezeptor, der das offenbarte Ladungstransportmaterial enthält. Bei den Phenothiazin- oder Phenoxazin-Derivaten handelt es sich um Derivate der folgenden Formel: 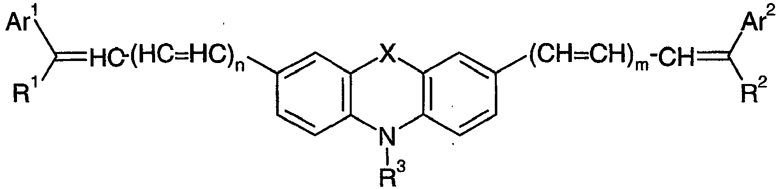 worin Ar¹ und Ar² Arylgruppen sind, R¹ und R² H, Niederalkyl oder Aryl , R³ Niederalkyl, ein alicyclischer Kohlenwasserstoffrest mit 5 bis 7 Kohlenstoffatomen, Aryl oder Aralkyl bedeuten, X S oder O und m und n 0 oder 1 bedeuten. Bezüglich einer Lumineszenz, insbesondere einer Elektrolumineszenz, der vorstehend genannten Verbindungen enthalten sowohl US 5,942,615 als auch JP-A 11-158165 keine Information.

Im Stand der Technik sind nur wenige sehr spezielle Phenothiazin- und Phenoxazin-Derivate bekannt, die als lumineszierende Materialien in der Licht-emittierenden Schicht eines OLEDs eingesetzt werden.

So betrifft JP-A 2003-007466 ein OLED, das eine lange Lebensdauer und hohe Leuchtdichte aufweist, das als lumineszierendes Material ein Polymer enthält, das Wiederholungseinheiten auf Basis von Phenothiazin- oder Phenoxazin-Derivaten aufweist.

JP-A 2000-328052 betrifft lumineszierendes Material, das Licht im gelben bis roten Bereich des elektromagnetischen Spektrums emittiert und aus einer monocyclischen oder kondensierten polycyclischen Verbindung aufgebaut ist, die zwei spezielle Substituenten aufweist. Bei diesen speziellen Substituenten handelt es sich um Substituenten der folgenden Formel:

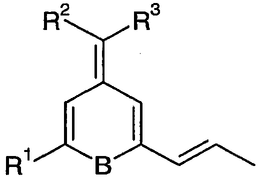

Darin bedeuten
- B: S oder O,
- R¹: H, Alkyl oder Aryl und
- R², R³: unabhängig voneinander ausgewählt aus H, CN, Halogen, Alkylcarbonyl und Alkoxycarbonyl, bevorzugt sind R² und R³ CN.

Als kondensierte polycyclische Verbindungen werden Phenothiazin- und Phenoxazin-Derivate genannt.

KR 2003-0029394 betrifft rote Leuchtstoffe, die zur organischen Elektrolumineszenz geeignet sind. Diese Leuchtstoffe weisen eine Phenocyanidin-Gruppe auf, die gute Lochtransporteigenschaften besitzt, und einen Anthracenylrest, der ein gutes Elektronentransportvermögen besitzt. Je nach Substitutionsmuster der Leuchtstoffe können diese nicht nur Lumineszenz im gelben und roten, sondern auch im grünen Bereich des elektromagnetischen Spektrums zeigen. Diese speziellen Leuchtstoffe weisen eine der folgenden Formeln auf

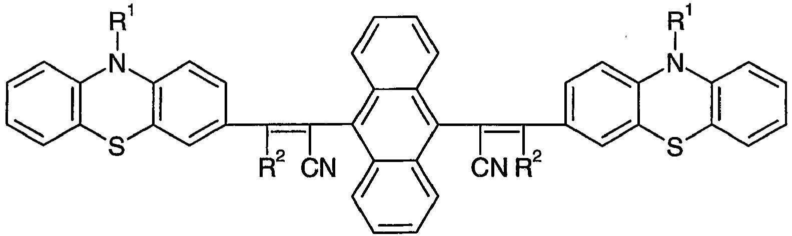

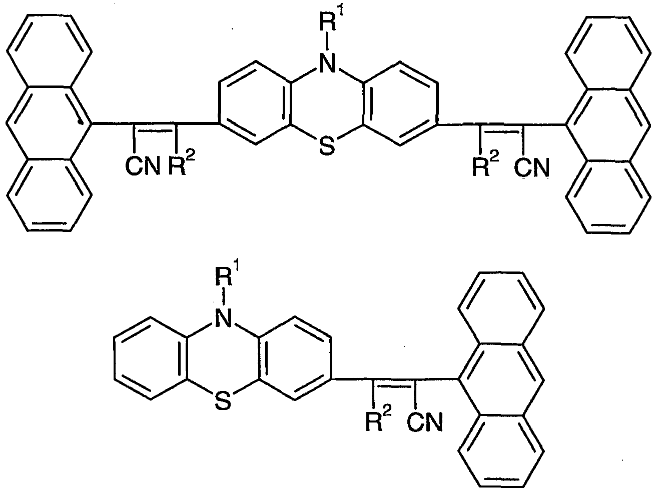

Die Reste R¹ und R² können H, Aryl, Heteroaryl, Halogen oder gesättigte bzw. ungesättigte Kohlenwasserstoffe sein. Als Besonderheit dieser Verbindungen wird hervorgehoben, dass diese nicht nur Lichtemissionseigenschaften, sondern auch Lochtransport-Eigenschaften und Elektronentransport-Eigenschaften aufgrund ihres besonderen Substitutionsmusters aufweisen.

JP-A 2004-075750 betrifft Phenoxazin-Derivate der Formel 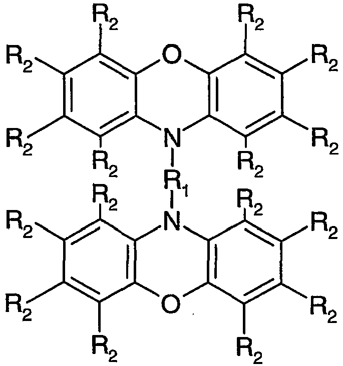 worin R₁ eine aromatische oder aliphatische Verbindungsgruppe ist und R₂ eine Alkyl-, Alkenyl-, Alkylether-, Alkoxy-, Amino-, Aryl- oder Aryloxygruppe ist. Die Phenoxazin-Derivate werden in der Licht emittierenden Schicht eines OLED als fluoreszierende Substanzen eingesetzt.

In US 2004/127666 A1 sind Fluoren- und tricyclische Amineinheiten enthaltende Polymere und deren Einsatz als Licht-emittierende Materialien in OLEDs offenbart (Seite 1, Formel 1, Abschnitt [0010] und Seite 6, Abschnitt [0071], Zeilen 1 und 2 des Abschnitts sowie Abschnitt [0075], Zeilen 3 bis 6 des Abschnitts). Die Polymere weisen dabei Einheiten auf, worin eine tricyclische Arylamineinheit in 3- und 7-Position mit einem Fluorenylrest substituiert ist (siehe Formel 1 auf Seite 1). Die in US 2004/127666 A1 eingesetzten Polymere weisen gemäß Abschnitt [0050] bevorzugt mittlere Molekulargewichte von etwa 10.000 Dalton oder mehr auf. Polymere der Formel I, die niedrigere Molekulargewichte als 10.000 Dalton aufweisen (siehe Abschnitt [0050]), sind in US 2004/127666 A1 nicht offenbart.

Gemäß JP 07-109449 A sind Verbindungen der folgenden Formel 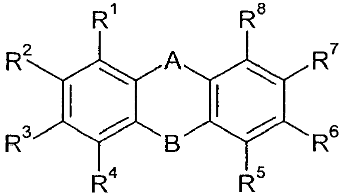 und deren Einsatz in OLEDs offenbart. Gemäß JP 07-109449 A ist es zwingend, dass mindestens einer der Reste R1 oder R4 sowie R5 oder R8 jeweils eine Amino- oder disubstiuierte Aminogruppe ist. Die Verbindungen gemäß JP 07-109449 A werden bevorzugt in der Licht-emittierenden Schicht oder in einer Lochinjektionsschicht einer OLED eingesetzt.

Aufgabe der vorliegenden Anmeldung ist es, weitere Emittersubstanzen, insbesondere blau emittierende Substanzen, für den Einsatz in OLEDs bereitzustellen, die leicht zugänglich sind und gute Leuchtdichten und Quantenausbeuten bei Einsatz in OLEDs aufweisen, und die sich bevorzugt dadurch auszeichnen, dass sie in Substanz in der Licht-emittierenden Schicht in OLEDs - ohne Einbindung in ein Matrixmaterial - eingesetzt werden können. Eine weitere Aufgabe der vorliegenden Anmeldung ist es, Substanzen bereitzustellen, die als Loch- und Excitonenblocker geeignet sind.

Diese Aufgaben werden gelöst durch die Verwendung von Verbindungen der Formel I 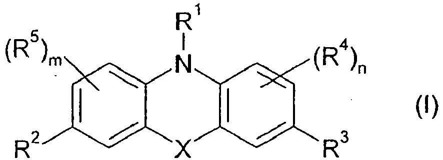 worin bedeuten:
- X: SO₂, SO, bevorzugt SO₂;
- R¹: H, Alkyl, Aryl, Heteroaryl, bevorzugt H, Methyl, Ethyl, unsubstituiertes Phenyl oder 4-Alkoxyphenyl; oder 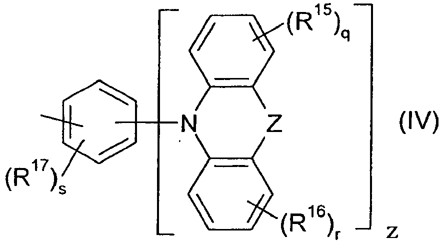 oder 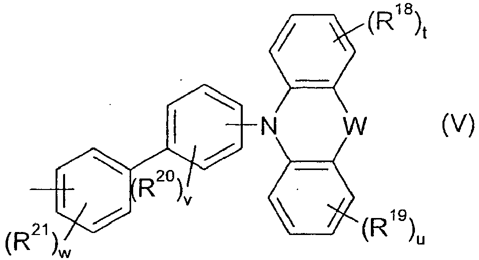
- R², R³: unabhängig voneinander H, Alkyl, Aryl oder COR³¹, wobei R² und R³ nicht gleichzeitig H bedeuten, bevorzugt H oder Aryl, besonders bevorzugt H, unsubstituiertes Phenyl, 1-Naphthyl oder 2-Naphthyl,
oder
mindestens einer der Reste R² oder R³ ist ein Rest der Formel II 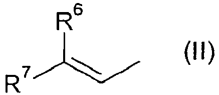 wobei der weitere Rest R² oder R³ die gleiche Bedeutung aufweist oder H, Alkyl, Aryl, bevorzugt H oder Aryl, ganz besonders bevorzugt H oder unsubstituiertes Phenyl bedeutet, bevorzugt ist der weitere Rest R² oder R³ ebenfalls ein Rest der Formel (II), oder
einer der Reste R² oder R³ ist ein Rest der Formel III 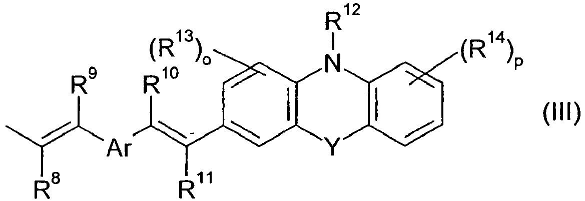 wobei der weitere Rest R² oder R³ H, Alkyl, Aryl, bevorzugt H oder Aryl, ganz besonders bevorzugt H oder unsubstituiertes Phenyl bedeutet;
- R⁴, R⁵, R¹³, R¹⁴, R¹⁵, R¹⁶, R¹⁸, R¹⁹, R²⁰, R²¹: unabhängig voneinander Alkyl, Aryl, Alkenyl, Alkinyl oder Alkylaryl, bevorzugt Alkyl oder Aryl;
- R¹⁷: Halogen, Alkoxy, Alkyl, Aryl, Alkenyl, Alkinyl oder Alkylaryl, bevorzugt Fluor, Alkoxy, Alkyl oder Aryl;
- m, n: 0, 1, 2 oder 3, bevorzugt 0 oder 1, besonders bevorzugt 0;
- o, p, q, r, s, t, u, v, w: 0, 1, 2, 3 oder 4, bevorzugt 0 oder 1, besonders bevorzugt 0;
- z: 1 oder 2, bevorzugt 1;
- R⁶, R⁷: unabhängig voneinander H, Alkyl, Aryl, Alkenyl, Alkinyl, Alkylaryl, Alkoxy oder Aryloxy, bevorzugt H, Alkyl oder Aryl, besonders bevorzugt Alkyl oder Aryl, ganz besonders bevorzugt Methyl oder unsubstituiertes Phenyl;
- R⁸, R⁹, R¹⁰, R¹¹: unabhängig voneinander H, Alkyl, Aryl, Alkenyl, Alkinyl oder Alkylaryl, bevorzugt H, Alkyl oder Aryl, besonders bevorzugt H oder Alkyl, ganz besonders bevorzugt H;
- R¹²: H, Alkyl oder Aryl, bevorzugt H, Methyl, Ethyl, unsubstituiertes Phenyl oder 4-Alkoxyphenyl;
- Y, Z,: W S, SO₂, O, SO, bevorzugt S oder SO₂; und
- Ar: Arylen, bevorzugt Phenylen, Naphthylen oder Biphenylen;
- R³¹: H, Alkyl, Aryl;
als Loch- und Excitionenblocker, in organischen Leuchtdioden.

Die erfindungsgemäß eingesetzten Verbindungen der Formel I sind leicht zugänglich.

Die Verbindungen der Formel I können als Loch- und Excitionenblocker in organischen Leuchtdioden verwendet werden.

Die Alkyl-, Alkenyl- und Alkinylreste sowie die Alkylreste der Alkoxygruppen gemäß der vorliegenden Anmeldung können sowohl geradkettig, verzweigt, cyclisch und/oder optional substituiert sein mit Substituenten ausgewählt aus der Gruppe bestehend aus Aryl, Alkoxy, NR²²R²³ und Halogen, wobei R²² und R²³ unabhängig voneinander H, Alkyl, Aryl, bevorzugt Aryl, bedeuten oder R²² und R²³ bilden gemeinsam einen cyclischen Rest, bevorzugt einen 5- oder 6-gliedrigen Rest.

Geeignete Halogensubstituenten sind Fluor, Chlor, Brom und Jod, bevorzugt Fluor, Chlor und Brom, besonders bevorzugt Fluor und Chlor.

Geeignete Arylsubstituenten sind nachstehend genannt. Bevorzugt sind die Alkyl-, Alkenyl- und Alkinylreste unsubstituiert.

Beispiele für geeignete Alkylgruppen sind Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst. Bevorzugte Alkylgruppen sind Methyl und Ethyl.

Unter cyclischen Alkylresten (Cycloalkylreste) sind Cycloalkyl sowie Cycloalkyl-alkyl-(Alkyl, das wiederum mit Cycloalkyl substituiert ist) zu verstehen, wobei Cycloalkyl mindestens drei Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantyl. Die Cycloalkylreste können unsubstituiert oder gegebenenfalls mit einem oder mehreren weiteren Resten substituiert sein, wie vorstehend bei den Alkylresten beispielhaft aufgeführt wurde.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl.

Geeignete Alkoxygruppen weisen die allgemeine Formel -OR²⁴ auf. R²⁴ stellt dabei einen Alkylrest dar, wie er vorstehend definiert wurde. Geeignete Alkoxysubstituenten sind ausgewählt aus der Gruppe bestehend aus OCH₃, OC₂H₅, OC₃H₇ und OC₄H₉. Dabei sind unter der Bezeichnung C₃H₇ und C₄H₉ sowohl die n-Isomere als auch verzweigte Isomere wie Isopropyl, Isobutyl, sec-Butyl und tert-Butyl umfasst. Besonders bevorzugt sind Methoxy und Ethoxy. Des Weiteren kann R²⁴ ein Aralkylrest, z.B. Benzyl oder Phenylethyl, sein.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Das heißt, die Bezeichnung Aryl umfasst in der vorliegenden Erfindung beispielsweise auch bicyclische Reste, in denen sowohl beide Reste aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl. Besonders bevorzugt ist Aryl Phenyl oder Naphthyl, ganz besonders bevorzugt Phenyl. Im Sinne der vorliegenden Anmeldung bedeutet Ph Phenyl.

Die Arylreste können unsubstituiert oder mit einem oder mehreren weiteren Resten substituiert sein. Geeignete weitere Reste sind ausgewählt aus der Gruppe bestehend aus Alkyl, Aryl, Alkoxy, NR²²R²³ und Halogen. Des Weiteren sind Reste der Formel OC(O)R³² geeignet, wobei R³² H, Alkyl oder Aryl, bevorzugt Aryl, besonders bevorzugt Phenylcarbonyloxy, bedeutet, sowie Reste der Formel OR³³, wobei R³³ H, Alkyl oder Aryl, bevorzugt H, bedeutet, sowie Heteroarylreste und Aryloxyreste. Bevorzugte Alkyl-, Aryl-, Alkoxy- und Halogenreste und Heteroarylreste sowie Aryloxyreste sowie Definitionen der Reste R²², R²³ sind vorstehend bereits genannt. Bevorzugt sind die Arylreste unsubstituiert oder mit einer oder mehreren Alkoxygruppen substituiert. Besonders bevorzugt bedeutet Aryl unsubstituiertes Phenyl oder 4-Alkoxyphenyl. Ganz besonders bevorzugt bedeutet Aryl unsubstituiertes Phenyl.

Unter Heteroaryl sind Heteroarylreste zu verstehen, die sich von den vorstehend genannten Arylresten dadurch unterscheiden, dass in dem Grundgerüst der Arylreste mindestens ein Kohlenstoffatom durch ein Heteroatom ersetzt ist. Bevorzugte Heteroatome sind N, O und S. Ganz besonders bevorzugt sind ein, zwei oder drei Kohlenstoffatome des Grundgerüstes der Arylreste durch Heteroatome ersetzt. Insbesondere bevorzugt ist das Grundgerüst ausgewählt aus Systemen wie Pyridyl, Pyridazinyl, Pyrimidinyl, Pyrazinyl, Triazinyl, cyclischen Estern, cyclischen Amiden und fünfgliedrigen Heteroaromaten wie Triazolyl, Imidazolyl, Pyrazolyl, Thienyl, Pyrrolyl und Furyl sowie kondensierten Systemen wie Benzimidazolyl. Das Grundgerüst kann an einer, mehreren oder allen substituierbaren Positionen des Grundgerüsts substituiert sein. Geeignete Substituenten sind dieselben, die bereits bezüglich Aryl genannt wurden.

Geeignete Aryloxygruppen weisen die allgemeine Formel -OR²⁵ auf. R²⁵ stellt dabei einen Arylrest dar, wie er vorstehend definiert wurde. Besonders bevorzugt ist Phenoxy.

Unter Arylen ist gemäß der vorliegenden Anmeldung eine Phenylen, Naphthylen oder Biphenylengruppe zu verstehen, die unsubstituiert oder mit den bezüglich der Arylreste genannten Substituenten substituiert sein kann. Bevorzugt ist die Arylengruppe unsubstituiert. Bevorzugte Phenylengruppen sind 1,3- oder 1,4-Phenylen, wobei 1,4-Phenylen besonders bevorzugt ist; bevorzugte Naphthylengruppen sind 1,5-, 1,6-, 2,6 oder 2,7-Naphthylen, wobei 1,5- oder 2,6-Naphthylen bevorzugt sind; bevorzugte Biphenylengruppen sind 4,4'-Biphenylen.

Geeignete Aminogruppen weisen die Formel NR²²R²³ auf. Darin bedeuten R²² und R²³ unabhängig voneinander H, Alkyl oder Aryl, bevorzugt Aryl. Des Weiteren können R²² und R²³ gemeinsam mit dem N-Atom der Aminogruppe einen cyclischen Rest bilden, bevorzugt einen 5- oder 6-gliedrigen cyclischen Rest. Dieser kann neben dem N-Atom weitere Heteroatome ausgewählt aus N, O und S enthalten.

In einer bevorzugten Ausführungsform betrifft die vorliegende Erfindung die Verwendung von Emittersubstanzen oder Loch- und Excitionenblockem der Formel I, worin die Symbole und Reste die folgenden Bedeutungen aufweisen:
- X: SO₂;
- R¹: H, Methyl, Ethyl, unsubstituiertes Phenyl oder 4-Alkoxyphenyl oder mit einer OH, OC(O)Ph, OCH₂Ph oder N-Phenylbenzimidazolyl-Gruppe, drei CH₃-Gruppen oder zwei F-Resten substituiertes Phenyl, 4-Methoxyphenyl, Thienyl oder Pyridyl; oder 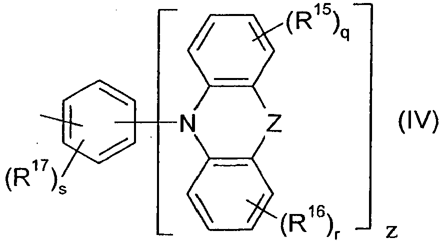 oder 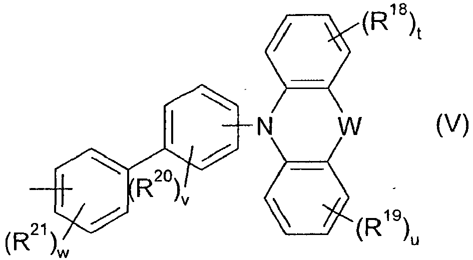
- R², R³: H, unsubstituiertes Phenyl, 1-Naphthyl, 2-Naphthyl oder CHO, wobei mindestens einer der Reste R² oder R³ unsubstituiertes Phenyl, 1-Naphthyl oder 2-Naphthyl bedeutet,
oder
R² und R³ bedeuten 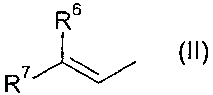 oder
R² bedeutet H und R³ bedeutet 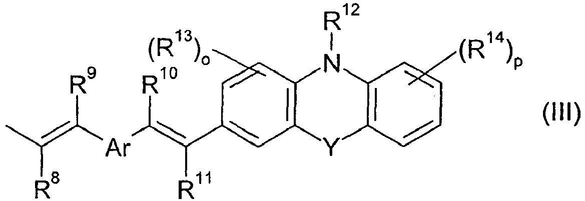
- m, n, o, p,q, r, s, t, u, v und w: 0, 1 oder 2, bevorzugt 0;
- z: 1 oder 2, bevorzugt 1;
- R⁶, R⁷: unsubstituiertes Phenyl;
- R⁸, R⁹, R¹⁰, R¹¹: H;
- R¹²: H, Methyl, Ethyl, unsubstituiertes Phenyl oder 4-Alkoxyphenyl oder mit drei CH₃-Gruppen substituiertes Phenyl;
- R¹⁷: Alkyl, Alkoxy, bevorzugt Methoxy;
- R²² R²³: Aryl
- Y, Z, W: S oder SO₂, bevorzugt SO₂; und
- Ar: Phenylen, Naphthylen oder Biphenylen.

Bevorzugte als Loch- und Excitonenblocker eingesetzte Verbindungen der Formel I sind ausgewählt aus der Gruppe bestehend aus Verbindungen der Formeln Ia, Id, Ie, Ig und Ih, besonders bevorzugt sind die Loch- und Excitonenblocker ausgewählt aus Verbindungen der Formeln Ia und Ih. 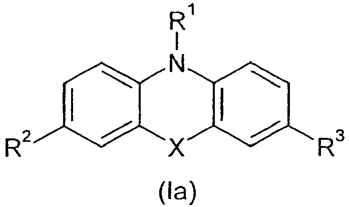 und 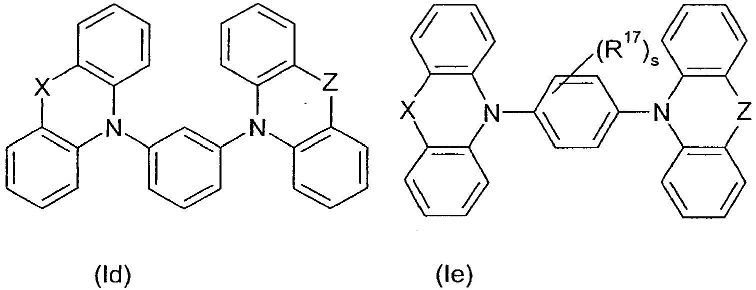 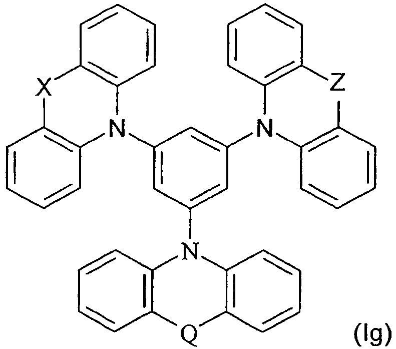 worin bedeuten:
- X, Z, Q: unabhängig voneinander S oder SO₂, bevorzugt SO₂; und
- R¹: H, Methyl, Ethyl oder unsubstituiertes Phenyl;
- R², R³: H oder unsubstituiertes Phenyl, wobei mindestens R² oder R³ unsubstituiertes Phenyl bedeutet;
- R¹⁷: Methoxy;
- S: 0 oder 2, wobei die Substituenten im Falle von s = 2 bevorzugt in der 2-und der 5-Position angeordnet sind.

Besonders bevorzugt sind die Verbindungen der Formeln Ia symmetrisch, d.h., in der Verbindung der Formel Ia sind R² und R³ besonders bevorzugt identisch in den Verbindungen der Formeln Id und Ie sind X und Z besonders bevorzugt identisch und in der Verbindung der Formel Ig sind X, Z und Q besonders bevorzugt identisch. 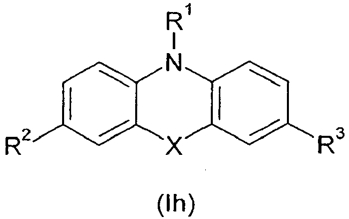 worin bedeuten
- X: S oder SO₂, bevorzugt SO₂;
- R¹: mit einer OH, OC(O)Ph, OCH₂Ph oder N-Phenyl-benzimidazolyl-Gruppe, drei CH₃-Gruppen oder zwei F-Resten substituiertes Phenyl, 4-Methoxyphenyl, Thienyl oder Pyridyl;
- R²: H;
- R³: H oder CHO, bevorzugt H.

Die vorstehend genannten erfindungsgemäß verwendeten Phenothiazin-S-oxid- oder Phenothiazin-S,S-dioxid-Derivate der Formel I können nach dem Fachmann bekannten Verfahren hergestellt werden.

Die Herstellung der Verbindungen der Formel I, worin X, Z und Q S bedeuten, kann entweder durch entsprechende Substitution des kommerziell erhältlichen Phenothiazin-Grundgerüsts, worin R¹, R², R³, R⁴ und R⁵ H bedeuten, erfolgen. Die Reste R², R³, R⁴ und R⁵ werden dabei - sofern es sich nicht um H handelt - durch elektrophile aromatische Substitution eingeführt. Geeignete Reaktionsbedingungen sind dem Fachmann bekannt. Der Rest R¹ wird - sofern es sich nicht um H handelt - durch elektrophile Substitution am Stickstoff eingeführt, z.B. durch Umsetzung mit einem geeigneten Alkyl- oder Arylhalogenid. In einer weiteren bevorzugten Ausführungsform wird das Grundgerüst funktionalisiert, zum Beispiel halogeniert oder mit Aldehydgruppen funktionalisiert. Das funktionalisierte Grundgerüst wird anschließend durch eine Kupplungsreaktion oder durch Wittig-Reaktion zu den gewünschten Phenothiazinen mit den entsprechenden die Reste R², R³, R⁴ und R⁵ tragenden Verbindungen umgesetzt.

Alternativ kann die Herstellung der Verbindungen der Formel I ausgehend von bereits funktionalisierten, zur Herstellung der Phenothiazin-S-oxid- oder Phenothiazin-S,S-dioxid-Derivate geeigneten, Bausteinen erfolgen. Die Herstellung der funktionalisierten Bausteine erfolgt dabei gemäß dem Fachmann bekannten Verfahren. Die Phenothiazin-Derivate können ausgehend von mit den Resten R², R³, R⁴ und R⁵ funktionalisierten Diphenylamin-Derivaten durch Erhitzen mit Schwefel hergestellt werden. Die Herstellung der funktionalisierten Diphenylamin-Derivate ist dem Fachmann bekannt.

Bevorzugt erfolgt die Herstellung der erfindungsgemäß verwendeten Verbindungen der Formel I ausgehend von den kommerziell erhältlichen Phenothiazin-Grundgerüsten.

Die Verbindungen der Formel I, worin X SO₂ oder SO bedeutet, werden zum Beispiel durch Oxidation des entsprechenden funktionalisierten Phenothiazins hergestellt. Geeignete Verfahren zur Oxidation von Phenothiazinen zu Phenothiazin-S-oxiden und Phenothiazin-S,S-dioxiden sind dem Fachmann bekannt und zum Beispiel in M. Tosa et al. Heterocyclic Communications, Vol. 7, No. 3,2001, S. 277 bis 282 angegeben.

Die Oxidation zu Phenothiazin-S-oxid-Derivaten erfolgt zum Beispiel mittels H₂O₂ in Ethanol oder Ethanol-Aceton-Mischungen, H₂O₂ in Oxalsäure, Ammoniumpersulfat, Salpetersäure, salpetriger Säure, anorganischen Stickstoffoxiden, NO₂/O₂, NO⁺BF₄⁻/O₂, CrO₃ in Pyridin, Ozon, Tetramethyloxiran, Perfluoroalkyloxaziridinen oder mittels elektrochemischen Methoden. Des Weiteren kann die Oxidation der entsprechend funktionalisierten Phenothiazine der Formel I zu den entsprechenden Phenoxazin-S-oxid-Derivaten der Formel I mittels m-Chlorperbenzoesäure in CH₂Cl₂ bei Temperaturen von 0 bis 5 °C oder mittels einer Mischung aus rauchender Salpetersäure und Eisessig in CCl₄ erfolgen (siehe zum Beispiel M. Tosa et al. Heterocyclic Communications, Vol. 7, No. 3, 2001, S. 277 bis 282).

Die Oxidation zu Phenothiazin-S,S-dioxid-Derivaten erfolgt zum Beispiel mittels Persäuren wie Peressigsäure, die zum Beispiel aus H₂O₂ und AcOH gebildet wird, oder m-Chlorperbenzoesäure, Natriumperborat, NaOCl oder Schwermetallsystemen wie KMnO₄/H₂O, Et₃PhN⁺MnO₄⁻ in organischen Medien, OsO₄/N-Methylmorpholin-N-oxid. Beispielsweise kann die Oxidation der entsprechend funktionalisierten Phenothiazine der Formel I zu den entsprechenden Phenothiazin-S,S-dioxid-Derivaten der Formel I mittels einer wässrigen Lösung von KMnO₄ und C₁₆H₃₅N(CH₃)₃⁺Cl⁻ in CHCl₃ bei Raumtemperatur oder mittels m-Chlorperbenzoesäure in CH₂Cl₂ bei Raumtemperatur erfolgen (siehe zum Beispiel M. Tosa et al. Heterocyclic Communications, Vol. 7, No. 3, 2001, S. 277 - 282).

Zur Herstellung von Phenothiazin-S,S-dioxid-Derivaten, worin X SO₂ bedeutet, werden das Phenothiazin-Derivat und das Oxidationsmittel, bevorzugt m-Chlorperbenzoesäure, in einem molaren Verhältnis von im Allgemeinen 1 : 1,8 bis 1 : 4, bevorzugt 1 bis 1,9 zu 1 : 3,5, besonders bevorzugt 1 : 1,9 bis 1 : 3 eingesetzt.

Zur Herstellung von Phenothiazin-S-oxid-Derivaten, worin X SO bedeutet, werden das Phenothiazin-Derivat und das Oxidationsmittel in einem molaren Verhältnis von im Allgemeinen 1 : 0,8 bis 1 : 1,5, bevorzugt 1 : 1 bis 1 : 1,3 eingesetzt. Oxidationsmittel, mit denen keine Weiteroxidation zu den entsprechenden S,S-Dioxid-Derivaten erfolgt, z.B. H₂O₂, können in einem größeren Überschuss als dem vorstehend angegebenen in Bezug auf das Phenothiazin-Derivat eingesetzt werden.

Die Oxidation erfolgt im Allgemeinen in einem Lösungsmittel, bevorzugt in einem Lösungsmittel ausgewählt aus der Gruppe bestehend aus halogenierten Kohlenwasserstoffen wie Methylenchlorid und dipolar aprotischen Lösungsmitteln wie Acetonitril oder Sulfolan.

Im Allgemeinen wird die Oxidation zu den Phenothiazin-S-oxid-Derivaten bei einer Temperatur von -10 °C bis +50 °C, bevorzugt -5 °C bis +30 °C, besonders bevorzugt 0 °C bis + 20°C durchgeführt. Üblicherweise erfolgt die Oxidation bei Normaldruck. Die Reaktionsdauer der Oxidation beträgt im Allgemeinen 0,25 bis 24 Stunden, bevorzugt 1 bis 15 Stunden, besonders bevorzugt 2 bis 10 Stunden.

Die Oxidation zu den Phenothiazin-S,S-dioxid-Derivaten erfolgt im Allgemeinen bei einer Temperatur von + 0 bis + 100 °C. Üblicherweise erfolgt die Oxidation bei Normaldruck.

In einer bevorzugten Ausführungsform werden die Phenothiazin-S-oxid-Derivate der Formel I durch Oxidation der entsprechenden Phenothiazin-Derivate der Formel I mit m-Chlorperbenzoesäure als Oxidationsmitel in CH₂Cl₂ bei 0 bis 20 °C hergestellt.

Die Phenothiazin-S,S-dioxid-Derivate der Formel I werden bevorzugt durch Oxidation der entsprechenden Phenothiazin-Derivate der Formel I mit m-Chlorperbenzoesäure als Oxidationsmittel in CH₂Cl₂ bei 0 bis 40 °C hergestellt.

Die Isolierung und Aufarbeitung der erhaltenen Phenothiazin-S-oxide und Phenothiazin-S,S-dioxide erfolgt nach dem Fachmann bekannten Verfahren.

Im Folgenden wird die Herstellung der erfindungsgemäß verwendeten Verbindungen der Formel I anhand von Herstellungsverfahren der bevorzugt verwendeten Verbindungen der Formeln Ia, Ib, Ic, Id, Ie, If und Ig sowie der bevorzugt verwendeten Verbindungen der Formeln Ih und Ii exemplarisch dargestellt. Aufgrund dieser Information sowie mit Kenntnis des Standes der Technik ist der Fachmann in der Lage, die weiteren erfindungsgemäß verwendeten Verbindungen herzustellen.

### a) Herstellung der bevorzugt verwendeten Verbindungen der Formeln Ia und Ih

Die Herstellung der bevorzugt verwendeten Verbindungen der Formeln Ia und Ih erfolgt z.B. ausgehend von einem Grundgerüst der folgenden Formel VI 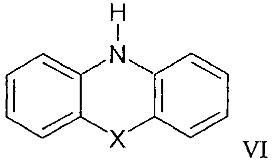 worin X S oder SO₂ bedeutet,
durch
aa) N-Alkylierung oder N-Arylierung des Grundgerüsts der Formel VI,
ab) Halogenierung, und
ac) Kupplungsreaktion mit den den gewünschten Resten R² und R³ entsprechenden. Vorläuferverbindungen;
ad) Oxidation, im Falle von X = S;
wobei der Schritt aa) nur dann durchgeführt wird, wenn R¹ nicht H bedeutet.

Geeignete Reaktionsbedingungen zur Durchführung der Schritte aa), ab), ac) und ad) sind dem Fachmann bekannt. Im Folgenden sind bevorzugte Ausführungsformen der Schritte aa), ab), ac) und ad) genannt.

### Schritt aa)

Die N-Alkylierung bzw. N-Arylierung erfolgt bevorzugt durch Umsetzung des Grundgerüsts der Formel VI mit einem Alkylhalogenid bzw. Arylhalogenid der Formel R¹-Hal, wobei R¹ bereits vorstehend definiert wurde und Hal Cl, Br oder I, bevorzugt I, bedeutet.

Die N-Alkylierung bzw. N-Arylierung wird in Anwesenheit einer Base durchgeführt. Geeignete Basen sind dem Fachmann bekannt und bevorzugt ausgewählt aus der Gruppe bestehend aus Alkali- und Erdalkalihydroxiden wie NaOH, KOH, Ca(OH)₂, Alkalihydriden wie NaH, KH, Alkaliamiden wie NaNH₂, Alkali- oder Erdalkalimetallcarbonaten wie K₂CO₃, und Alkalimetallalkoxiden wie NaOMe, NaOEt. Des Weiteren sind Gemische der vorstehend genannten Basen geeignet. Besonders bevorzugt sind NaOH, KOH oder NaH.

Die N-Alkylierung (zum Beispiel offenbart in M. Tosa et al., Heterocycl. Communications, Vol. 7, No. 3, 2001, S. 277 - 282) bzw. N-Arylierung (zum Beispiel offenbart in H. Gilman und D. A. Shirley, J. Am. Chem. Soc. 66 (1944) 888; D. Li et al., Dyes and Pigments 49 (2001) 181 - 186) wird bevorzugt in einem Lösungsmittel durchgeführt. Geeignete Lösungsmittel sind z.B. polare aprotische Lösungsmittel wie Dimethylsulfoxid, Dimethylformamid oder Alkohole. Es ist ebenfalls möglich, einen Überschuss des eingesetzten Alkyl- oder Arylhalogenids als Lösungsmittel einzusetzen, wobei der Einsatz eines Überschusses, an Alkyl- oder Aryliodiden bevorzugt ist. Die Umsetzung kann des Weiteren in einem unpolaren aprotischen Lösungsmittel, z.B. Toluol, durchgeführt werden, wenn ein Phasentransferkatalysator, z.B. tetra-n-Butylammoniumhydrogensulfat, anwesend ist (wie z. B. in I. Gozlan et al., J. Heterocycl. Chem. 21 (1984) 613 - 614 offenbart ist).

Die N-Arylierung kann zum Beispiel durch Kupfer-katalysierte Kupplung der Verbindung der Formel VI mit einem Arylhalogenid, bevorzugt einem Aryliodid erfolgen (Ullmann-Reaktion). Ein geeignetes Verfahren zur N-Arylierung von Phenothiazin in Anwesenheit von Kupferbronze ist zum Beispiel in H. Gilman et al., J. Am. Chem. Soc. 66 (1944) 888 - 893 offenbart.

Das molare Verhältnis der Verbindung der Formel VI zu dem Alkylhalogenid bzw. Arylhalogenid der Formel R¹-Hal beträgt im Allgemeinen 1 : 1 bis 1 : 2, bevorzugt 1 : 1 bis 1 : 1,5.

Die N-Alkylierung bzw. N-Arylierung wird im Allgemeinen bei einer Temperatur von 0 bis 220 °C, bevorzugt 20 bis 200 °C durchgeführt. Die Reaktionsdauer beträgt im Allgemeinen 0,5 bis 48 h, bevorzugt 1 bis 24 h. Im Allgemeinen wird die N-Alkylierung bzw. N-Arylierung bei Normaldruck durchgeführt.

Das erhaltene Rohprodukt wird gemäß dem Fachmann bekannten Verfahren aufgearbeitet.

Falls R¹ in der Verbindung der Formel Ia H ist, entfällt Schritt aa).

### Schritt ab)

Die Halogenierung kann gemäß dem Fachmann bekannten Verfahren durchgeführt werden. Bevorzugt erfolgt eine Bromierung oder Iodierung in 3- und 7-Position des gegebenenfalls N-alkylierten oder N-arylierten Grundgerüsts der Formel VI.

Eine Bromierung des gegebenenfalls in Schritt aa) N-alkylierten oder N-arylierten Grundgerüsts der Formel VI in der 3- und 7-Position des Grundgerüsts kann z. B. gemäß M. Jovanovich et al. J. Org. Chem. 1984, 49, 1905 - 1908 durch Umsetzung mit Brom in Essigsäure erfolgen. Des Weiteren kann eine Bromierung entsprechend dem in C. Bodea et al. Acad. Rep. Rom. 13 (1962) 81- 87 offenbarten Verfahren erfolgen.

Eine Iodierung des gegebenenfalls in Schritt aa) N-alkylierten oder N-arylierten Grundgerüsts der Formel VI in der 3- und 7-Position des Grundgerüsts kann zum Beispiel gemäß dem in M. Sailer et al. J. Org. Chem. 2003, 68, 7509 - 7512 offenbarten Verfahren erfolgen. Dabei erfolgt zunächst eine Lithiierung des entsprechenden, gegebenenfalls N-alkylierten oder N-arylierten 3,7-Dibromo-substituierten Grundgerüsts der Formel VI und eine anschließende Iodierung des lithiierten Produktes.

Die Lithiierung kann mit einer Lithium-Base ausgewählt aus der Gruppe bestehend aus n-Butyllithium und Lithiumdiisopropylamid bei Temperaturen von im Allgemeinen -78 bis +25 °C, bevorzugt -78 bis 0 °C, besonders bevorzugt -78 °C gemäß dem Fachmann bekannten Verfahren durchgeführt werden. Anschließend wird die Reaktionsmischung auf Raumtemperatur erwärmt und gemäß dem Fachmann bekannten Verfahren aufgearbeitet.

### Schritt ac)

Die Phenothiazin-Derivate werden bevorzugt durch Kupplungsreaktion mit den den gewünschten Resten R² und R³ entsprechenden Vorläuferverbindungen hergestellt. Geeignete Kupplungsreaktionen sind zum Beispiel die Suzuki-Kupplung und die Yamamoto-Kupplung, wobei die Suzuki-Kupplung bevorzugt ist.

Bei der Suzuki-Kupplung können an den Positionen 3 und 7 des Phenothiazin-Grundgerüsts der Formel VI, das gegebenenfalls N-alkyliert oder N-aryliert ist, halogenierte, insbesondere bromierte, Verbindungen, mit den den gewünschten Resten R² und R³ entsprechenden Boronsäuren oder Boronsäureestern unter Pd(0)-Katalyse in Gegenwart einer Base zu den entsprechenden, in 3,7-Position R², R³-substituierten Verbindungen umgesetzt werden.

Anstelle der den gewünschten Resten R² und R³ entsprechenden Boronsäuren oder Boronsäureester können auch andere borhaltige Verbindungen, die die gewünschten Reste R² und R³ tragen, in der Umsetzung mit den halogenierten Phenothiazin-Derivaten eingesetzt werden. Bei den borhaltigen Verbindungen handelt es sich um cyclische Verbindungen der allgemeinen Formeln R²-B(-O-[C(R²⁷)₂]ₙ-O-) und R³-B(-O-[C(R²⁷)₂]ₙ-O-), worin R² und R³ die für R² und R³ vorstehend genannten Bedeutungen aufweisen, R²⁷ gleich oder verschieden ist und ausgewählt aus Wasserstoff oder C₁-C₂₀-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Decyl, n-Dodecyl oder n-Octadecyl; bevorzugt C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso-Propyl, n-Butyl, iso-Butyl, sec-Butyl und tert.-Butyl, ganz besonders bevorzugt Methyl; und n eine ganze Zahl von 2 bis 10, vorzugsweise 2 bis 5 ist.

Die den gewünschten Resten R² und R³ entsprechenden Boronsäuren, Boronsäureester und borhaltigen Verbindungen können nach im Stand der Technik bekannten Verfahren hergestellt werden oder sind kommerziell erhältlich. Beispielsweise ist die Herstellung der Boronsäuren und Boronsäureester durch Umsetzung von Grignard- oder Lithium-Reagenzien mit Boranen, Diboranen oder Boraten möglich.

Als Pd(0)-Katalysatoren sind alle üblichen Pd(0)-Katalysatoren geeignet. Beispielsweise können Tris(dibenzylidenaceton)-dipalladium(0) oder Tetrakis(triphenylphosphin)-palladium(0) eingesetzt werden. Des Weiteren kann ein Pd(II)-Salz im Gemisch mit einem Liganden eingesetzt werden, zum Beispiel Pd(ac)₂ oder PdCl₂ und PPh₃, wobei Pd(0) in situ gebildet wird. Zur Durchführung der Kupplung kann ein Überschuss an PPh₃ zugegeben werden. Die Katalysatoren werden im allgemeinen in einer Menge von 0,001 bis 15 mol-%, bevorzugt 0,01 bis 10 mol-%, besonders bevorzugt 0,1 bis 5 mol-%, bezogen auf das eingesetzte halogenierte Phenothiazin-Derivat, eingesetzt.

In der Suzuki-Kupplung können alle üblicherweise in der Suzuki-Kupplung eingesetzten Basen eingesetzt werden. Bevorzugt werden Alkalicarbonate wie Natrium- oder Kaliumcarbonat eingesetzt. Die Base wird im Allgemeinen im molaren Überschuß von 2- bis 200-fach, bevorzugt 2- bis 100-fach, besonders bevorzugt 2- bis 80-fach, bezogen auf das eingesetzte halogenierte Phenothiazin-Derivat, eingesetzt.

Die den gewünschten Resten R² und R³ entsprechende Komponente (Boronsäure, der entsprechende Boronsäureester oder die anderen geeigneten borhaltigen Verbindungen) werden in einem Verhältnis zu dem halogenierten Phenothiazin-Derivat von 100 bis 400 mol-%, bevorzugt 100 bis 300 mol-%, besonders bevorzugt 100 bis 150 mol-% eingesetzt.

Die Umsetzung erfolgt im Allgemeinen bei einer Temperatur von 40 bis 140 °C, bevorzugt 60 bis 120 °C, besonders bevorzugt 70 bis 100 °C. Der Druck beträgt im Allgemeinen Normaldruck.

Die Umsetzung wird im Allgemeinen unter Ausschluss von Sauerstoff durchgeführt. Üblicherweise erfolgt die Umsetzung in einem Lösungsmittel ausgewählt aus der Gruppe bestehend aus Benzol, Toluol, Tetrahydrofuran, 1,4-Dioxan, Dimethoxyethan, Ethanol und Petrolether. Es ist ebenfalls möglich, eine Mischung von Tetrahydrofuran, Dimethoxyethan oder Ethanol und Wasser als Lösungsmittel einzusetzen.

In einer vorteilhaften Variante des Verfahrens wird ein halogeniertes Phenothiazin-Derivat in Lösung unter Schutzgas vorgelegt und mit der Base, die bevorzugt gelöst, z. B. in einem Dimethoxyethan/Wasser-Gemisch, vorliegt, und einer den gewünschten Resten R² und R³ entsprechenden Boronsäure versetzt. Im Anschluss daran wird unter Schutzgas der Pd(0)-Katalysator zugegeben. Es wird über einen Zeitraum von im Allgemeinen 2 bis 120 Stunden, bevorzugt 4 bis 72 Stunden, besonders bevorzugt 6 bis 48 Stunden bei den vorstehend genannten Temperaturen und Drucken gerührt. Im Anschluss daran wird das Reaktionsgemisch nach den dem Fachmann bekannten Verfahren aufgearbeitet.

Die Phenothiazinderivate können neben der Suzuki-Kupplung durch andere dem Fachmann bekannte Verfahren, insbesondere andere Kupplungsreaktionen, hergestellt werden.

In einer weiteren Ausführungsform der vorliegenden Erfindung werden die Phenothiazin-Derivate durch Umsetzung von an den Positionen 3 und 7 halogenierten, insbesondere bromierten, gegebenenfalls N-alkylierten oder N-arylierten Phenothiazinen mit einem Grundgerüst der Formel VI mit den den gewünschten Resten R² und R³ entsprechenden HalogenVerbindungen, insbesondere Brom-Verbindungen, unter Ni(0)-Katalyse (Yamamoto-Kupplung) erhalten.

In einer bevorzugten Ausführungsform der Yamamoto-Kupplung wird unter Sauerstoffausschluss eine Lösung, bevorzugt eine DMF-Lösung, des Katalysators hergestellt aus einer Ni(0)-Verbindung, bevorzugt Ni(COD)₂, und Bipyridyl in äquimolaren Mengen eingesetzt. Zu dieser Lösung werden unter Sauerstoff-Ausschluss das halogenierte, bevorzugt bromierte, Phenothiazin-Derivat und die den gewünschten Resten R² und R³ entsprechenden Brom-Verbindungen in einem Lösungsmittel, bevorzugt Toluol, zugegeben.

Die Reaktionsbedingungen wie Temperatur, Druck, Lösungsmittel und Verhältnis des halogenierten, bevorzugt bromierten Phenothiazin-Derivats zu der R² und R³ entsprechenden Komponente bei der Herstellung der Phenothiazin-Derivate mittels Yamamoto-Kupplung entsprechen denen der Suzuki-Kupplung.

Als Ni(0)-Verbindungen zur Herstellung des Katalysators sind alle üblichen Ni(0)-Verbindungen geeignet. Beispielsweise können Ni(C₂H₄)₃, Ni(1,5-Cyclooctadien)₂ ("Ni(COD)₂"), Ni(1,6-Cyclodecadien)₂ oder Ni(1,5,9-all-trans-Cyclododecatrien)2 eingesetzt werden. Die Katalysatoren werden im Allgemeinen in einer Menge von 1 bis 100 mol-%, bevorzugt 5 bis 80 mol-%, besonders bevorzugt 10 bis 70 mol-%, bezogen auf das eingesetzte halogenierte Phenothiazin-Derivat, eingesetzt.

Geeignete Verfahrensbedingungen und Katalysatoren, insbesondere für die Suzuki-Kupplung sind zum Beispiel in Suzuki-Miyaura-Kreuzkupplung: A. Suzuki, J. Organomet. Chem. 576 (1999) 147 - 168; B-Alkyl-Suzuki-Miyaura-Kreuzkupplung: S. R. Chemler et al., Angew. Chem. 2001, 113, 4676 - 4701 und der darin genannten Literatur offenbart.

### Schritt ad)

Bevorzugte Oxidationsmittel und Verfahrensbedingungen zur Oxidation der Phenothiazine zu den entsprechenden Phenothiazin-S,S-dioxid-Derivaten sind vorstehend genannt und zum Beispiel in M. Tosa et al., Heterocyclic Commun. 7 (2001) 277 - 282 offenbart.

### b) Herstellung der bevorzugten Verbindungen der Formel Id

Die bevorzugten Verbindungen der Formel Id werden z.B. gemäß dem folgenden Verfahren hergestellt. 1,3-Dihalogenbenzol, bevorzugt 1,3-Diiodbenzol, wird mit im Allgemeinen mit 1,8 bis 2,2, bevorzugt 2 Moläquivalenten Phenothiazin-S-oxid oder Phenothiazin-S,S-dioxid in Gegenwart von Kupferpulver und Alkalicarbonat, bevorzugt Kaliumcarbonat, auf im Allgemeinen 160 - 220 °C erhitzt und 8 - 48 h bei dieser Temperatur gehalten. Man lässt den Reaktionsansatz auf ca. 140 °C abkühlen, gibt Essigester dazu und erhitzt etwa 1 h lang unter Rückfluss zum Sieden. Die Aufarbeitung kann z.B. gemäß den folgenden Schritten erfolgen. Die Lösung wird heiß filtriert und nach dem Abkühlen mit Alkohol, bevorzugt Methanol, versetzt. Der Niederschlag wird abgesaugt, getrocknet und anschließend umkristallisiert.

Bei der vorstehend genannten Umsetzung entstehen Verbindungen der Formel Id, worin X und Z dieselbe Bedeutung aufweisen. Zur Herstellung von Verbindungen der Formel Id, worin X und Z verschiedene Bedeutungen aufweisen, kann zunächst eine Umsetzung des 1,3-Dihalogenbenzols mit 0,9 bis 1,1, bevorzugt 1, Moläquivalent Phenothiazin, Phenothiazin-S-oxid oder Phenothiazin-S,S-dioxid erfolgen und anschließend eine Umsetzung der erhaltenen Verbindung mit 0,9 bis 1,1, bevorzugt 1, Moläquivalent eines weiteren, von dem ersten Phenothiazin, Phenothiazin-S-oxid oder Phenothiazin-S,S-dioxid verschiedenen Phenothiazin, Phenothiazin-S-oxid oder Phenothiazin-S,S-dioxid.

Es ist ebenfalls möglich, die Verbindungen der Formel Id durch Umsetzung von 1,3-Difluorbenzol mit im Allgemeinen mit 1,8 bis 2,2, bevorzugt 2 Moläquivalenten eines mit NaH deprotonierten Phenothiazin-S-oxids oder Phenothiazin-S,S-dioxids umzusetzen. Die Aufarbeitung erfolgt nach dem Fachmann bekannten Verfahren.

Bei der vorstehend genannten Umsetzung entstehen Verbindungen der Formel Id, worin X und Z dieselbe Bedeutung aufweisen. Zur Herstellung von Verbindungen der Formel Id, worin X und Z verschiedene Bedeutungen aufweisen, kann zunächst eine Umsetzung des 1,3-Difluorbenzols mit 0,9 bis 1,1, bevorzugt 1, Moläquivalenten eines mit NaH deprotonierten Phenothiazins, Phenothiazin-S-oxids oder Phenothiazin-S,S-dioxids erfolgen und anschließend eine Umsetzung der erhaltenen Verbindung mit 0,9 bis 1,1, bevorzugt 1, Moläquivalenten eines weiteren, von dem ersten deprotonierten Phenothiazin, Phenothiazin-S-oxid oder Phenothiazin-S,S-dioxid verschiedenen mit NaH deprotonierten Phenothiazins, Phenothiazin-S-oxids oder Phenothiazin-S,S-dioxids.

### c) Herstellung der bevorzugten Verbindungen der Formel Ie)

Die bevorzugten Verbindungen der Formel Ie werden z.B. gemäß dem folgenden Verfahren hergestellt. 1,4-Dihalogenbenzol, bevorzugt 1,4-Diiodbenzol werden mit im Allgemeinen 1,8 bis 2,2, bevorzugt 2 Moläquivalenten Phenothiazin-S-oxid oder Phenothiazin-S,S-dioxid in Gegenwart von Kupferpulver und Alkalicarbonat, bevorzugt Kaliumcarbonat, auf im Allgemeinen 160 - 220 °C erhitzt und 8 - 48 h bei dieser Temperatur gehalten. Man lässt den Reaktionsansatz auf ca. 100 °C abkühlen, gibt heißes Wasser dazu und erhitzt etwa 1 h lang unter Rückfluss zum Sieden. Die Aufarbeitung kann z.B. gemäß den folgenden Schritten erfolgen. Die Lösung wird heiß filtriert. Der Rückstand wird getrocknet und anschließend in Methylenchlorid etwa 1 h lang unter Rückfluss zum Sieden erhitzt. Nach dem Abkühlen auf Raumtemperatur wird die Suspension filtriert. Das Filtrat wird an Kieselgel in Methylenchlorid chromatographiert.

Bei der vorstehend genannten Umsetzung entstehen - entsprechend der Herstellung der Verbindungen der Formel Id - Verbindungen der Formel Ie, worin X und Z dieselbe Bedeutung aufweisen. Zur Herstellung von Verbindungen der Formel Ie, worin X und Z verschiedene Bedeutungen aufweisen, kann zunächst eine Umsetzung des 1,4-Dihalogenbenzols mit 0,9 bis 1,1, bevorzugt 1, Moläquivalenten Phenothiazin, Phenothiazin-S-oxid oder Phenothiazin-S,S-dioxid erfolgen und anschließend eine Umsetzung der erhaltenen Verbindung mit 0,9 bis 1,1, bevorzugt 1, Moläquivalenten eines weiteren, von dem ersten Phenothiazin, Phenothiazin-S-oxid oder Phenothiazin-S,S-dioxid verschiedenen Phenothiazin, Phenothiazin-S-oxid oder Phenothiazin-S,S-dioxid.

Es ist ebenfalls möglich, die Verbindungen der Formel Ie durch Umsetzung von 1,4-Difluorbenzol mit einem mit NaH deprotonierten Phenothiazin-S-oxid oder Phenothiazin-S,S-dioxid umzusetzen. Die Herstellung von symmetrischen (X und Z weisen dieselbe Bedeutung auf) und unsymmetrischen Verbindungen der Formel Ie kann entsprechend der Herstellung von Verbindungen der Formel Id erfolgen.

### d) Herstellung der bevorzugten Verbindungen der Formel Ig)

Die bevorzugten Verbindungen der Formel Ig werden z.B. gemäß dem folgenden Verfahren hergestellt. Umsetzung von 1,3,5-Trifluorbenzol mit im Allgemeinen mit 2,8 bis 3,2, bevorzugt 3 Moläquivalenten eines mit NaH deprotonierten Phenothiazin-S-oxids oder Phenothiazin-S,S-dioxids. Die Aufarbeitung erfolgt nach dem Fachmann bekannten Verfahren.

Bei der vorstehend genannten Umsetzung entstehen Verbindungen der Formel Ig, worin X, Z und Q dieselbe Bedeutung aufweisen. Verbindungen der Formel Ig, worin X, Z und Q verschiedene Bedeutungen aufweisen, können durch sequentielle Umsetzung des 1,3,5-Trifluorbenzols mit verschiedenen mit NaH deprotonierten Phenothiazinen, Phenothiazin-S-oxiden oder Phenothiazin-S,S-dioxiden erfolgen.

Die erfindungsgemäß verwendeten Phenothiazin-S-oxid- oder Phenothiazin-S,S-dioxid-Derivate der Formel I eignen sich zum Einsatz in OLEDs. Sie werden als Loch- oder Excitionenblocker in OLEDs eingesetzt.

Die vorstehend genannten Verbindungen der Formel I sind hervorragend für den Einsatz als Loch- oder Excitonenblocker in organischen Leuchtdioden (OLEDs) geeignet. Ein weiterer Gegenstand der vorliegenden Erfindung sind organische Leuchtdioden umfassend eine Blockschicht für Löcher und Excitonen, enthaltend oder bestehend aus mindestens einer Verbindung der Formel I. Bevorzugte Verbindungen der Formel I sind vorstehend genannt.

Organische Leuchtdioden (OLEDs) sind grundsätzlich aus mehreren Schichten aufgebaut, z.B.:
1. Anode
2. Löcher-transportierende Schicht
3. Licht-emittierende Schicht
4. Elektronen-transportierende Schicht
5. Kathode

Es sind auch von dem vorstehend genannten Aufbau verschiedene Schichtenfolgen möglich, die dem Fachmann bekannt sind. Beispielsweise ist es möglich, dass das OLED nicht alle der genannten Schichten aufweist, zum Beispiel ist ein OLED mit den Schichten (1) (Anode), (3) (Licht-emittierende Schicht) und (5) (Kathode) ebenfalls geeignet, wobei die Funktionen der Schichten (2) (Löcher-transprotierende Schicht) und (4) (Elektronentransprotierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Ein weiterer Gegenstand der vorliegenden Erfindung ist eine organische Leuchtdiode (OLED) umfassend eine Blockschicht für Löcher und Excitonen, wobei die Blockschicht für Löcher und Excitonen mindestens eine Verbindung der Formel I enthält.

Die einzelnen der vorstehend genannten Schichten des OLEDs können wiederum aus 2 oder mehreren Schichten aufgebaut sein. Beispielsweise kann die Löcher-transportierende Schicht aus einer Schicht aufgebaut sein in die aus der Elektrode Löcher injiziert werden und einer Schicht, die die Löcher von der Loch injizierenden Schicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten organischen Verbindungen angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der Elektronen-transportierenden Schicht sollte mit der Arbeitsfunktion der Kathode angeglichen sein.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen Ib, IVa, Va und VIa des Periodensystems der Elemente sowie die Übergangsmetalle der Gruppe VIIIa. Wenn die Anode lichtdurchlässig sein soll, werden im Allgemeinen gemischte Metalloxide der Gruppen IIb, IIIb und IVb des Periodensystems der Elemente (alte IUPAC-Version) eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) des erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus 4,4'-Bis[N-(1-naphthyl)-N-phenyl-amino]biphenyl (□-NPD), N,N'-Diphenyl-N,N'-bis(3-methylphenyl)-[1,1'-biphenyl]-4,4'-diamin (TPD), 1,1-Bis[(di-4-tolylamino)phenyl]-cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4'-diamin (TTB), 4,4',4"-tris(N,N-Diphenylamino)triphenylamin (TDTA), Porphyrinverbindungen und Phthalocyaninen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Geeignete Elektronentransportmaterialien für die Schicht (4) der erfindungsgemäßen O-LEDs umfassen mit oxinoiden Verbindungen chelatisierte Metalle wie Tris(8-chinolinolato)aluminium (Alq₃), Verbindungen auf Phenanthrolinbasis wie 2,9-Dimethyl-4,7-diphenyl-1,10-phenanthrolin (DDPA = BCP) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Puf-ferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Von den vorstehend als Lochtransportmaterialien und Elektronentransportmaterialien genannten Materialien können einige mehrere Funktionen erfüllen. Zum Beispiel sind einige der Elektronen leitenden Materialien gleichzeitig Löcher blockende Materialien, wenn sie ein tief liegendes HOMO aufweisen.

Die Ladungstransportschichten können auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Beispielsweise können die Lochtransportmaterialien mit Elektronenakzeptoren dotiert werden, zum Beispiel können Phthalocyanine bzw. Arylamine wie TPD oder TDTA mit Tetrafluorotetracyano-chinodimethan (F4-TCNQ) dotiert werden. Die Elektronentransprotmaterialien können zum Beispiel mit Alkalimetallen dotiert werden, beispielsweise Alq₃ mit Lithium. Die elektronische Dotierung ist dem Fachmann bekannt und zum Beispiel in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo. Appl. Phys. Lett., Vol. 82, No. 25, 23 June 2003 und Pfeiffer et al., Organic Electronics 2003, 4, 89 - 103 offenbart.

Die Kathode (5) ist eine Elektrode, die zur Einführung von Elektronen oder negativen Ladungsträgern dient. Die Kathode kann jedes Metall oder Nichtmetall sein, das eine geringere Arbeitsfunktion aufweist als die Anode. Geeignete Materialien für die Kathode sind ausgewählt aus der Gruppe bestehend aus Alkalimetallen der Gruppe Ia, zum Beispiel Li, Cs, Erdalkalimetallen der Gruppe IIa, Metallen der Gruppe IIb des Periodensystems der Elemente (alte IUPAC-Version), umfassend die Seltenerdmetalle und die Lanthanide und Aktinide. Des Weiteren können Metalle wie Aluminium, Indium, Calcium, Barium, Samarium und Magnesium sowie Kombinationen davon eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Das OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält das erfindungsgemäße OLED zusätzlich zu den Schichten (1) bis (5) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Blockschicht für Löcher zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Es ist jedoch auch möglich, dass das OLED nicht alle der genannten Schichten (1) bis (5) aufweist, zum Beispiel ist ein OLED mit den Schichten (1) (Anode), (3) (Licht-emittierende Schicht) und (5) (Kathode) ebenfalls geeignet, wobei die Funktionen der Schichten (2) (Löcher-transportiende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z.B. in WO 00/70655 offenbart.

Des Weiteren kann jede der genannten Schichten des erfindungsgemäßen OLEDs aus zwei oder mehreren Schichten ausgebaut sein. Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, ein OLED mit einer hohen Effizienz zu erhalten.

Die Herstellung des erfindungsgemäßen OLEDs kann nach dem Fachmann bekannten Methoden erfolgen. Im Allgemeinen wird das erfindungsgemäße OLED durch aufeinander folgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden.

Im Allgemeinen haben die verschiedenen Schichten folgende Dicken: Anode (1) 500 bis 5000 Å, bevorzugt 1000 bis 2000 Å; Löcher-transportierende Schicht (2) 50 bis 1000 Å, bevorzugt 200 bis 800 Å, Licht-emittierende Schicht (3) 10 bis 1000 Å, bevorzugt 100 bis 800 Å, Elektronen transportierende Schicht (4) 50 bis 1000 Å, bevorzugt 200 bis 800 Å, Kathode (5) 200 bis 10.000 Å, bevorzugt 300 bis 5000 Å. Die Lage der Rekombinationszone von Löchern und Elektronen in dem erfindungsgemäßen OLED und somit das Emissionsspektrum des OLED können durch die relative Dicke jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelnen Schichten in dem OLED ist von den eingesetzten Materialien abhängig. Die Schichtdicken von gegebenenfalls eingesetzten zusätzlichen Schichten sind dem Fachmann bekannt.

Die Effizienz der erfindungsgemäßen OLEDs kann durch Optimierung der anderen Schichten verbessert werden. Beispielsweise können hoch effiziente Kathoden wie Ca, Ba oder LiF eingesetzt werden. Geformte Substrate und neue Löcher-transportierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energielevel der verschiedenen Schichten einzustellen und um Elektrolumineszenz zu erleichtern. Die erfindungsgemäß verwendeten Phenothiazin-S-oxid- oder Phenothiazin-S,S-dioxid-Derivate der Formel I sind als Loch- und Excitonenblocker geeignet und können erfindungsgemäß in der Loch- und Excitonenblockschicht eines OLEDs eingesetzt werden.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschinne in Handys, Laptops, Digitalkameras, Fahrzeugen sowie Zielanzeigen an Bussen und Bahnen.

Weiterhin können die erfindungsgemäß eingesetzten Phenothiazin-S-oxid- oder Phenothiazin-S,S-dioxid-Derivate der Formel I in OLEDs mit inverser Struktur eingesetzt werden. Bevorzugt werden die erfindungsgemäß eingesetzten Verbindungen der Formel I in diesen inversen OLEDs wiederum in der Loch- und Excitonenblockschicht eingesetzt. Der Aufbau von inversen OLEDs und die üblicherweise darin eingesetzten Materialien sind dem Fachmann bekannt.

Ein weiterer Gegenstand der vorliegenden Erfindung sind Phenothiazin-S,S-dioxid-Derivate ausgewählt aus der Gruppe bestehend aus Verbindungen der Formeln XIV, XVI, XVII, XVIII, XIX, XX und XXII 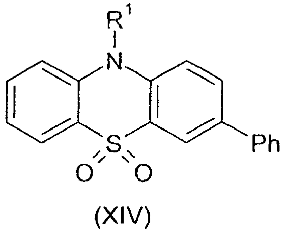 und 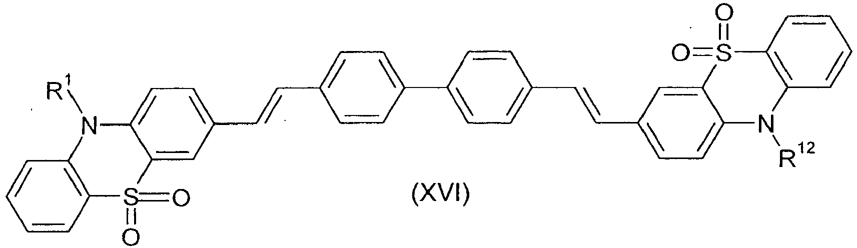 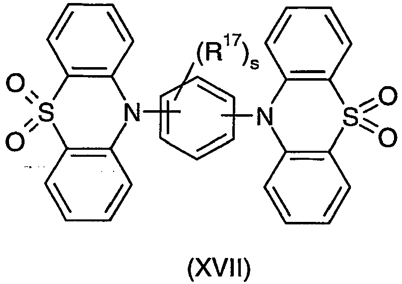 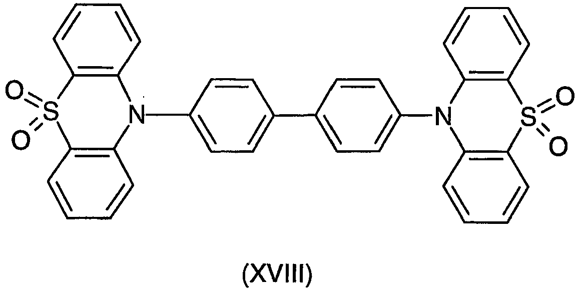 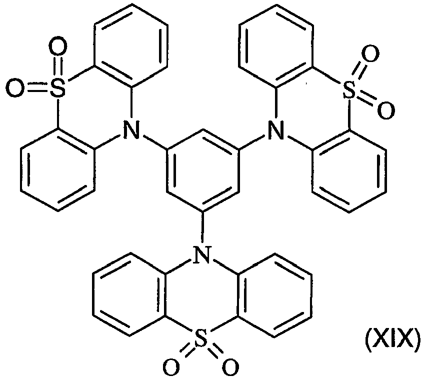 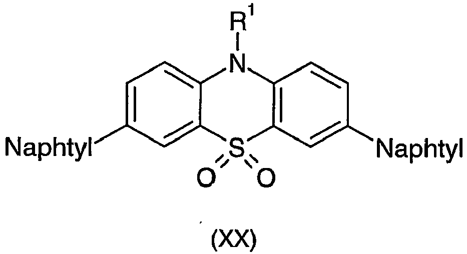 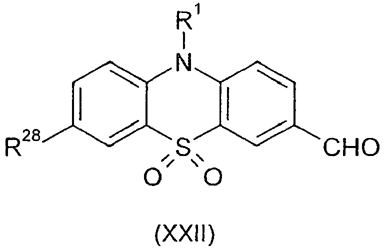 worin bedeuten:
- R¹, R¹²: H, Me, Et oder unsubstituiertes Phenyl;
- R¹⁷: Methoxy;
- s: 0 oder 2, wobei die Substituenten im Falle von s = 2 bevorzugt in der 2-und der 5-Position angeordnet sind;
- Ph: unsubstituiertes Phenyl;
- Naphthyl: 1- oder 2-Naphthyl; und
- R²⁸: H oder -CHO;
und

Phenothiazin-S-oxid-derivate der Formel XXX 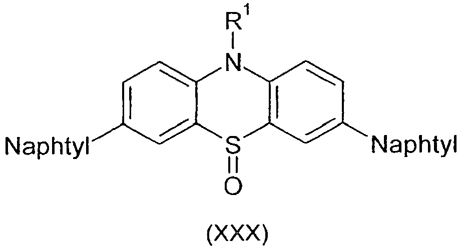 worin bedeuten:
- R¹: H, Me, Et oder unsubstituiertes Phenyl; und
- Naphthyl: 1- oder 2-Naphthyl.

Ein weiterer Gegenstand der vorliegenden Erfindung sind Phenothiazin-S,S-dioxid-Derivate ausgewählt aus der Gruppe bestehend aus Verbindungen der Formeln XXXI und XXXII 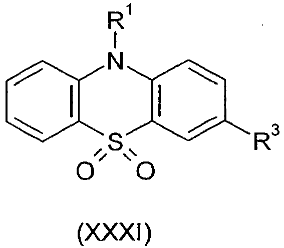 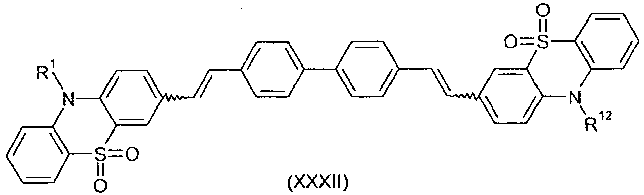 worin bedeuten
- R¹: mit einer OH, OC(O)Ph, OCH₂Ph oder N-Phenyl-benzimidazolyl-Gruppe, drei CH₃-Gruppen oder zwei F-Resten substituiertes Phenyl oder Pyridyl; bevorzugt 2-Hydroxyphenyl, 4-Hydroxyphenyl, 2-Benzoyloxyphenyl, 4-Benzoyloxyphenyl, 4-Benzyloxyphenyl, 3,5-Difluorophenyl, Mesitylenyl, 4-(2'-N-Phenyl-benzimidazolyl)phenyl, 4-Methoxyphenyl, 2-Thienyl, 2-Pyridyl,
- R³: H oder CHO, bevorzugt H,
- R¹²: ein mit drei CH₃-Gruppen substituiertes Phenyl, bevorzugt Mesitylenyl.

Die vorstehend genannten Phenothiazin-S,S-dioxid-Derivate der Formeln XIV, XVI, XVII, XVIII, XIX, XX und XXII und XXXI, XXXII sowie die Phenothiazin-S-oxid-Derivate der Formel XXX sind besonders gut für die Verwendung in OLEDs geeignet, wobei die Phenothiazin-S,S-dioxid-Derivate der Formel XXII insbesondere als Zwischenprodukte zur Herstellung der erfindungsgemäßen Phenothiazin-S,S-dioxid-Derivate geeignet sind. Die erfindungsgemäßen Verbindungen der Formeln XIV, XVI, XVII, XVIII, XIX, XX, XXII und XXX sowie XXXI und XXXII sind insbesondere als Ladungstransportmaterialien oder Emittersubstanzen, die bevorzugt Licht im blauen Bereich des elektromagnetischen Spektrums emittieren, geeignet. Die erfindungsgemäßen Phenothiazin-S,S-dioxid-Derivate der Formeln XIV, XVII, XIX, XXII, bevorzugt XIV, XXI, XXII sowie XXXI sind insbesondere als Emitter- oder Lochblocker geeignet. Des Weiteren können die Verbindungen der Formel XVI als Matrixmaterialien eingesetzt werden. Bevorzugt werden die erfindungsgemäßen Verbindungen in der Licht emittierenden Schicht eines OLEDs als Emittersubstanzen, die bevorzugt Licht im blauen Bereich des elektromagnetischen Spektrums emittieren, eingesetzt oder als Loch- und Excitonenblocker. Ganz besonders bevorzugt ist die Verwendung als Emittersubstanzen in der Licht-emittierenden Schicht eines OLED oder in der Loch- und Excitonenblockschicht.

Ein weiterer Gegenstand der vorliegenden Anmeldung ist daher die Verwendung der erfindungsgemäßen Phenothiazin-S,S-dioxid-Derivate in OLEDs sowie ein OLED enthaltend mindestens eine der erfindungsgemäßen Verbindungen. Bevorzugte Ausführungsformen geeigneter OLEDs sowie geeignete Materialien für die einzelnen Schichten der OLEDs und für die Kathode und Anode sind vorstehend genannt.

Bevorzugt werden die erfindungsgemäßen Verbindungen in der Licht-emittierenden Schicht eingesetzt. Ein weiterer Gegenstand der vorliegenden Anmeldung ist daher eine Licht-emittierende Schicht enthaltend mindestens ein erfindungsgemäßes Phenothiazin-S,S-dioxid-Derivat. Ein weiterer Gegenstand der vorliegenden Anmeldung ist ein OLED enthaltend die erfindungsgemäße Licht-emittierende Schicht. In einer weiteren bevorzugten Ausführungsförm werden die erfindungsgemäßen Verbindungen in der Loch- und Excitonenblockschicht eingesetzt. Weitere Gegenstände sind daher eine Loch- und Excitonenblockschicht enthaltend mindestens eine erfindungsgemäße Verbindung sowie ein OLED enthaltend die erfindungsgemäße Loch- und Excitonenblockschicht. Bevorzugte Ausführungsformen geeigneter OLEDs sowie geeignete Materialien für die einzelnen Schichten der OLEDs und für die Anode und Kathode sind vorstehend genannt. Die erfindungsgemäßen Verbindungen können in Abhängigkeit von dem Substitutionsmuster der erfindungsgemäßen Verbindungen verschiedene Funktionen in der Licht-emittierenden Schicht oder in der Loch- und Excitonenblockschicht erfüllen. Bevorzugt werden die erfindungsgemäßen Verbindungen als Emittersubstanzen eingesetzt.

Die Herstellung der erfindungsgemäßen Phenothiazin-S,S-dioxid-Derivate der Formeln XIV, XVI, XVII, XVIII, XIX, XX und XXII und XXXI und XXXII und Phenothiazin-S-oxid-Derivate der Formel XXX kann nach den vorstehend genannten Verfahren erfolgen. Die entsprechenden Verfahren zur Herstellung der erfindungsgemäßen Phenothiazin-S,S-dioxid-Derivate der Formeln XIV, XVI, XVII, XVIII, XIX, XX und XXII und Phenothiazin-S-oxid-Derivate der Formel XXX sind ebenfalls Gegenstand der vorliegenden Anmeldung.

Die nachfolgenden Beispiele erläutern die Erfindung zusätzlich.

### Beispiele

### Beispiel 1: 3-Phenyl-phenothiazin-5,5-dioxid

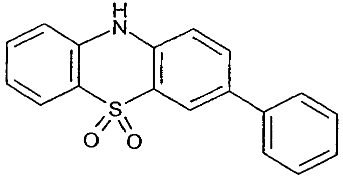

Eine Suspension von 2,00 g (7,2 mmol) 3-Phenylphenothiazin (J. Cymerman-Craig, W. P. Rogers und G. P. Warwick, Aust. J. Chem. 1955, 8, 252 - 257) in 45 ml Methylenchlorid wurde bei Raumtemperatur unter Rühren portionsweise mit 3,40 g (13,8 mmol) 70 %iger m-Chlorperbenzoesäure versetzt. Nach 4stündigem Rühren bei Raumtemperatur wurde der Niederschlag abfiltriert, mit Methylenchlorid gewaschen und im Vakuum getrocknet. Das Rohprodukt (0,95 g) wurde zweimal aus Essigsäure umkristallisiert. Nach der Trocknung des hellgrauen Feststoffs im Hochvakuum bei 100 °C wurden 0,492 g (22 % d. Th.) analysenreine Substanz mit einem Schmelzpunkt von 269 - 272 °C erhalten, dessen Lösung in Tetrahydrofuran bei λ = 383 nm fluoreszierte.

### Beispiel 2: 10-Methyl-3,7-diphenylphenothiazin

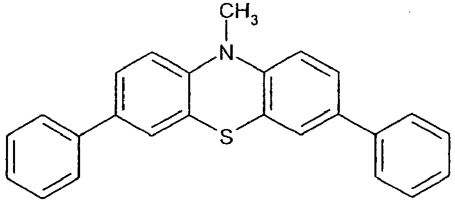

2,50 g (6,7 mmol) 3,7-Dibrom-10-methylphenothiazin (C. Bodea und M. Terdic, Acad. Rep. Rom. 1962, 13, 81 - 87), 1,85 g (14,9 mmol) 98 %ige Phenylboronsäure, 0,11 g (0,14 mmol) Palladium-bis(triphenylphosphan)-dichlorid und 1,03 g (7,4 mmol) Kaliumcarbonat wurden in 55 ml Dimethoxyethan und 28 ml Wasser unter Stickstoff unter Rückfluss zum Sieden (75 °C) fünf Stunden lang erhitzt. Die Reaktionsmischung wurde auf Raumtemperatur abgekühlt und über Nacht nachgerührt. Der Niederschlag wurde abgesaugt, nacheinander mit 125 ml Ethanol und heißem Wasser gewaschen und bei 70 °C im Vakuum getrocknet. Das Rohprodukt (2,30 g) wurde zwei Stunden lang in Cyclohexan unter Rückfluss zum Sieden erhitzt. Nach Filtration der heißen Suspension wurde der Rückstand getrocknet, in 40 ml Methylenchlorid gelöst und über eine mit Kieselgel gefüllte Glasfritte filtriert. Nach Entfernen des Lösungsmittels wurden 1,05 g (43 % n. Th.) hellgelber analysenreiner Feststoff mit einem Schmelzpunkt von 239 - 241 °C erhalten, dessen Lösung in Chloroform bei λ = 464 nm fluoreszierte.

### Beispiel 3: 10-Methyl-3,7-diphenylphenothiazin-5,5-dioxid

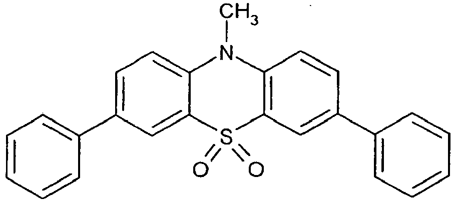

Eine Lösung von 1,95 g (5,3 mmol) 10-Methyl-3,7-diphenylphenothiazin in 65 ml Methylenchlorid wurde mit 2,67 g (10,7 mmol) 70 %iger m-Chlorperbenzoesäure portionsweise bei Raumtemperatur versetzt und zwei Stunden bei 20 - 25 °C gerührt. Die Reaktionslösung wurde nacheinander jeweils zweimal mit 10 ml 10 %iger Kalilauge, 10 ml 5 %iger Salzsäure und 10 ml gesättigter Natriumhydrogencarbonatlösung ausgeschüttelt. Die organische Phase wurde abgetrennt und säulenchromatographisch (Laufmittel: Essigester) gereinigt. Das erhaltene Rohprodukt (1,80 g) wurden aus Toluol umkristallisiert und anschließend im Hochvakuum sublimiert. Es wurden 0,65 g (31 % d. Th.) hellbeigefarbener analysenreiner Feststoff mit einem Schmelzpunkt von 242 - 245 °C erhalten, dessen Lösung in Chloroform bei λ = 386 nm fluoreszierte.

### Beispiel 4: 10-Methyl-3,7-bis(1-naphthyl)phenothiazin

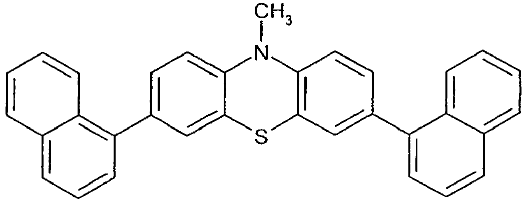

9,30 g (25,1 mmol) 3,7-Dibrom-10-methylphenothiazin, 9,50 g (55,2 mmol) 1-Naphthylboronsäure, 0,407 g (0,50 mmol) Palladium-bis(triphenylphosphan)-dichlorid und 3,80 g (27,5 mmol) Kaliumcarbonat wurden in 204 ml Dimethoxyethan und 101 ml Wasser unter Stickstoff fünf Stunden lang unter Rückfluss zum Sieden erhitzt. Die Reaktionsmischung wurde auf Raumtemperatur abgekühlt, über Nacht nachgerührt und dann filtriert. Der Rückstand wurde mit 470 ml Ethanol und heißem Wasser gewaschen und bei 70 °C im Vakuum getrocknet. Der Feststoff wurde in 100 ml Methylenchlorid gelöst und über Kieselgel filtriert. Nach dem Entfernen des Lösungsmittels im Vakuum wurde eine klebrige Masse erhalten, die nach Zugabe von 200 ml Methanol unter Rühren über Nacht kristallisierte. Die Kristalle wurden abgesaugt, mit 300 ml Methanol gewaschen und bei 40 °C im Vakuum getrocknet. Es wurden 10,33 g hellgelbe Mikrokristalle mit einem Schmelzpunkt von 185 - 190 °C erhalten. Das Rohprodukt wurde zweimal aus Essigester umkristallisiert. Es wurden 5,71 g (49 % d. Th.) analysenreine fast farblose Mikrokristalle mit einem Schmelzpunkt von 191-194 °C erhalten, deren Lösung in Chloroform gelöst bei λ = 468 nm fluoreszierte.

### Beispiel 5: 10-Methyl-3,7-bis(1-naphthyl)phenothiazin-5-oxid

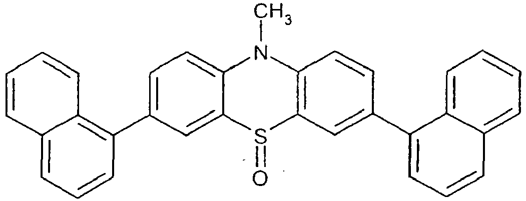

Zu einer eisgekühlten Suspension von 2,50 g (5,37 mmol) 10-Methyl-3,7-bis(1-naphthyl)-phenothiazin in 60 ml Methylenchlorid wurde eine Lösung von 1,20 g (5,35 mmol) 77 %iger m-Chlorperbenzoesäure in 20 ml Methylenchlorid innerhalb von 30 min getropft. Die Reaktionslösung wurde 2 Stunden lang bei 0 - 5 °C gerührt. Anschließend wurden weitere 0,60 g (2,70 mmol) m-Chlorperbenzoesäure, gelöst in 10 ml Methylenchlorid, zugetropft. Die Lösung wurde 2 Stunden lang bei 0 - 5 °C nachgerührt und dann auf Raumtemperatur erwärmt. Nach dem Ausschütteln der Reaktionslösung mit jeweils zweimal 15 ml 10 % KOH, 15 ml 5 % HCl und 25 ml gesättigter Natriumhydrogencarbonatlösung wurde die organische Phase säulenchromatographisch an Kieselgel (Laufmittel: Methylenchlorid) gereinigt. Die erste Fraktion enthielt das Sulfon (siehe Beispiel 6), von dem 0,38 g (14 % d. Th.) analysenreiner farbloser Feststoff mit einem Schmelzpunkt von 221 - 225 °C isoliert wurden, der in Chloroform gelöst bei λ = 385 nm fluoreszierte. Nach Abtrennen des Sulfons wurde das Laufmittel auf Essigester umgestellt. Nach Entfernen des Lösungsmittels wurde eine klebrige Paste erhalten, die nach Zusatz von Wasser kristallisierte. Es wurden 1,53 g (59 % d. Th.) 10-Methyl-3,7-bis(1-naphthyl)phenothiazin-5-oxid als analysenreiner hellbrauner Feststoff mit einem Zersetzungspunkt > 150 °C erhalten, dessen Lösung in Chloroform bei λ = 388 nm fluoreszierte. Als Nebenprodukt wurde 10-Methyl-3,7-bis(1-naphthyl)phenothiazin-5,5-dioxid erhalten.

### Beispiel 6: 10-Methyl-3,7-bis(1-naphthyl)phenothiazin-5,5-dioxid

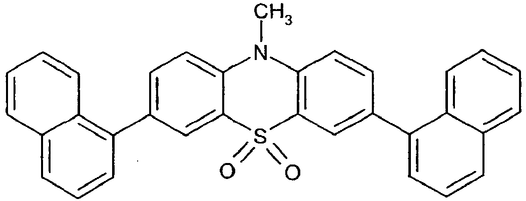

Zur Herstellung als Nebenprodukt in der Synthese von 10-Methyl-3,7-bis(1-naphthyl)-phenothiazin-5-oxid siehe unter Beispiel 5. Für eine gezielte Herstellung des Sulfons werden mindestens zwei Moläquivalente m-Chlorperbenzoesäure verwendet.

Es wurden farblose Mikrokristalle mit einem Schmelzpunkt von 221 - 225 °C erhalten, deren Lösung in Chloroform bei λ = 385 nm fluoreszierte.

### Beispiel 7: 10-Methyl-3,7-bis(2-naphthyl)phenothiazin

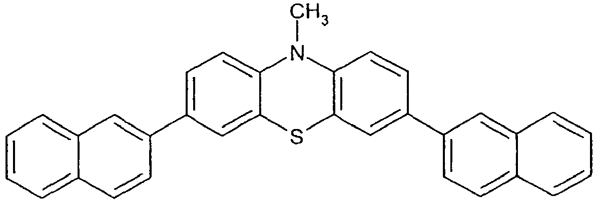

9,30 g (25,1 mmol) 3,7-Dibrom-10-methylphenothiazin, 9,50 g (55,2 mmol) 2-Naphthylboronsäure, 0,407 g (0,50 mmol) Palladium-bis(triphenylphosphan)-dichlorid und 3,80 g (27,5 mmol) Kaliumcarbonat wurden in 204 ml Dimethoxyethan und 101 ml Wasser unter Stickstoff fünf Stunden lang unter Rückfluss zum Sieden erhitzt. Die Reaktionsmischung wurde auf Raumtemperatur abgekühlt, über Nacht nachgerührt und dann filtriert. Der Rückstand wurde mit 470 ml Ethanol und heißem Wasser gewaschen und bei 70 °C im Vakuum getrocknet. Der Feststoff wurde in 200 ml Methylenchlorid gelöst und über Kieselgel filtriert. Nach dem Entfernen des Lösungsmittels im Vakuum wurden 9,6 g grünlichgelber Feststoff erhalten (Schmelzpunkt 276 - 281 °C), der aus 500 ml Toluol umkristallisiert wurde. Es wurden 7,10 g (61 % d. Th.) analysenreine gelbglänzende Mikrokristalle mit einem Schmelzpunkt von 285 - 289 °C erhalten, deren Lösung in Chloroform bei λ = 402 nm fluoreszierte.

### Beispiel 8: 10-Methyl-3,7-bis(2-naphthyl)phenothiazin-5-oxid

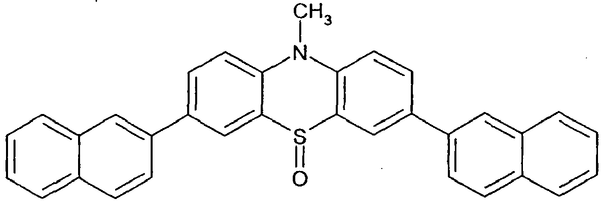

Zu einer eisgekühlten Suspension von 2,50 g (5,37 mmol) 10-Methyl-3,7-bis(2-naphthyl)-phenothiazin in 60 ml Methylenchlorid wurde eine Lösung von 1,20 g (5,35 mmol) 77 %iger m-Chlorperbenzoesäure in 20 ml Methylenchlorid innerhalb von 30 min getropft. Die Reaktionslösung wurde 2 Stunden lang bei 0 - 5 °C gerührt. Anschließend wurden weitere 0,60 g (2,70 mmol) m-Chlorperbenzoesäure, gelöst in 10 ml Methylenchlorid, zugetropft. Die Lösung wurde 2 Stunden lang bei 0 - 5 °C nachgerührt und dann auf Raumtemperatur erwärmt. Nach dem Ausschütteln der Reaktionslösung mit jeweils zweimal 15 ml 10 % KOH, 15 ml 5 % HCl und 25 ml gesättigter Natriumhydrogencarbonatlösung wurde die organische Phase säulenchromatographisch an Kieselgel (Laufmittel: Methylenchlorid) gereinigt. Die erste Fraktion enthielt 0,62 g Sulfon (siehe Beispiel 9), das aus 36 ml o-Dichlorbenzol umkristallisiert wurde. Es wurden 0,44 g (16 % d. Th.) gelblicher Feststoff mit einem Schmelzpunkt von 328 - 332 °C erhalten. Nach Abtrennen des Sulfons wurde das Laufmittel auf Essigester umgestellt. Nach Entfernen des Lösungsmittels wurden 1,30 g Feststoff erhalten, der aus 134 ml Essigsäure umkristallisiert wurde. Es wurden 0,54 g (21 % d. Th.) 10-Methyl-3,7-bis(2-naphthyl)phenothiazin-5-oxid als analysenreiner beigefarbener Feststoff mit einem Schmelzpunkt von 275 - 280 °C erhalten, dessen Lösung in Chloroform bei λ = 402 nm fluoreszierte. Als Nebenprodukt wurde 10-Methyl-3,7-bis(2-naphthyl)phenothiazin-5,5-dioxid erhalten.

### Beispiel 9: 10-Methyl-3,7-bis(2-naphthyl)phenothiazin-5,5-dioxid

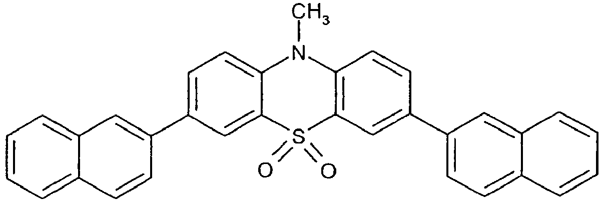

Zur Herstellung als Nebenprodukt in der Synthese von 10-Methyl-3,7-bis(2-naphthyl)-phenothiazin-5-oxid siehe unter Beispiel 8. Für eine gezielte Herstellung des Sulfons werden mindestens zwei Moläquivalente m-Chlorperbenzoesäure verwendet.
Es wurden gelbliche Mikrokristalle mit einem Schmelzpunkt von 328 - 332 °C erhalten.

### Beispiel 10: 10-Methyl-3,7-bis(2-phenylpropenyl)phenothiazin

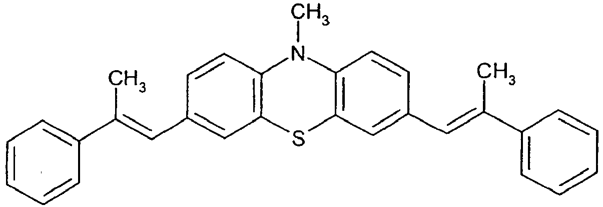

Zu einer Lösung von 6,50 g (26,8 mmol) (1-Phenylethyl)phosphonsäurediethylester (Beispiel 13 in US 5,130,603) in 60 ml wasserfreiem Dimethylsulfoxid wurden unter Rühren und bei Raumtemperatur 3,46 g (30,4 mmol) Kalium-tert.-butylat und dann 3,60 g (13,4 mmol) 10-Methylphenothiazin-3,7-dicarbaldehyd (H. Oelschläger und H. J. Peters, Arch. Pharm. (Weinheim) 320, 379 - 381, 1987) gegeben, wobei die Temperatur von 25 auf 41 °C anstieg. Nach fünfstündigem Rühren bei Raumtemperatur wurde die Reaktionslösung mit 150 ml Methanol versetzt, 15 min gerührt und über ein Schwarzbandfilter filtriert. Der Rückstand wurde mit 300 ml Methanol gewaschen und im Vakuum bei 80 °C getrocknet. Das Rohprodukt (3,6 g) wurde in 100 ml Methylenchlorid gelöst, an Kieselgel gereinigt und aus 480 ml Acetonitril umkristallisiert. Es wurden 1,79 g (30 % d. Th.) analysenreine leuchtend gelbe Mikrokristalle mit einem Schmelzpunkt von 189 - 191 °C erhalten, deren Lösung in Chloroform bei λ = 479 nm fluoreszierte.

### Beispiel 11: 10-Methyl-3,7-bis(2-phenylpropenyl)phenothiazin-5,5-dioxid

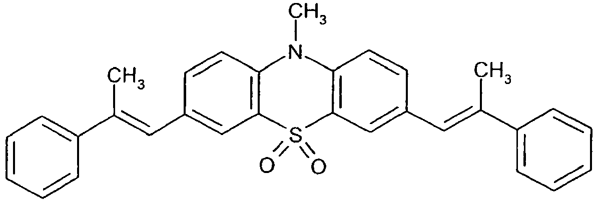

Eine Lösung von 1,70 g (3,81 mmol) 10-Methyl-3,7-bis(2-phenylpropenyl)phenothiazin in 65 ml Methylenchlorid wurde portionsweise mit 1,90 g (7,60 mmol) m-Chlorperbenzoesäure bei Raumtemperatur versetzt und zwei Stunden nachgerührt. Die Reaktionslösung wurde jeweils zweimal mit 10 ml 10 % KOH, 10 ml 5 % HCl und 10 ml gesättigter Natriumhydrogencarbonatlösung ausgeschüttelt. Die organische Phase wurde mit Methylenchlorid auf 100 ml verdünnt und anschließend an Kieselgel gereinigt, wobei das Laufmittel auf Essigester umgestellt wurde. Der so gereinigte Feststoff (1,00 g) wurde aus 40 ml Essigester umkristallisiert. Es wurden 0,57 g (31 % d. Th.) analysenreine farblose Mikrokristalle mit einem Schmelzpunkt von 190 - 193 °C erhalten, deren Lösung in Chloroform bei λ = 394 nm fluoreszierte.

### Beispiel 12: 3,7-Bis(2,2-diphenylvinyl)-10-methylphenothiazin

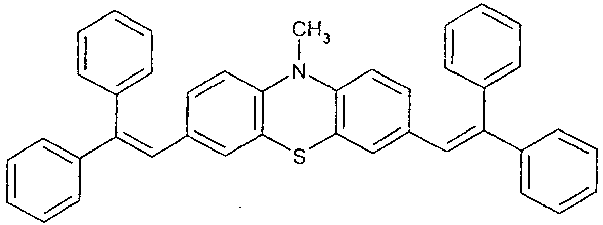

Zu einer Lösung von 8,20 g (26,8 mmol) Benzhydrylphosphonsäurediethylester (hergestellt analog zu Beispiel 13 in US 5,130,603) in 60 ml wasserfreiem Dimethylsulfoxid wurden unter Rühren und bei Raumtemperatur 3,46 g (30,4 mmol) Kalium-tert.-butylat und dann 3,60 g (13,4 mmol) 10-Methylphenothiazin-3,7-dicarbaldehyd gegeben, wobei die Temperatur von 25 auf 43 °C anstieg. Nach fünfstündigem Rühren bei Raumtemperatur wurde die Reaktionslösung mit 150 ml Methanol versetzt, 15 min gerührt und über ein Schwarzbandfilter filtriert. Der Rückstand wurde mit 300 ml Methanol gewaschen und im Vakuum bei 80 °C getrocknet. Das Rohprodukt (4,7 g) wurde in 150 ml Methylenchlorid gelöst, an Kieselgel gereinigt und aus 83 ml Butylglykol umkristallisiert. Es wurden 3,56 g (23 % d. Th.) grünliche Mikrokristalle mit einem Schmelzpunkt von 233 - 241 °C erhalten.

### Beispiel 13: 3,7-Bis(2,2-diphenylvinyl)-10-methylphenothiazin-5,5-dioxid

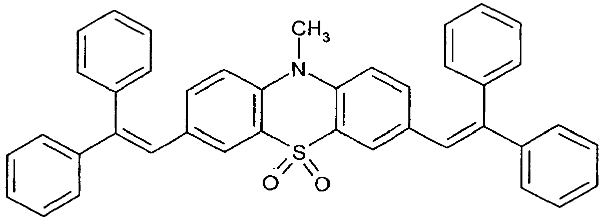

Eine Lösung von 1,80 g (3,16 mmol) 10-Methyl-3,7-bis(2-phenylpropenyl)phenothiazin in 65 ml Methylenchlorid wurde portionsweise mit 1,60 g (6,5 mmol) 70 %iger m-Chlorperbenzoesäure bei Raumtemperatur versetzt und zwei Stunden nachgerührt. Die Reaktionslösung wurde jeweils zweimal mit 10 ml 10 % KOH, 10 ml 5 % HCl und 10 ml gesättigter Natriumhydrogencarbonatlösung ausgeschüttelt. Nach dem Entfernen des Lösungsmittels wurde der Feststoff (1,20 g) wurde aus 184 ml Essigsäure umkristallisiert. Es wurden 0,86 g (45 % d. Th.) analysenreine fast farblose Mikrokristalle mit einem Schmelzpunkt von 246 - 252 °C erhalten, die in Chloroform gelöst bei λ = 453 nm fluoreszierten.

### Beispiel 14: 3,7-Bis[1,3-bis(trifluoracetyl)-2,3-dihydroimidazol-2-yl]-10-phenylphenothiazin

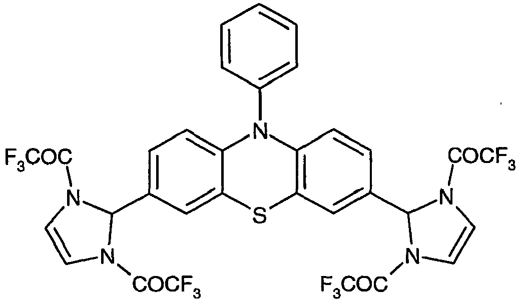

Zu einer Lösung von 28,2 g (0,416 mol) Imidazol in 396 ml wasserfreiem Acetonitril wurden unter Rühren und Stickstoff 228,8 g (1,09 mol) Trifluoracetanhydrid innerhalb von 5 min getropft. Nachdem die Lösung auf Rückflusstemperatur erhitzt worden war, wurden 55,0 g (0,198 mol) 10-Phenylphenothiazin (J. Cymerman-Craig, W. P. Rogers und G. P. Warwick, Aust. J. Chem. 1955, 8, 252 - 257) zugegeben. Die Lösung wurde 8 Stunden lang unter Rückfluss zum Sieden erhitzt. Anschließend wurde das Lösungsmittel abdestilliert, und der Rückstand mit Eis/Wasser und Natriumcarbonat versetzt. Der Feststoff wurde abgesaugt und in 825 ml Methanol suspendiert. Die Suspension wurde über eine Nutsche gesaugt, und der Rückstand zweimal mit jeweils 50 ml Ether gewaschen und im Vakuum getrocknet. Es wurden 110 g gelblicher Feststoff erhalten.

### Beispiel 15: 10-Phenylphenothiazin-3,7-dicarbaldehyd

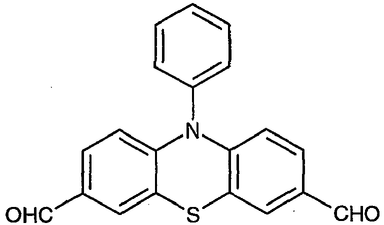

Eine Lösung von 14,0 g (17,6 mmol) 3,7-Bis[1,3-bis(trifluoracetyl)-2,3-dihydroimidazol-2-yl]-10-phenylphenothiazin in 350 ml Acetonitril wurde nach Zugabe von 200 ml 1,9 n HCl 3 Stunden lang unter Rückfluss zum Sieden erhitzt. Nach Abkühlen auf Raumtemperatur wurde die Suspension filtriert. Nach Einengen des Filtrats wurde der gebildete Niederschlag abgesaugt, mit Wasser neutral gewaschen und bei 80 °C im Vakuum getrocknet (3,0 g dunkelgelber Feststoff). Der Feststoff wurde in 25 ml Methylenchlorid gelöst und an Kieselgel gereinigt. Es wurden 1,29 g orangefarbener Feststoff mit einem Schmelzpunkt von 194 - 198 °C erhalten.

### Beispiel 16: 3,7-Bis(2,2-diphenylvinyl)-10-phenylphenothiazin

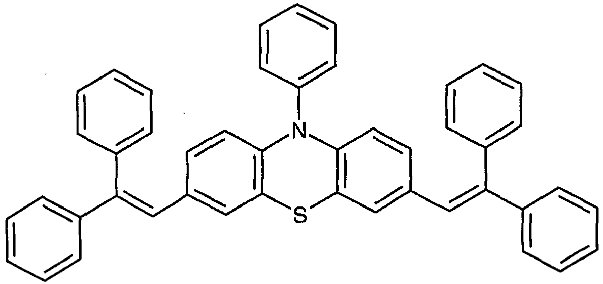

Zu einer Lösung von 15,61 g (51,3 mmol) Benzhydrylphosphonsäurediethylester (hergestellt analog zu Beispiel 13 in US 5,130,603) in 121 ml über Molekularsieb getrocknetem Dimethylsulfoxid wurden bei Raumtempertur unter Rühren 6,63 g (58,1 mmol) Kaliumtert.-butylat und dann 8,50 g (25,7 mmol) 10-Phenyl-phenothiazin-3,7-dicarbaldehyd gegeben. Nach fünfstündigem Rühren bei Raumtemperatur wurde die Reaktionslösung mit 1500 ml Methanol verdünnt und 15 min nachgerührt. Der Niederschlag wurde abfiltriert, mit Methanol gewaschen und bei 80 °C im Vakuum getrocknet. Es wurden 6,92 g (43 % d. Th.) gelblicher Feststoff mit einem Schmelzpunkt von 232 - 237 °C erhalten.

### Beispiel 17: 3,7-Bis(2,2-diphenylvinyl)-10-phenylphenothiazin-5,5-dioxid

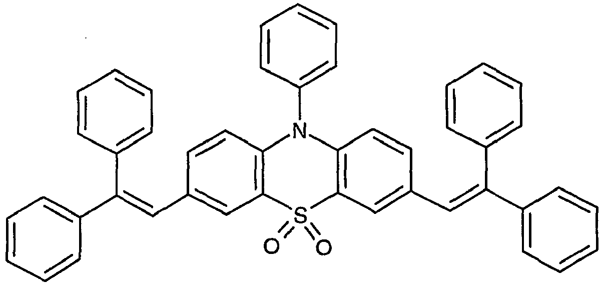

Eine Lösung von 6,85 g (10,8 mmol) 3,7-Bis(2,2-diphenylvinyl)-10-phenylphenothiazin in 250 ml Methylenchlorid wurde portionsweise mit 5,84 g (26,1 mmol) 77 %iger m-Chlorperbenzoesäure bei Raumtemperatur versetzt und zwei Stunden nachgerührt. Die Reaktionslösung wurde jeweils zweimal mit 30 ml 10 % KOH, 30 ml 5 % HCl und 30 ml gesättigter Natriumhydrogencarbonatlösung ausgeschüttelt. Nach dem Entfernen des Lösungsmittels wurde der Feststoff (7,19 g) in 40 ml Methylenchlorid gelöst und an Kieselgel gereinigt. Nach Entfernen des Lösungsmittels wurden 4,86 g (65 % d. Th.) analysenreine gelbliche Mikrokristalle mit einem Schmelzpunkt von 269 - 274 °C erhalten, deren Lösung in Methylenchlorid bei λ = 454 nm fluoreszierte.

### Beispiel 18: 10-Methyl-3-(2-{4'-[2-(10-methylphenothiazin-3-yl)-vinyl]-biphenyl-4-yl}-vinyl)-phenothiazin

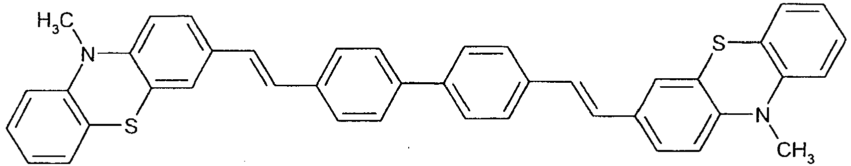

Zu einer Lösung von 1,42 g (3,14 mmol) [4'-(Diethoxyphosphorylmethyl)-biphenyl-4-yl-methyl]-phosphonsäurediethylester (US 3,984,399, Beispiel 2, Spalte 29, Zeile 58 - 66) in 32 ml über Molekularsieb getrocknetem Dimethylsulfoxid wurden unter Stickstoff bei Raumtempertur unter Rühren 0,80 g (7,1 mmol) Kalium-tert.-butylat und dann 1,82 g (3,3 mmol) 10-Methyl-phenothiazin-3-carbaldehyd gegeben. Nach vierstündigem Rühren bei Raumtemperatur wurde die Reaktionslösung mit 70 ml Methanol verdünnt und 15 min nachgerührt. Der Niederschlag wurde abfiltriert, mit 172 ml Methanol gewaschen und bei 35 °C im Vakuum getrocknet. Das Rohprodukt (1,70 g) wurde in 255 ml N-Methylpyrrolidon umkristallisiert. Nach dem Entfernen von Lösungsmittelresten im Hochvakuum bei 225 °C wurden 0,98 g (50 % d. Th.) analysenreiner gelber Feststoff mit einem Schmelzpunkt von 380 °C erhalten, dessen Lösung in N-Methylpyrrolidon bei λ = 576 nm fluoreszierte.

### Beispiel 19: 10-Methyl-3-(2-{4'-[2-(10-methyl-5,5-dioxo-phenothiazin-3-yl)-vinyl]-biphenyl-4-yl}-vinyl)-phenothiazin-5,5-dioxid

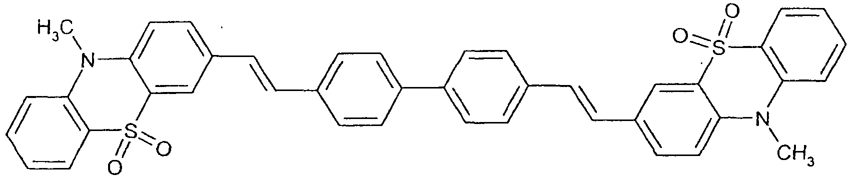

Zu einer Lösung von 1,82 g (4,00 mmol) [4'-(Diethoxy,phosphorylmethyl)-biphenyl-4-yl-methyl]-phosphonsäurediethylester in 41 ml über Molekularsieb getrocknetem Dimethylsulfoxid wurden unter Stickstoff bei Raumtempertur unter Rühren 1,02 g (9,05 mmol) Kalium-tert.-butylat und dann 2,20 g (8,05 mmol) 10-Methyl-5,5-dioxo-phenothiazin-3-carbaldehyd (Beispiel 21) gegeben. Nach vierstündigem Rühren bei Raumtemperatur wurde die Reaktionslösung mit 90 ml Methanol verdünnt und 15 min nachgerührt. Der Niederschlag wurde abfiltriert, mit 220 ml Methanol gewaschen und bei 35 °C im Vakuum getrocknet. Das Rohprodukt (2,50 g) wurde in 58 ml Dimethylsulfoxid umkristallisiert. Nach dem Entfernen von Lösungsmittelresten im Hochvakuum bei 200 °C wurden 1,64 g (59 % d. Th.) analysenreiner gelber Feststoff mit einem Schmelzpunkt von 360 °C erhalten, dessen Lösung in N-Methylpyrrolidon bei λ = 460 nm fluoreszierte.

### Beispiel 20: 10-Phenyl-3-(2-{4'-[2-(10-phenylphenothiazin-3-yl)-vinyl]-biphenyl-4-yl}-vinyl)-phenothiazin

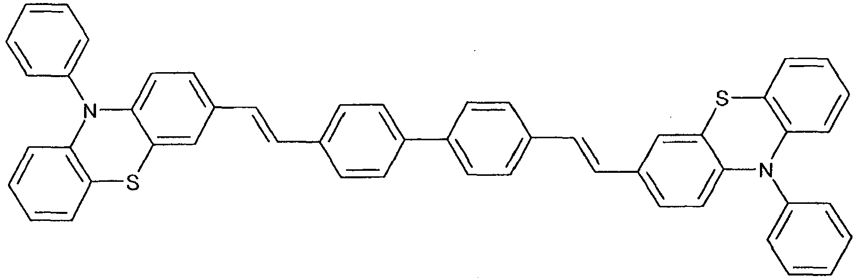

Zu einer Lösung von 4,83 g (10,7 mmol) [4'-(Diethoxyphosphorylmethyl)-biphenyl-4-yl-methyl]-phosphonsäurediethylester in 80 ml über Molekularsieb getrocknetem Dimethylsul-foxid wurden unter Stickstoff bei Raumtemperatur unter Rühren 2,73 g (24,1 mmol) Kalium-tert.-butylat und dann 7,80 g (25,7 mmol) 10-Phenyl-phenothiazin-3-carbaldehyd gegeben. Nach vierstündigem Rühren bei Raumtemperatur wurde die Reaktionslösung mit 200 ml Methanol verdünnt und 15 min nachgerührt. Der Niederschlag wurde abfiltriert, mit 500 ml Methanol gewaschen und bei 35 °C im Vakuum getrocknet. Das Rohprodukt (5,85 g) wurde in 400 ml Toluol umkristallisiert. Es wurden 3,85 g gelber Feststoff mit einem Schmelzpunkt von 278 - 290 °C erhalten.

### Beispiel 21: 10-Phenyl-5,5-dioxo-phenothiazin-3-carbaldehyd

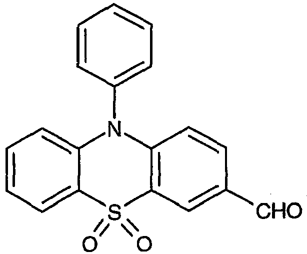

Eine Lösung von 1,01 g (3,33 mmol) 10-Phenylphenothiazin-3-carbaldehyd in 35 ml Methylenchlorid wurde portionsweise mit 1,48 g (6,6 mmol) 77 %iger m-Chlorperbenzoesäure bei Raumtemperatur versetzt und zwei Stunden nachgerührt. Die Reaktionslösung wurde jeweils zweimal mit 10 ml 10 % KOH, 10 ml 5 % HCl und 10 ml gesättigter Natriumhydrogencarbonatlösung ausgeschüttelt. Nach dem Entfernen des Lösungsmittels wurde der Feststoff (0,87 g) in einer Mischung aus 19,6 ml Methylen-chlorid und 0,4 ml Methanol gelöst und an Kieselgel gereinigt. Nach Entfernen des Lösungsmittels wurden 0,395 g gelbliche Mikrokristalle mit einem Schmelzpunkt von 260 - 272 °C erhalten.

### Beispiel 22: 10-Phenyl-3-(2-{4'-[2-(10-phenyl-5,5-dioxo-phenothiazin-3-yl)-vinyl]-biphenyl-4-yl}-vinyl)-phenothiazin-5,5-dioxid

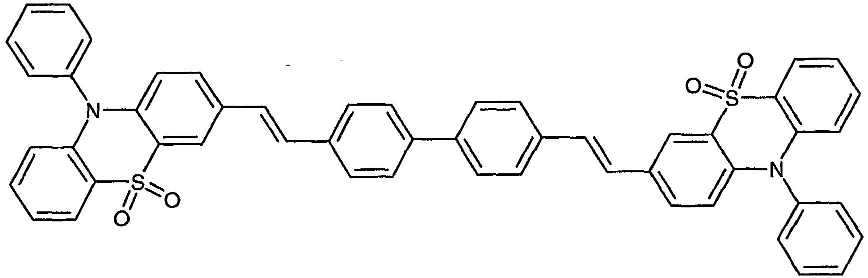

Zu einer Lösung von 4,00 g (8,80 mmol) [4'-(Diethoxyphosphorylmethyl)-biphenyl-4-yl-methyl]-phosphonsäurediethylester in 45 ml über Molekularsieb getrocknetem Dimethylsulfoxid wurden unter Stickstoff bei Raumtemperatur unter Rühren 2,28 g (19,9 mmol) Kalium-tert.-butylat und dann 5,90 g (17,6 mmol) 10-Phenyl-5,5-dioxo-phenothiazin-3-carbaldehyd (Beispiel 21) gegeben. Nach 72 h Rühren bei Raumtemperatur wurde die Reaktionslösung mit 200 ml Methanol verdünnt und 1 h nachgerührt. Der Niederschlag wurde abfiltriert, mit 500 ml Methanol gewaschen und bei 80 °C im Vakuum getrocknet. Das Rohprodukt (5,44 g) wurde in 250 ml o-Dichlorbenzol umkristallisiert. Das Kristallisat wurde abgesaugt, nacheinander mit o-Dichlorbenzol und Ethanol gewaschen, abgesaugt und bei 80 °C im Vakuum getrocknet. Nach dem Entfernen von Lösungsmittelresten im Hochvakuum (2 x 10⁻⁵ mbar) bei 250 °C wurden 4,35 g (60 % d. Th.) gelbe Mikrokristalle mit einem Schmelzpunkt von 392 °C erhalten, deren Lösung in Methylenchlorid bei λ = 456 nm fluoreszierte.

### Beispiel 23: 1,3-Phenylen-10,10'-bis(phenothiazin)

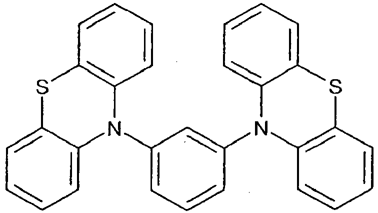

Die Herstellung erfolgte nach K. Okada et al., J. Am. Chem. Soc. 1996, 118, 3047 - 3048. 18,5 g (91,9 mmol) Phenothiazin, 15,6 g (46,3 mmol) 98 %iges 1,3-Diiodbenzol, 19,4 g (140 mmol) Kaliumcarbonat und 1,16 g (18,3 mmol) aktiviertes Kupferpulver wurden auf 200 °C erhitzt und 24 h bei dieser Temperatur gerührt. Die Reaktionsmischung wurde auf 140 °C abgekühlt und mit dann mit 200 ml Essigsäureethylester versetzt. Die Suspension wurde eine Stunde lang unter Rückfluss zum Sieden erhitzt und anschließend heiß filtriert. Das Filtrat wurde mit 300 ml Methanol verdünnt, wobei ein Niederschlag ausfiel, der abgesaugt, mit Methanol gewaschen und bei 80 °C im Vakuum getrocknet wurde. Es wurden 8,91 g rosafarbener Feststoff mit einem Schmelzpunkt von 186 - 188 °C erhalten.

### Beispiel 24: 1,3-Phenylen-10,10'-bis(phenothiazin-5,5-dioxid)

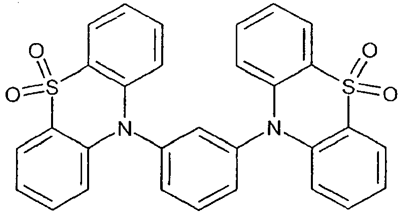

6,28 g (13,3 mmol) 1,3-Phenylen-10,10'-bis(phenothiazin) wurden in 220 ml Methylenchlorid gelöst. Nach 15 min Rühren bei Raumtemperatur wurden 17,9 g (79,9 mmol) 77 %ige m-Chlorperbenzoesäure portionsweise zugegeben. Die Reaktionslösung wurde 24 h bei Raumtemperatur gerührt, währenddessen ein Niederschlag ausfiel. Die Lösung wurde filtriert, und der Rückstand mit Methylenchlorid gewaschen und trocken gesaugt. Der Feststoff wurde in heißem Wasser suspendiert. Die wässrige Suspension wurde mit 5 %iger Kalilauge auf pH 11 gestellt und anschließend heiß filtriert. Der Rückstand wurde mit heißem Wasser gewaschen und bei 80 °C im Vakuum getrocknet. Der Feststoff (5,07 g) wurde aus Dimethylformamid umkristallisiert. Es wurden 3,72 g farblose Mikrokristalle mit einem Schmelzpunkt von 412 °C analysenrein erhalten, deren Lösung in Toluol bei λ = 375 nm (S) fluoreszierte.

### Beispiel 25: 1,4-Phenylen-10,10'-bis(phenothiazin-5,5-dioxid)

### a) 1,4-Phenylen-10,10'-bis(phenothiazin)

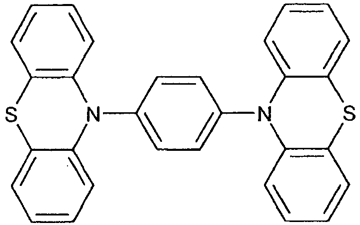

Die Herstellung erfolgte nach K. Okada et al., J. Am. Chem. Soc. 1996, 118, 3047 - 3048. 19,9 g (98,9 mmol) Phenothiazin, 16,6 g (49,8 mmol) 99 %iges 1,4-Diiodbenzol, 20,9 g (151 mmol) Kaliumcarbonat und 1,25 g (19,7 mmol) aktiviertes Kupferpulver wurden auf 196 °C erhitzt und 17 h bei dieser Temperatur gerührt. Nach Abkühlung der Reaktionsmischung auf Raumtemperatur wurden 200 ml heißes Wasser zugegeben. Die Suspension wurde eine Stunde lang gerührt und anschließend filtriert. Der Rückstand wurde mit heißem Wasser gewaschen und bei 80 °C im Vakuum getrocknet. Das Rohprodukt (21,6 g) wurden in 200 ml Methylenchlorid eine Stunde lang unter Rückfluss zum Sieden erhitzt. Nach Abkühlung der Lösung auf Raumtemperatur wurde diese über Kieselgel filtriert. Es wurden drei Fraktionen erhalten, von denen die beiden ersten vereinigt (11,7 g) und aus Essigsäureethylester umkristallisiert wurden. Die dritte Fraktion enthielt das gewünschte Wertprodukt (5,0 g). Insgesamt wurden 13,47 g beigefarbener Feststoff mit einem Schmelzpunkt von 254 - 263 °C erhalten.

### b) 1,4-Phenylen-10,10'-bis(phenothiazin-5,5-dioxid)

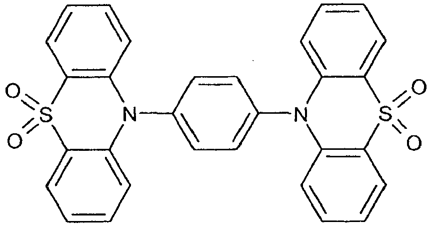

4,98 g (10,5 mmol) 1,4-Phenylen-10,10'-bis(phenothiazin) wurden in 175 ml Methylen-chlorid gelöst. Nach 1 h Rühren bei Raumtemperatur wurden 10,41 g (46,5 mmol) 77 %ige m-Chlorperbenzoesäure portionsweise zugegeben. Die Reaktionslösung wurde 24 h bei Raumtemperatur gerührt, währenddessen ein Niederschlag ausfiel. Die Lösung wurde filtriert, und der Rückstand mit Methylenchlorid gewaschen und trocken gesaugt. Der Feststoff wurde in 200 ml heißem Wasser suspendiert. Die wässrige Suspension wurde mit 5 ml 10 %iger Kalilauge auf pH 11,3 gestellt, 1 h gerührt und anschließend heiß filtriert. Der Rückstand wurde mit heißem Wasser gewaschen und bei 80 °C im Vakuum getrocknet. Der Feststoff (5,37 g) wurde zweimal aus Sulfolan umkristallisiert. Es wurden 2,87 g (51 %) schwach rosafarbene Mikrokristalle mit einem Schmelzpunkt von > 360 °C analysenrein erhalten, deren Lösung in Methylenchlorid bei λ = 480 nm fluoreszierte.

### Beispiel 26: 10-Methylphenothiazin-5,5-dioxid

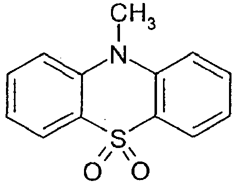

Die Herstellung erfolgte nach M. Tosa et al., Heterocyclic Commun. 7 (2001) 277 - 282.

Eine Lösung von 10,0 g (45,9 mmol) 98 %igem 10-Methylphenothiazin in 350 ml Methylenchlorid wurde bei Raumtemperatur mit 22,65 g (91,9 mmol) 70 %iger m-Chlorperbenzoesäure versetzt und 5 h bei 20 - 25 °C gerührt. Nach Filtration der Lösung wurde das Filtrat nacheinander zweimal mit jeweils 100 ml 10 %iger Kalilauge, 100 ml 5 %iger Salzsäure und 70 ml gesättigter Natriumhydrogencarbonat ausgeschüttelt. Die organische Phase wurde auf 150 ml eingeengt und dann über Kieselgel filtriert. Aus der zweiten Fraktion wurden 4,89 g (43 % d. Th.) beigefarbene Mikrokristalle mit einem Schmelzpunkt von 226 - 235 °C (Lit. 225 - 226 °C) analysenrein isoliert. Umkristallisation in Essigsäure lieferte farblose Kristalle, die bei 226 - 233 °C schmolzen. Eine Lösung der Substanz in Chloroform fluoreszieile bei λ = 351, 376 (S) nm.

### Beispiel 27: 10-Phenylphenothiazin-5,5-dioxid

### a) 10-Phenylphenothiazin

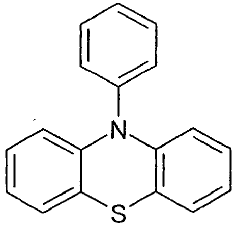

Die Herstellung erfolgte nach D. Li et al., Dyes and Pigments 49 (2001) 181 - 186. 96,0 g (482 mmol) Phenothiazin, 298,5 g (1434 mmol) 98 %iges Iodbenzol, 80,0 g (579 mmol) Kaliumcarbonat und 2,00 g (31,5 mmol) Kupferpulver wurden auf 190 - 200 °C erhitzt und 6 h bei dieser Temperatur gerührt. Anschließend wurde das überschüssige Iodbenzol abdestilliert. Die Reaktionsmischung wurde mit 480 ml Ethanol verdünnt und 1 h lang unter Rückfluss zum Sieden erhitzt. Die Lösung wurde heiß filtriert. Nach dem Abkühlen wurde der Niederschlag abgesaugt, mit Ethanol gewaschen und im Vakuum getrocknet. Es wurden 77,7 g (58,5 % d. Th.) graue Mikrokristalle mit einem Schmelzpunkt von 95 - 96 °C (Lit. 95 - 97 °C) erhalten.

### b) 10-Phenylphenothiazin-5,5-dioxid

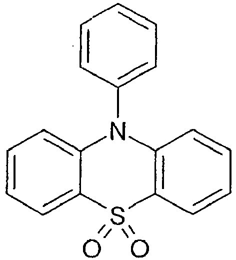

Die Herstellung der literaturbekannten Verbindung (H. Gilman und R. O. Ranck, J. Org. Chem. 1958, 23, 1903 - 1906) erfolgte in Analogie zu der von M. Tosa et al., Heterocyclic Commun. 7 (2001) 277 - 282.

Eine Lösung von 5,50 g (20,0 mmol) 10-Phenylphenothiazin in 220 ml Methylenchlorid wurde bei Raumtemperatur mit 11,84 g (48,0 mmol) 70 %iger m-Chlorperbenzoesäure versetzt und 8 h bei 20 - 25 °C gerührt. Die Lösung wurde bis zur Trockne eingeengt. Der Rückstand wurde in heißem Wasser suspendiert und auf 80 - 85 °C erwärmt. Bei dieser Temperatur wurde der pH mit 32 ml 10 %iger Kalilauge auf 7 - 8 gestellt. Die Lösung wurde 30 min nachgerührt, anschließend filtriert, mit heißem Wasser gewaschen und im Vakuum bei 80 °C getrocknet, Das Rohprodukt (5,77 g) wurde in 30 ml Methylenchlorid gelöst und über Kieselgel filtriert. Aus der zweiten Fraktion wurden 2,92 g (47 % d. Th.) beigefarbene Mikrokristalle mit einem Schmelzpunkt von 212 - 217 °C (Lit. 212 - 213 °C) analysenrein isoliert. Umkristallisation in Essigsäure lieferte farblose Kristalle, die bei 212 - 217 °C schmolzen. Eine Lösung der Substanz in Chloroform fluoreszierte bei λ = 348, 386 (S), 452 (S) nm.

### Beispiel 28: 10-(4-Methoxyphenyl)-phenothiazin-5,5-dioxid

### a) 10-(4-Methoxyphenyl)-phenothiazin

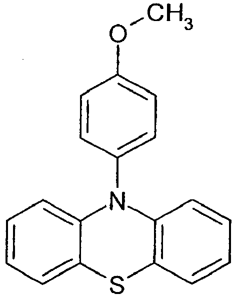

18,77 g (94,2 mmol) Phenothiazin, 66,5 g (284 mmol) 98 %iges 4-Iodanisol, 15,7 g (114 mmol) Kaliumcarbonat und 0,392 g (6,17 mmol) Kupferpulver wurden auf 190 - 200 °C erhitzt und 48 h bei dieser Temperatur gerührt. Anschließend wurde das überschüssige Iodbenzol abdestilliert. Die Reaktionsmischung wurde mit 200 ml heißem Wasser versetzt und 1 h lang bei 90 °C erhitzt. Die Lösung wurde heiß filtriert. Nach dem Abkühlen wurde der Niederschlag abgesaugt, mit Ethanol gewaschen und im Vakuum getrocknet. Das Rohprodukt (29,0 g) wurde aus 345 ml Essigsäure umkristallisiert. Es wurden 22,54 g (78,4 % d. Th.) beigefarbene Mikrokristalle mit einem Schmelzpunkt von 173 - 176 °C (Lit. 172 - 174 °C (E. R. Biehl et al., J. Heterocyclic Chem. 12 (1975) 397 - 399)) erhalten.

### b) 10-(4-Methoxyphenyl)phenothiazin-5,5-dioxid

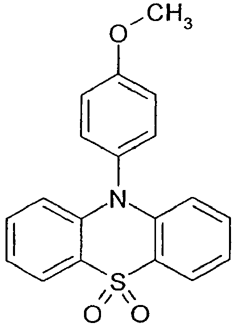

Eine Lösung von 5,00 g (16,4 mmol) 10-(Methoxyphenyl)-phenothiazin in 175 ml Methylenchlorid wurde bei Raumtemperatur mit 9,76 g (39,6 mmol) 70 %iger m-Chlorperbenzoesäure versetzt und 4 h bei 20 - 25 °C gerührt. Die Lösung wurde bis zur Trockne eingeengt. Der Rückstand wurde mit 200 ml heißem Wasser aufgenommen und auf 80 - 85 °C erwärmt. Bei dieser Temperatur wurde der pH mit 25 ml 10 %iger Kalilauge auf 7 - 8 gestellt. Die Lösung wurde 30 min nachgerührt, anschließend filtriert, mit heißem Wasser gewaschen und im Vakuum bei 80 °C getrocknet. Das Rohprodukt (5,25 g) wurde in 70 ml Methylenchlorid gelöst und über Kieselgel filtriert. Nach Entfernen des Lösungsmittels wurden 4,41 g (80 % d. Th.) farblose Mikrokristalle mit einem Schmelzpunkt von 265 - 266 °C analysenrein isoliert. Umkristallisation in Essigsäure lieferte farblose Kristalle, die bei 264 - 270 °C schmolzen. Eine Lösung der Substanz in Chloroform fluoreszierte bei λ = 474 nm.

### Beispiel 29: 10-Mesitylphenothiazin-5,5-dioxid

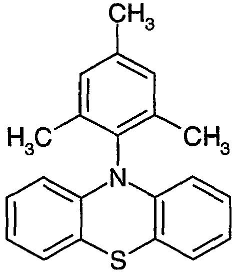

9,92 g (49,8 mmol) Phenothiazin, 25,0 g (99,6 mmol) 98 %iges 2,4,6-Trimethyliodbenzol, 8,30 g (60,0 mmol) Kaliumcarbonat und 0,207 g (3,26 mmol) Kupferpulver wurden auf 180 °C erhitzt und 24 h bei dieser Temperatur gerührt. Anschließend wurde das überschüssige 2,4,6-Trimethyliodbenzol abdestilliert. Die Reaktionsmischung wurde mit 300 ml Wasser versetzt und über Nacht gerührt. Die Suspension wurde filtriert, mit heißem Wasser neutral gewaschen und im Vakuum bei 80 °C getrocknet. Das Rohprodukt (16,6 g) wurde in 500 ml Ethanol 2 h lang unter Rückfluss zum Sieden erhitzt und dann mit 200 ml Wasser verdünnt. Der Niederschlag wurde abgesaugt, im Vakuum bei 80 °C getrocknet (10,5 g) und in 150 ml Toluol gelöst. Die Lösung wurde über Kieselgel filtriert. Nach Einengen des Filtrats wurden 7,22 g hellbraune Mikrokristalle mit einem Schmelzpunkt von 192 - 200 °C erhalten.

### b) 10-Mesitylphenothiazin-5,5-dioxid

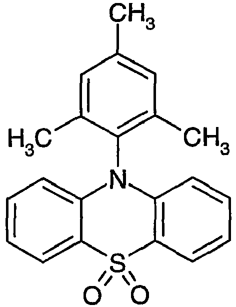

Eine Lösung von 1,50 g (4,73 mmol) 10-Mesitylphenothiazin in 55 ml Methylenchlorid wurde bei Raumtemperatur mit 2,80 g (11,4 mmol) 70 %iger m-Chlorperbenzoesäure versetzt und 8 h bei 20 - 25 °C gerührt. Die Lösung wurde nacheinander zweimal mit jeweils 20 ml 10 %iger Kalilauge, 20 ml 5 %iger Salzsäure und 15 ml gesättigter Natriumhydrogencarbonatlösung ausgeschüttelt. Die Lösung wurde über Kieselgel filtriert. Nach Einengencarbonatlösung ausgeschüttelt. Die Lösung wurde über Kieselgel filtriert. Nach Einengen des Filtrats wurden 1,43 g (86 % d. Th.) beigefarbene Mikrokristalle mit einem Schmelzpunkt von 242 - 246 °C analysenrein isoliert. Umkristallisation in Essigsäure lieferte farblose Kristalle, die bei 240-246 °C schmolzen. Eine Lösung der Substanz in Chloroform fluoreszierte bei λ = 349, 370 (S) nm.

### Beispiel 30: 1,3,5-Phenylen-10,10',10"-tris(phenothiazin-5,5-dioxid)

### a) 1,3,5-Phenylen-10,10',10"-tris(phenothiazin)

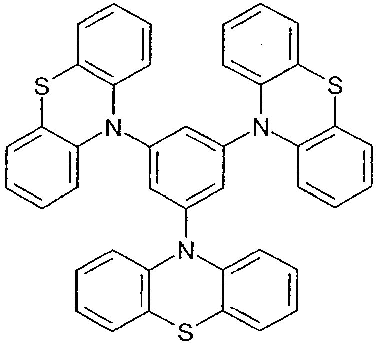

Zu einer Suspension von 6,00 g (150 mmol) Natriumhydrid (60 %ige Dispersion in Parafinöl) in 150 ml wasserfreiem Dimethylformamid wurden unter Rühren und Stickstoff 30,19 g (150 mmol) Phenothiazin bei Raumtemperatur gegeben, wobei die Reaktionstemperatur auf 40 °C anstieg. Nach Ende der Wasserstoffentwicklung (ca. 20 min) wurde eine Lösung von 6,20 g (46,0 mmol) 98 %iges 1,3,5-Trifluorbenzol in 10 ml Dimethylformamid innerhalb von 15 min zur Reaktionslösung getropft. Anschließend wurde die Reaktionslösung zunächst zwei Stunden bei 80 °C, dann 16 Stunden bei 100 °C erhitzt. Nach dem Abkühlen auf Raumtemperatur wurde die Reaktionslösung in 500 ml Eiswasser gefällt. Der Niederschlag wurde abgesaugt, mit heißem Wasser neutral gewaschen und dann in 500 ml Methanol dispergiert. Die Suspension wurde eine Stunde lang zum Sieden unter Rückfluss erhitzt. Nach dem Abkühlen auf Raumtemperatur wurde der Feststoff abgesaugt, mit Methanol gewaschen und bei 50 °C im Vakuum getrocknet. Es wurden 24,80 g Feststoff erhalten, die in Essigester eine Stunde lang unter Rückfluss zum Sieden erhitzt wurden. Nach dem Abkühlen auf Raumtemperatur wurde der Feststoff abgesaugt, mit Essigester gewaschen und noch einmal in Essigester eine Stunde lang unter Rückfluss zum Sieden erhitzt. Nach dem Abkühlen auf Raumtemperatur wurde der Feststoff abgesaugt, mit Essigester gewaschen und

### b) 1,3,5-Phenylen-10,10',10"-tris(phenothiazin-5,5-dioxid)

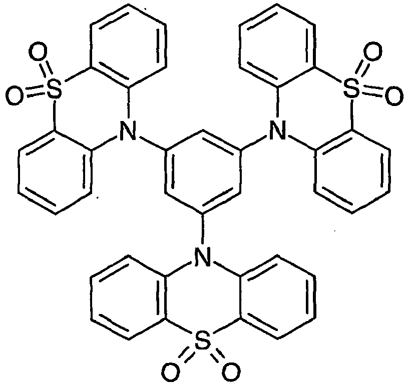

Eine Lösung von 6,70 g (10,0 mmol) 1,3,5-Phenylen-10,10',10"-tris(phenothiazin) in 180 ml Methylenchlorid wurde bei Raumtemperatur portionsweise mit 22,19 g (90,0 mmol) 70 %iger m-Chlorperbenzoesäure versetzt und 24 h bei 20 - 25 °C gerührt. Die Reaktionsmischung wurde bis zur Trockne eingeengt, dann mit 150 ml heißem Wasser und 46 ml 10 %iger Kalilauge versetzt. Der Feststoff wurde abgesaugt, mit heißem Wasser neutral gewaschen und bei 80 °C im Vakuum getrocknet. Es wurden 7,63 g beigefarbene Mikrokristalle mit einem Schmelzpunkt von > 360 °C erhalten.

### Beispiel 31: Benzoesäure-4-(5,5-dioxo-phenothiazin-10-yl)-phenylester

### a) 10-(2-Hydroxyphenyl)-phenothiazin

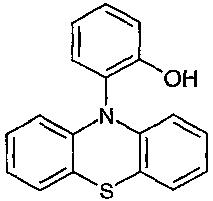

35.9 g (180 mmol) Phenothiazin, 44.5 g (198 mmol) 98 %iges 4-Jodphenol (2-Jodphenol kann ebenfalls eingesetzt werden.), 29.9 g (216 mmol) Kaliumcarbonat und 0.75 g (12 mmol) Kupferpulver wurden auf 198 °C erhitzt und 3.5 h bei dieser Temperatur gerührt. Die Reaktionsschmelze wurde auf 140 °C abgekühlt und dann mit 150 ml Wasser innerhalb von 3 min versetzt, wobei die Reaktionsmischung erstarrte. Nach dem Abkühlen mit Trockeneis wurde der Feststoff isoliert, in einer Reibschale zerkleinert und mit 150 ml Wasser versetzt. Die Suspension wurde einer Wasserdampfdestillation unterworfen, um überschüssiges Jodphenol zu entfernen. Anschließend wurde der Feststoff abgesaugt und mit Wasser gewaschen. Der wasserfeuchte Feststoff wurde in 400 ml Ethanol suspendiert, über Nacht bei Raumtemperatur gerührt, dann abgesaugt, mit Ethanol gewaschen und im Vakuum bei 80 °C getrocknet. Das Rohprodukt (16.6 g) wurde in 500 ml Ethanol 2 h lang unter Rückfluss zum Sieden erhitzt und dann mit 200 ml Wasser verdünnt. Der Niederschlag wurde abgesaugt, im Vakuum bei 80 °C getrocknet (10.5 g) und in 150 ml Toluol gelöst. Die Lösung wurde über Kieselgel filtriert. Nach Einengen des Filtrats wurden 7.22 g hellbraune Mikrokristalle mit einem Schmelzpunkt von 192 - 200 °C erhalten.

### b) Benzoesäure-2-(phenothiazin-10-yl)-phenylester

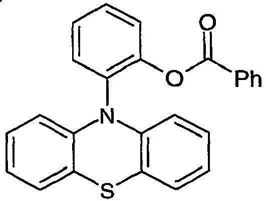

Zu einer Lösung von 4.00 g (13.7 mmol) 10-(2-Hydroxyphenyl)-phenothiazin in 24 ml Pyridin wurden bei 0 - 5 °C innerhalb von 30 min 16.0 g (114 mmol) Benzoylchlorid unter Rühren zugetropft. Nach 2 h Rühren bei Raumtemperatur wurde die Reaktionslösung auf 60 - 65 °C erwärmt und 15 min bei dieser Temperatur gerührt. Nach dem Abkühlen auf Raumtemperatur wurde die Suspension noch über Nacht gerührt. Nach Zugabe von 300 ml Eiswasser wurde die Suspension langsam mit 19 ml konz. Salzsäure versetzt (pH 0.9) und 1 h lang gerührt. Die Suspension wurde über eine Glasfritte filtriert. Der Rückstand wurde mit 21 Wasser neutral gewaschen und bei 60 °C im Vakuum getrocknet. Es wurden 2.87 g (53 % d. Th.) farblose Mikrokristalle mit einem Schmelzpunkt von 144-148 °C erhalten.

### c) Benzoesäure-2-(5,5-dioxo-phenothiazin-10-yl)-phenylester

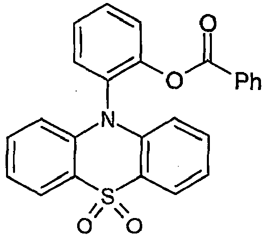

Eine Lösung von 1.32 g (3.33 mmol) Benzoesäure-2-(phenothiazin-10-yl)-phenylester in 50 ml Methylenchlorid wurde bei Raumtemperatur mit 1.81 g (7.33 mmol) 70 %iger m-Chlorperbenzoesäure versetzt und 24 h bei 20 °C gerührt. Die Lösung wurde im Vakuum bis zur Trockne eingeengt. Der Rückstand wurde in 50 ml Wasser aufgenommen. Die Suspension wurde auf 80 °C erwärmt und nach Zugabe von 4.5 ml 10 %iger Kalilauge 20 min gerührt. Der beigefarbene Feststoff wurde heiß abgesaugt, mit heißem Wasser gewaschen und bei 70 °C getrocknet (1.285 g). Eine Lösung des Feststoffs in 22 ml Methylenchlorid wurde über Kieselgel MN 60 filtriert, wobei mit einem Gemisch aus 100 Teilen Methylenchlorid und 1 Teil Methanol nachgewaschen wurde. Nach Einengen des Filtrats wurde der Rückstand aus Essigsäure umkristallisiert. Es wurden 0.93 g (65 % d. Th.) farblose Mikrokristalle erhalten, die bei 228 - 232 °C schmolzen.

### Beispiel 32: 10-(2-Hydroxyphenyl)-phenothiazin-5,5-dioxid

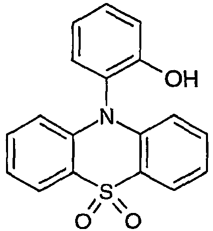

Eine Lösung von 1.50 g (5.14 mmol) 10-(2-Hydroxyphenyl)-phenothiazin in 50 ml Methylenchlorid wurde bei Raumtemperatur mit 2.79 g (11.31 mmol) 70 %iger m-Chlorperbenzoesäure versetzt und 5.5 h bei Raumtemperatur gerührt. Die Lösung wurde im Vakuum bis zur Trockne eingeengt. Der Rückstand wurde in 100 ml Wasser aufgenommen. Die Suspension wurde auf 80 °C erwärmt und nach Zugabe von 7.5 ml 10 %iger Kalilauge 20 min gerührt. Der Feststoff wurde heiß abgesaugt, mit heißem Wasser gewaschen und bei 70 °C getrocknet (1.45 g). Der beigefarbene Feststoff wurde zweimal aus jeweils 42 ml Essigsäure umkristallisiert. Es wurden 0.92 g (55 % d. Th.) farblose Mikrokristalle erhalten, die bei 282 - 287 °C schmolzen.

### Beispiel 33: Benzoesäure-4-(5,5-dioxo-phenothiazin-10-yl)-phenylester

### a) (4-Iodphenyl)-benzylether

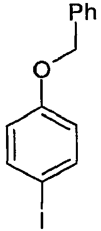

Eine Reaktionsmischung aus 21.40 g (95.3 mmol) 98 %iges 4-Iodphenol, 12.19 g (95.3 mmol) 99 %iges Benzylchlorid, 20.73 g (150 mmol) Kaliumcarbonat und 250 ml Aceton wurden auf Rückflusstemperatur erwärmt und 30 Stunden lang zum Sieden erhitzt. Nach dem Abkühlen auf Raumtemperatur wurde die Reaktionsmischung filtriert. Das Filtrat wurde eingeengt und anschließend im Eisbad gekühlt, wobei ein Niederschlag ausfiel. Dieser wurde über ein Blaubandfilter abgetrennt und dann getrocknet. Das Rohprodukt (23.22 g) wurde aus 70 ml Ethanol umkristallisiert. Es wurden 17.20 g (58 % d. Th.) farblose Mikrokristalle mit einem Schmp. von 61 - 62 °C (Lit. 62 °C) erhalten.

### b) 10-(4-Benzyloxyphenyl)-phenothiazin

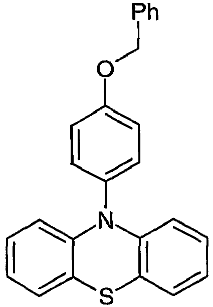

5.56 g (27.9 mmol) Phenothiazin, 8.65 g (27.9 mmol) (4-Iodphenyl)-benzylether, 4.64 g (33.5 mmol) Kaliumcarbonat und 0.116 g (1.82 mmol) Kupferpulver wurden auf 190 °C erhitzt und 24 h bei dieser Temperatur gerührt. Die Reaktionsschmelze wurde auf 110 °C abgekühlt, dann mit 200 ml Toluol verdünnt und eine Stunde lang bei 112 °C gerührt. Die Lösung wurde heiß filtriert. Das auf Raumtemperatur abgekühlte Filtrat wurde an Kieselgel in Toluol gereinigt. Das beigefarbene Rohprodukt (6.52 g) wurde aus 125 ml Ethanol umkristallisiert. Es wurden 4.79 g (45 % d. Th.) beigefarbene Mikrokristalle mit einem Schmp. von 144-146 °C erhalten.

### c) 10-(4-Benzyloxyphenyl)-phenothiazin-5,5-dioxid

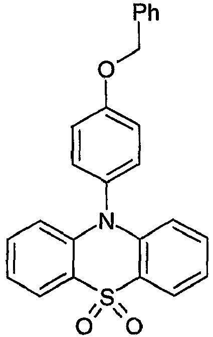

Eine Lösung von 4.60 g (12.1 mmol) 10-(4-Benzyloxyphenyl)-phenothiazin in 130 ml Methylenchlorid wurde bei Raumtemperatur mit 6.53 g (26.5 mmol) 70 %iger m-Chlorperbenzoesäure versetzt und 3 h bei Raumtemperatur gerührt. Die Lösung wurde im Vakuum bis zur Trockne eingeengt. Der Rückstand wurde in 150 ml Wasser aufgenommen. Die Suspension wurde auf 80 °C erwärmt und nach Zugabe von 14 ml 10 %iger Kalilauge 20 min gerührt. Der Feststoff wurde heiß abgesaugt, mit heißem Wasser gewaschen und bei 100 °C getrocknet. Es wurden 4.78 g (96 % d. Th.) beigefarbener Feststoff mit einem Schmp. von 203-208 °C erhalten. Um Methylenchloridreste zu entfernen, wurden 0.96 g Feststoff im Hochvakuum bei 200 °C sublimiert. Es wurden 0.78 g analysenreine farblose Mikrokristalle erhalten, die bei 204-208 °C schmolzen.

### d) 10-(4-Hydroxyphenyl)-phenothiazin-5,5-dioxid

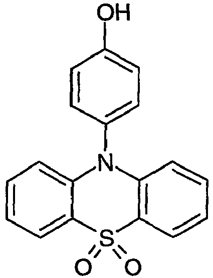

3.10 g (7.50 mmol) 10-(4-Benzyloxyphenyl)-phenothiazin-5,5-dioxid, 2.30 g (35.7 mmol) 98 %iges Ammoniumformiat und 7.5 g 10 %iges Palladium auf Aktivkohle wurden in 225 ml Aceton eine Stunde lang unter Rückfluss zum Sieden erhitzt. Nach dem Abkühlen auf Raumtemperatur wurde die Lösung filtriert. Das Filtrat wurde eingeengt und nach Zugabe von 10 ml Methanol über Nacht gerührt. Der Feststoff wurde abgesaugt, mit Methanol gewaschen und bei 110 °C im Vakuumtrockenschrank getrocknet. Es wurden 1.47 g (61 % d. Th.) analysenreine hellgraue Mikrokristalle mit einem Schmp. von 308-311 °C erhalten.

### e) Benzoesäure-4-(5,5-dioxo-phenothiazin-10-yl)-phenylester

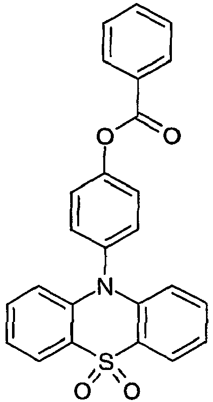

Zu einer Lösung von 0.72 g (2.22 mmol) 10-(4-Hydroxyphenyl)-phenothiazin-5,5-dioxid in 120 ml Acetonitril wurden 0.68 g (6.68 mmol) Triethylamin und 0.34 g (2.44 mmol) Benzoylchlorid gegeben. Nach 45 min. Rühren bei Raumtemperatur wurde das Lösungsmittel abdestilliert. Der Rückstand wurde in 100 ml heißem Wasser aufgenommen und 30 min bei 75 °C gerührt. Der Feststoff wurde heiß abgesaugt, mit heißem Wasser gewaschen und bei 120 °C im Umlufttrockenschrank getrocknet. Es wurden 0.87 g (92 % d. Th.) farblose Mikrokristalle mit einem Schmp. von 233-235 °C erhalten.

### Beispiel 34: 10-(3,5-Difluorphenyl)-phenothiazin-5,5-dioxid

### a) 10-(3,5-Difluorphenyl)-phenothiazin

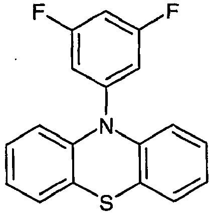

Zu einer Suspension von 2.00 g (50.0 mmol) Natriumhydrid (60 %ige Dispersion in Parafinöl) in 100 ml wasserfreiem Dimethylformamid wurden unter Rühren und Stickstoff 10.07 g
(50.0 mmol) Phenothiazin innerhalb von 10 min bei Raumtemperatur gegeben, wobei die Reaktionstemperatur auf 32 °C anstieg. Nach Ende der Wasserstoffentwicklung (ca. 20 min) wurde eine Lösung von 7.41 g (55.0 mmol) 98 %iges 1,3,5-Trifluorbenzol in 50 ml Dimethylformamid innerhalb von 15 min in die auf 85 °C erhitzte Reaktionslösung getropft. Anschließend wurde die Reaktionslösung 24 Stunden lang bei dieser Temperatur gerührt. Nach dem Abkühlen auf Raumtemperatur wurde die Reaktionslösung langsam in 500 ml Eiswasser gefällt. Die wässrige Suspension wurde nach Zugabe von 18 g Natriumchlorid 2 h gerührt und anschließend filtriert. Der Rückstand wurde mit Wasser gewaschen und getrocknet. Der Feststoff wurde in 200 ml Hexan suspendiert und 1 h unter Rückfluss erhitzt. Nach Abkühlen auf Raumtemperatur wurde die Suspension filtriert. Das Filtrat wurde im Vakuum bis zur Trockne eingeengt. Der Rückstand (7.00 g) wurde in 230 ml Gemisch aus 40 Teilen Hexan und 1 Teil Essigester aufgerührt und filtriert. Das Filtrat wurde an Kieselgel mit einem Laufmittel aus 40 Teilen Hexan und 1 Teil Essigester chromatographiert. Nach Entfernen des Lösungsmittels wurde der Rückstand bei 80 °C und 1.8 x 10⁻⁵ mbar getrocknet, wobei ein Teil der Substanz sublimierte. Der in der Vorlage zurückgebliebene Feststoff wurde aus 40 ml Ethanol umkristallisiert. Es wurden 1.18 g (7.6 % d. Th.) analysenreine farblose Mikrokristalle mit einem Schmelzpunkt von 111-115 °C erhalten.

### b) 10-(3,5-Difluorphenyl)-phenothiazin-5,5-dioxid

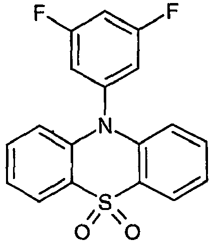

Eine Lösung von 1.40 g (4.50 mmol) 10-(3,5-Difluorphenyl)-phenothiazin in 40 ml Methylenchlorid wurde bei Raumtemperatur mit 2.46 g (10.0 mmol) 70 %iger m-Chlorperbenzoesäure versetzt und 2 h bei Raumtemperatur gerührt. Die Lösung wurde im Vakuum bis zur Trockne eingeengt. Der Rückstand wurde in 100 ml 50 °C warmem Wasser 30 min gerührt. Die Suspension wurde mit 7.5 ml 10 %iger Kalilauge versetzt, 30 min gerührt und dann filtriert. Der Feststoff wurde heiß abgesaugt, mit heißem Wasser gewaschen und bei 80 °C getrocknet. Der beigefarbene Feststoff (1.54 g) wurde zweimal aus Essigsäure umkristallisiert. Nach dem Trocknen bei 130 °C im Hochvakuum wurden 0.73 g (47 % d. Th.) analysenreine farblose Mikrokristalle erhalten, die bei 255-258 °C schmolzen.

### Beispiel 35: 10-(2-Pyridyl)-phenothiazin-5,5-dioxid

### a) 10-(2-Pyridyl)-phenothiazin

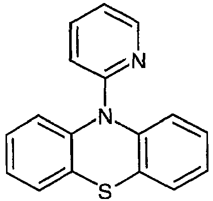

4.46 g (22.4 mmol) Phenothiazin, 9.36 g (47.6 mmol) 98 %iges 2-Jodpyridin, 3.72 g (26.9 mmol) Kaliumcarbonat und 0.093 g (1.5 mmol) Kupferpulver wurden auf 192 °C erhitzt und 24 h bei dieser Temperatur gerührt. Die Reaktionsschmelze wurde auf 100 °C abgekühlt, dann langsam mit 200 ml Ethanol verdünnt und eine Stunde lang unter Rückfluss zum Sieden erhitzt. Nach dem Abkühlen auf Raumtemperatur wurde die Reaktionsmischung filtriert, wobei eine klebrige Paste zurückblieb, die in 100 ml Methylenchlorid gelöst wurde. Die Methylenchloridlösung wurde an Kieselgel gereinigt, wobei zwei Fraktionen erhalten wurden. Die zweite Fraktion wurde bis zur Trockne eingeengt. Es wurden 2.55 g (41 % d. Th.) beigefarbene Mikrokristalle mit einem Schmelzpunkt von 107-109 °C (Lit.: 109-110°C) erhalten.

### b) 10-(2-Pyridyl)-phenothiazin-5,5-dioxid

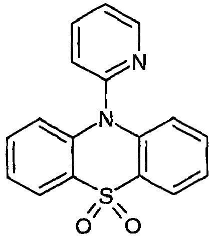

Eine Lösung von 2.20 g (7.96 mmol) 10-(2-Pyridyl)-phenothiazin in 80 ml Methylenchlorid wurde bei Raumtemperatur unter Kühlung mit 4.32 g (17.5 mmol) 70 %iger m-Chlorperbenzoesäure versetzt und 2 h bei Raumtemperatur gerührt. Die Lösung wurde im Vakuum bis zur Trockne eingeengt. Der Rückstand wurde in 200 ml 70 °C heißem Wasser aufgenommen und mit 10 ml 10 % KOH versetzt. Nach 30 min Rühren wurde die Suspension filtriert. Der Rückstand wurde mit heißem Wasser gewaschen und bei 50 °C im Vakuum getrocknet. Der farblose Feststoff (1.87 g) wurde in 20 ml eines Gemisches aus 99 Teilen Methylenchlorid und einem Teil Methanol gelöst und an Kieselgel gereinigt. Die gereingte Lösung wurde bis zur Trockne eingeengt. Nach dem Trocknen des Rückstands bei 70 °C im Vakuum wurde dieser aus 10 ml Essigsäure umkristallisiert. Es wurden 1.09 g (44 % d. Th.) analysenreine farblose Mikrokristalle erhalten, die bei 186 - 190 °C, schmolzen. Nach längerem Stehen des essigsauren Filtrats bei Raumtemperatur fielen noch 0.32 g farblose Mikrokristalle mit einem Schmelzpunkt von 184 - 188 °C aus (Gesamtausbeute: 57 %).

### Beispiel 36: 10-[4-(N-Phenyl-2-benzimidazolyl)-phenyl]-phenothiazin-5,5-dioxid

### a) N-Phenyl-2-(4-iodphenyl)-benzimidazol

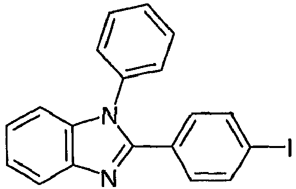

37.47 g (136 mmol) 97 %iges 4-Iodbenzoylchlorid und 12.82 g (68.2 mmol) 98 %iges o-Aminodiphenylamin wurden auf 100 °C erhitzt, wobei ab 85 - 90 °C eine rührfähig Schmelze entstand. Nach Beendigung der Gasentwicklung (5 min) erstarrte die Schmelze. Die Reaktionsmasse wurde noch 3 Stunden bei 100 °C gehalten. Nach dem Abkühlen wurde diese mit 100 ml Ethanol unter Rühren versetzt. Der Niederschlag wurde abgesaugt, mit Ethanol gewaschen und erneut in 400 ml Ethanol aufgerührt. Die Suspension wurde auf 75 °C erwärmt, wobei eine Lösung entstand, die mit 29 ml 25 % Ammoniak auf pH 8 gestellt wurde. Nach dem Abkühlen auf 5-10 °C wurde der Niederschlag abgesaugt, mit kaltem Ethanol gewaschen und bei 75 °C im Vakuumtrockenschrank getrocknet. Es wurden 18.37 g (68 % d. Th.) hellgraue Mikrokristalle mit einem Schmp. von 178-181 °C erhalten.

### b) 10-[4-(N-Phenyl-2-benzimidazolyl)-phenyl]-phenothiazin

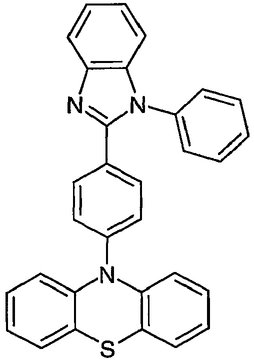

7.77 g (39.0 mmol) Phenothiazin, 17.00 g (42.9 mmol) N-Phenyl-2-(4-iodphenyl)-benzimidazol, 6.47 g (46.8 mmol) Kaliumcarbonat und 0.162 g (2.54 mmol) Kupferpulver wurden auf 195 - 200 °C erhitzt und 19 h bei dieser Temperatur gerührt. Die Reaktionsschmelze wurde auf 130 °C abgekühlt und dann mit 100 ml Ethanol verdünnt. Nach 30 min. Erhitzen unter Rückfluss wurde die Lösung heiß filtriert. Die Suspension wurde bis zur Trockne eingeengt und dann mit 150 ml Methylenchlorid versetzt. Nach Filtration wurde das Filtrat durch Kieselgel filtriert. Es wurden 11.03 g beigefarbene Mikrokristalle erhalten.

### c) 10-[4-(N-Phenyl-2-benzimidazolyl)-phenyl]-phenothiazin-5,5-dioxid

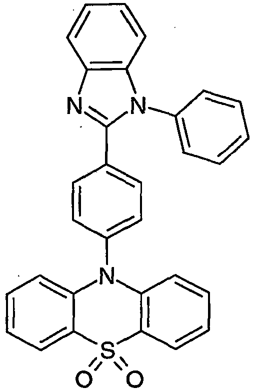

Eine Lösung von 6.14 g 10-[4-(N-Phenyl-2-benzimidazolyl)-phenyl]-phenothiazin in 180 ml Methylenchlorid wurde bei Raumtemperatur unter Kühlung mit 7.17 g (28.9 mmol) 70 %iger m-Chlorperbenzoesäure versetzt und 1 h bei Raumtemperatur gerührt. Die Lösung wurde mit 60 ml 10 % KOH versetzt. Nach Entfernung des Methylenchlorids wurde die Suspension mit 100 ml heißem Wasser verdünnt. Der Niederschlag wurde abgesaugt, mit heißem Wasser gewaschen und bei 80 °C im Vakuum getrocknet. Das Rohprodukt (4.82 g) wurde aus 48 ml Essigsäure umkristallisiert. Es wurden 3.02 g (46 % d. Th.) beigefarbene Mikrokristalle mit einem Schmp. von 286-288 °C erhalten.

### Beispiel 37: 10-Mesityl-3-(2-{4'-[2-(10-mesityl-5,5-dioxo-phenothiazin-3-yl)-vinyl]-biphenyl-4-yl}-vinyl)-phenothiazin-5,5-dioxid

### a) 10-Mesityl-phenothiazin-3-carbaldehyd

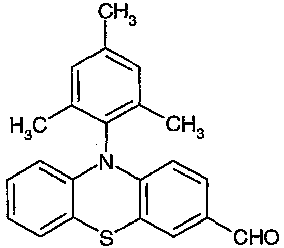

Zu einer Lösung von 5.00 g (15.8 mmol) 10-Mesitylphenothiazin und 3.23 g (23.6 mmol) 99 %igem N-Methylformanilid in 5 ml o-Dichlorbenzol wurden bei 20 °C unter Rühren und Eiskühlung innerhalb von 15 min 3.66 g (23.6 mmol) 99 %iges Phosphoroxychlorid getropft. Nach 30 min Rühren bei 20 °C wurde die Reaktionslösung auf 70 °C erwärmt und 4 Stunden lang bei dieser Temperatur gehalten. Die Reaktionslösung wurde mit einer Lösung von 16 g Natriumacetat in 35 ml Wasser innerhalb von 5 min versetzt. Nach 1 Stunde Rühren bei
70 °C wurde N-Methylformanilid und o-Dichlorbenzol mit einer Wasserdampfdestillation entfernt. 250 ml Methylenchlorid wurden zum Destillationssumpf gegeben. Die organische Phase wurde abgetrennt und durch Kieselgel filtriert. Das Filtrat wurde eingeengt und bei 40 °C im Vakuum getrocknet. Es wurden 5.05 g (93 % d. Th.) analysenreines grünliches Pulver mit einem Schmelzpunkt von 136-140 °C erhalten.

### b) 10-Mesityl-5,5-dioxo-phenothiazin-3-carbaldehyd

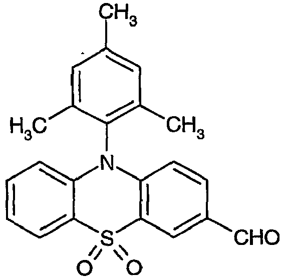

Eine Lösung von 4.00 g (11.6 mmol) 10-(Mesityl)-phenothiazin-3-carbaldehyd in 150 ml Methylenchlorid wurde bei Raumtemperatur mit 6.28 g (25.5 mmol) 70 %iger m-Chlorperbenzoesäure portionsweise versetzt und 3 h bei Raumtemperatur gerührt. Die Lösung wurde im Vakuum bis zur Trockne eingeengt. Der Rückstand wurde in 25 ml eines Lösungsmittelgemischs aus 98 Teilen Methylenchlorid und 2 Teilen Methanol gelöst und säulenchromatographisch an Kieselgel gereinigt. Die Lösung wurde bis zur Trockne eingeengt und bei 50 °C im Vakuum getrocknet. Es wurden 2.75 g (63 % d. Th.) gelbliche Mikrokristalle erhalten, die bei 194-197 °C schmolzen.

### c) 10-Mesityl-3-(2-{4'-[2-(10-mesityl-5,5-dioxo-phenothiazin-3-yl)-vinyl]-biphenyl-4-yl}-vinyl)-phenothiazin-5,5-dioxid

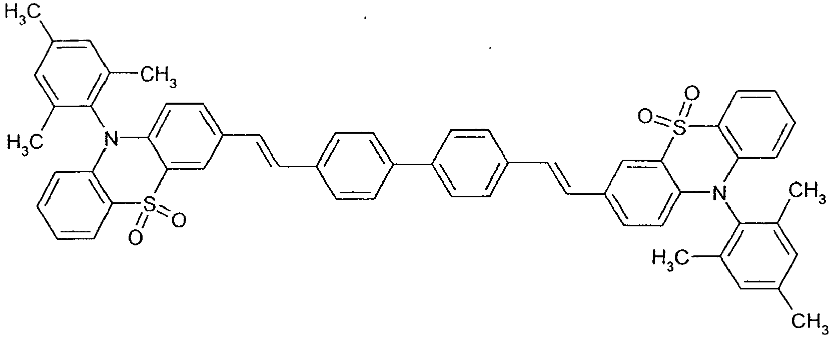

Zu einer Lösung von 1.63 g (3.58 mmol) [4'-(Diethoxyphosphorylmethyl)-biphenyl-4-yl-methyl]-phosphonsäurediethylester (US 3,984,399, Beispiel 2, Spalte 29, Zeile 58 - 66) in 40 ml über Molekularsieb getrocknetem Dimethylsulfoxid wurden unter Stickstoff bei Raumtemperatur unter Rühren 0.93 g (8.1 mmol) Kalium-tert.-butylat und dann 2.70 g (7.16 mmol) 10-Mesityl-5,5-dioxo-phenothiazin-3-carbaldehyd (Beispiel 35 b) gegeben. Nach 4 h Rühren bei Raumtemperatur wurde die Reaktionslösung mit 200 ml Methanol verdünnt und 1 h nachgerührt. Der Niederschlag wurde abfiltriert, mit 100 ml Methanol gewaschen, in 300 ml Methanol wieder aufgerührt, erneut filtriert und bei 35 °C im Vakuum getrocknet. Das Rohprodukt (2.90 g) wurde zweimal aus Dimethylformamid und einmal aus Chlorbenzol umkristallisiert. Das Kristallisat wurde abgesaugt, nacheinander mit Chlorbenzol und Ethanol gewaschen, abgesaugt und bei 80 °C im Vakuum getrocknet. Nach dem Entfernen von Lösungsmittelresten im Hochvakuum (2 x 10⁻⁵ mbar) bei 250 °C wurden 0.98 g (30 % d. Th.) analysenreine leuchtendgelbe Mikrokristalle mit einem Schmp. > 350 °C. erhalten, deren Lösung in Chloroform bei λ = 456, 485 (S), 522 (S) nm fluoreszierte.

### Beispiel 38: 10-(2-Thiophenyl)-phenothiazin-5,5'-dioxid

### a) 10-(2-Thiophenyl)-phenothiazin

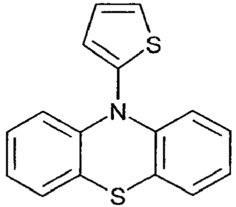

8,4 g (42 mmol) Phenothiazin, 9,1 g (42 mmol) 98 %iges 2-Jodthiophen, 7,0 g (51 mmol) Kaliumcarbonat und 0,18 g (2,8 mmol) Kupferpulver wurden auf 140 °C erhitzt und 7 Stunden bei dieser Temperatur gerührt. Die Reaktionsschmelze wurde abgekühlt, mit 200 ml Ethanol versetzt, unter Rückfluss erhitzt und heiß filtriert. Der Rückstand wurde zweimal mit Ethanol gewaschen. Die Ethanollösung wurde eingeengt und nach Säulenchromatographie (Laufmittel Essigester/Cyclohexan 2:5) wurden 3,13 g Wertprodukt erhalten, das gleich für den nachfolgenden Reaktionsschritt b) benutzt wurde.

### b) 10-(2-Thiophenyl)-phenothiazin-5,5'-dioxid

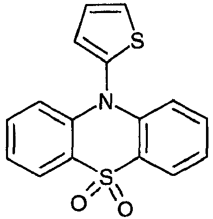

3,13 g (11,1 mmol) 10-(2-Thiophenyl)-phenothiazin aus Reaktionschritt a) wurden in 100 ml Methylenchlorid gelöst. Bei 0-5°C wurden 5,52 g (22,4 mmol) 77 %ige m-Chlorperbenzoe-säure portionsweise zugegeben. Die Reaktionslösung wurde 2 Stunden bei Raumtemperatur gerührt. Das Reaktionsgemisch wurde eingeengt und der Rückstand in 250 ml Methylenchlorid gelöst. Die organische Phase wurde dreimal mit je 20 ml 10 %iger Kalilauge und fünfmal mit je 50 ml VE-Wasser gewaschen. Nach Säulenchromatographie (Laufmittel Methylenchlorid) wurde das Wertprodukt erhalten, das durch Kristallisation aus Essigsäure und anschließend Sublimation weiter gereinigt wurde. Es wurden 0,34 g (10 % d. Th.) farblose Mikrokristalle mit einem Schmelzpunkt von 230-234 °C erhalten.

### Beispiel 39: 2,5-(1,4-Dimethoxyphenylen)-10,10'-bis(phenothiazin)-5,5'-dioxid

### a) 2,5-(1,4-Dimethoxyphenylen)-10,10'-bis(phenothiazin)

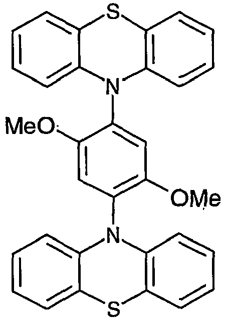

Die Herstellung erfolgte nach K. Okada et al., J. Am. Chem. Soc. 1996, 118, 3047-3048.

0,8 g (4,0 mmol) Phenothiazin, 0,88 g (2,2 mmol) 97%-iges 1,4- Dijod-2,5-dimethoxybenzol, 0,66 g (4,8 mmol) Kaliumcarbonat und 0,016g (0,25 mmol) aktiviertes Kupferpulver wurden in DMSO (10 ml) auf 140 °C erhitzt und 15 Stunden bei dieser Temperatur gerührt. Nach Abkühlung der Reaktionsmischung auf Raumtemperatur wurde 30 ml Methylenchlorid zugegeben und die Suspension filtriert. Die Methylenchloridlösung wurde dreimal mit je 25 ml 0,1 molarer Salzsäure gewaschen, getrocknet und eingeengt. Es wurden 0,99 g der Zielverbindung als farbloser Feststoff erhalten.

### b) 2,5-(1,4-Dimethoxyphenylen)-10,10'-bis(phenothiazin)-5,5'-dioxid

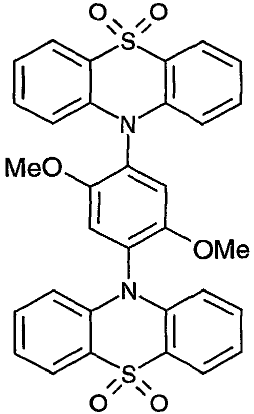

0,43 g (0,81 mmol) 2,5-(1,4-Dimethoxyphenylen)-10,10'-bis(phenothiazin) aus Reaktionsschritt a) wurden in 15 ml Methylenchlorid gelöst. Nach 1 Stunde Rühren bei Raumtemperatur wurden 0,56 g (3,2 mmol) 77 %ige m-Chlorperbenzoesäure portionsweise zugegeben.

Die Reaktionslösung wurde 24 Stunden bei Raumtemperatur gerührt, wobei ein Niederschlag ausfiel. Die Suspension wurde eingeengt und der Rückstand in vollentsalztem Wasser mit 10 ml 10 %iger Natronlauge auf pH 12 gestellt, 1 Stunde bei 70 °C gerührt und anschließend filtriert. Der Rückstand wurde mit heißem Wasser gewaschen und bei 80 °C im Vakuum getrocknet. Es wurden 0,20 g (41 %) Wertprodukt erhalten.

### Beispiel 40: Herstellung einer OLED

Eine OLED mit dem folgenden Schichtaufbau wurde durch Aufdampfen der einzelnen Schichten hergestellt: ITO / NPD (40 nm) / 3,7-Bis(2,2-diphenylvinyl)-10-methylphenothiazin-5,5-dioxid (Beispiel 13) (40 nm) / BCP (6 nm) / LiF (1 nm) / Aluminium.
NPD = 4,4'-Bis[N-(1-naphthyl)-N-phenyl-amino]biphenyl
BCP = 2,9-Dimethyl-4,7-diphenyl-1,10-phenanthrolin
LiF = Lithiumfluorid

Die OLED wies einen maximalen photometrischen Wirkungsgrad von 2,7 cd/A bei einer Spannung von 4 V und eine maximale Leuchtdichte von 5750 cd/m² bei 9 V auf. Das Intensitätsmaximum der Elektrolumineszenz lag bei 455 nm.

### Beispiel 41: Herstellung einer OLED (mPTO2 als Loch- und Excitonblocker)

Eine OLED mit dem folgenden Schichtaufbau wurde durch Aufdampfen der einzelnen Schichten hergestellt: ITO / DPBIC (28 nm) / Ester : CN-PMIC (33 %) (20 nm) / mPTO2 (Beispiel 5) (5 nm) / TPBI (40 nm) / LiF (0.7 nm) / Aluminium. DPBIC (Lochleiter und Excitonblocker) =

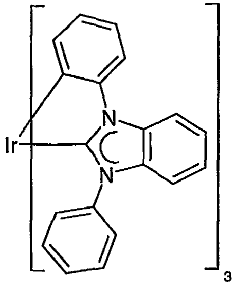

Ester (Matrix für Emitter) =

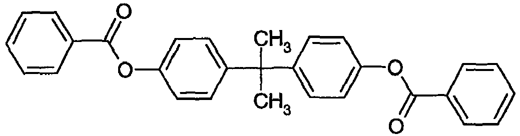

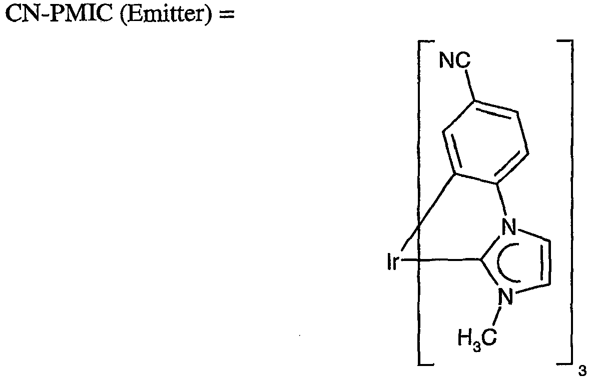

### mPTO2 (Loch- und Excitonblocker) = 1,3-Phenylen-10,10'-bis(phenothiazin-5,5-dioxid)

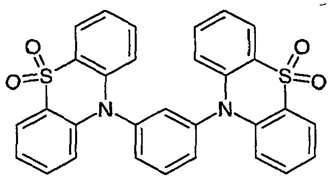

### TPBI (Elektronenleiter) = 1,3,5-Phenylen-2,2',2"-tris(1-phenylbenzimidazol) LiF = Lithiumfluorid

Die oben beschriebene OLED wies die folgenden elektrooptischen Daten auf:

| | |
|---|---|
| Emissionsmaximum | 464 nm |
| Farbkoordinaten CIE (x, y) | 0,15; 0,15 |
| Maximaler Photometrischer Wirkungsgrad | 15,1 cd/A |
| Photometrischer Wirkungsgrad bei 100 cd/m² (600 cd/m²) | 14,4 cd/A (12,1 cd/A) |
| Leistungseffizienz | 11,1 lm/W |
| Externe Quantenausbeute | 12,1 % |
| Maximale Leuchtdichte | 2000 cd/m² |

## Patentansprüche

1. Verwendung von Verbindungen der Formel I 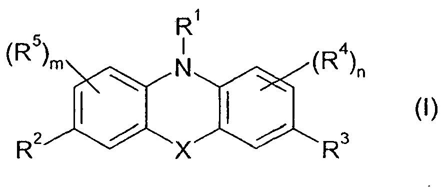 worin bedeuten:
X SO₂, SO;
R¹ H, Alkyl, Aryl, Heteroaryl; oder 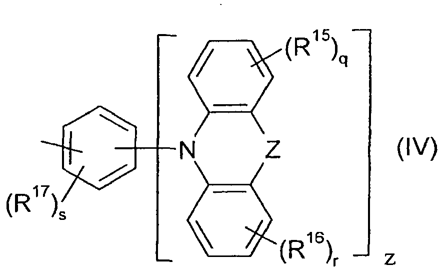 oder 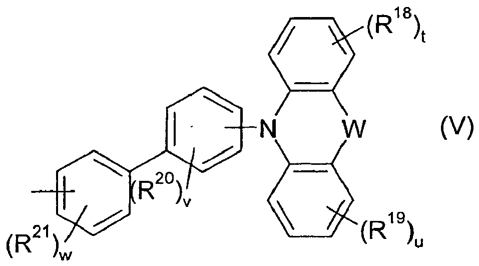
R², R³ unabhängig voneinander H, Alkyl, Aryl oder COR³¹, wobei R² und R³ nicht gleichzeitig H bedeuten oder
mindestens einer der Reste R² oder R³ ist ein Rest der Formel II 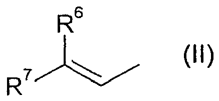 wobei der weitere Rest R² oder R³ die gleiche Bedeutung aufweist oder H, Alkyl, Aryl bedeutet, bevorzugt ist der weitere Rest R² oder R³ ebenfalls ein Rest der Formel (II),
oder
einer der Reste R² oder R³ ist ein Rest der Formel III 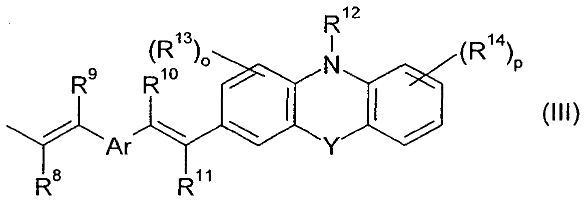 wobei der weitere Rest R² oder R³ H, Alkyl, Aryl bedeutet;
R⁴, R⁵, R¹³, R¹⁴, R¹⁵, R¹⁶, R¹⁸, R¹⁹, R²⁰, R²¹ unabhängig voneinander Alkyl, Aryl, Alkenyl, Alkinyl oder Alkylaryl;
R¹⁷ Halogen, Alkoxy, Alkyl, Aryl, Alkenyl, Alkinyl oder Alkylaryl;
m, n 0, 1, 2 oder 3, bevorzugt 0 oder 1, besonders bevorzugt 0;
o, p, q, r, s, t, u, v, w 0, 1, 2, 3 oder 4, bevorzugt 0 oder 1, besonders bevorzugt 0;
z 1 oder 2;
R⁶, R⁷ unabhängig voneinander H, Alkyl, Aryl, Alkenyl, Alkinyl, Alkylaryl, Alkoxy oder Aryloxy;
R⁸, R⁹, R¹⁰, R¹¹ unabhängig voneinander H, Alkyl, Aryl, Alkenyl, Alkinyl oder Alkylaryl;
R¹² H, Alkyl, Aryl;
R²², R²³ unabhängig voneinander H, Alkyl, Aryl; oder R²² und R²³ bilden gemeinsam einen cyclischen Rest;
Y, Z, W S, SO₂; O, SO und
Ar Arylen;
R³¹ H, Alkyl, Aryl;
als Loch- und Excitonenblocker in organischen Leuchtdioden.

2. Verwendung nach Anspruch 1, **dadurch gekennzeichnet, dass** ein Loch- oder Excitonenblocker der Formel I eingesetzt wird, worin die Symbole und Reste die folgenden Bedeutungen aufweisen:
X SO₂;
R¹ H, Methyl, Ethyl, unsubstituiertes Phenyl oder 4-Alkoxyphenyl; oder mit einer OH, OC(O)Ph, OCH₂Ph oder N-Phenyl-benzimidazolyl-Gruppe, zwei F-Resten, drei CH₃-Gruppen oder substituiertes Phenyl, 4-Methoxyphenyl, Thienyl oder Pyridyl oder 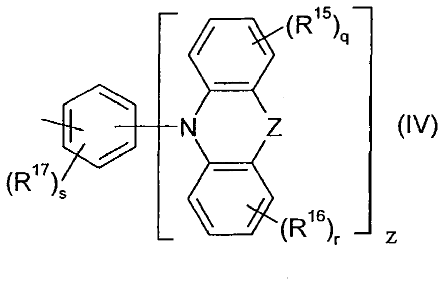 oder 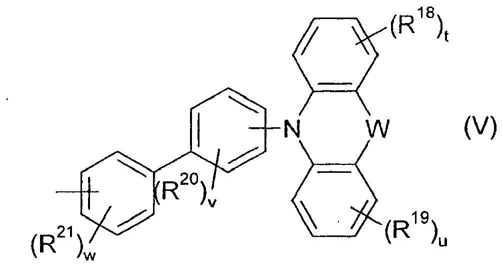
R², R³ H, unsubstituiertes Phenyl, 1-Naphthyl, 2-Naphthyl oder CHO, wobei mindestens einer der Reste R² oder R³ unsubstituiertes Phenyl, 1-Naphthyl oder 2-Naphthyl bedeutet,
oder
R² und R³ bedeuten 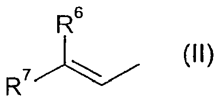 oder
R² bedeutet H und R³ bedeutet 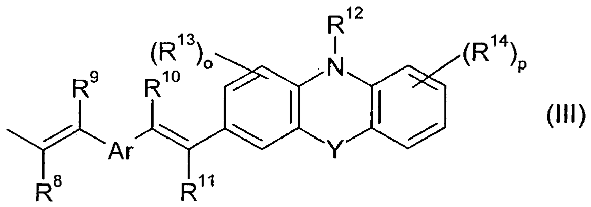
m, n, o, p, q, r, s, t, u, v und w 0, 1 oder 2, bevorzugt 0;
z 1 oder 2;
R⁶, R⁷ unsubstituiertes Phenyl;
R⁸, R⁹, R¹⁰, R¹¹ H;
R¹² H, Methyl, Ethyl, unsubstituiertes Phenyl oder 4-Alkoxyphenyl oder mit drei CH₃-Gruppen substituiertes Phenyl;
R¹⁷ Alkyl, Alkoxy, bevorzugt Methoxy;
R²², R²³ Aryl;
Y, Z, W S oder SO₂; und
Ar Phenylen, Naphthylen oder Biphenylen.

3. Verwendung nach Anspruch 1 oder 2, **dadurch gekennzeichnet, dass** die Loch- oder Excitonenblocker der Formel I ausgewählt sind aus der Gruppe bestehend aus Verbindungen der Formeln Ia, Id, Ie, Ig und Ih 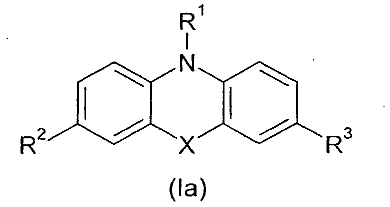 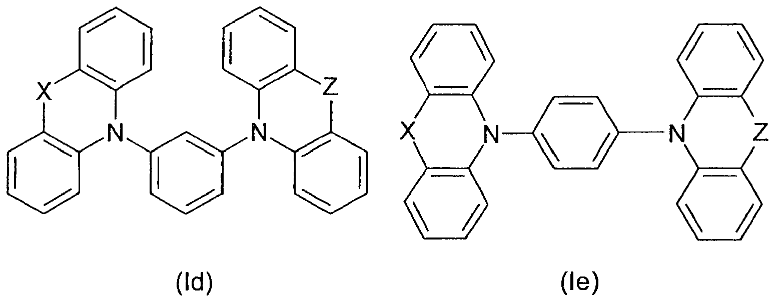 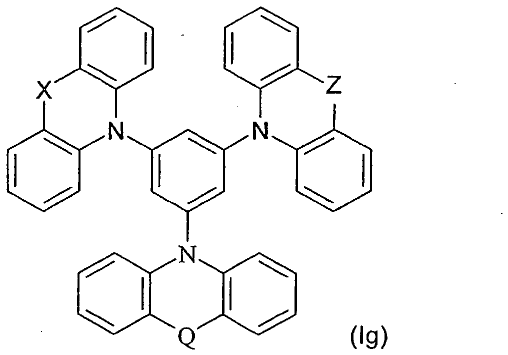 und 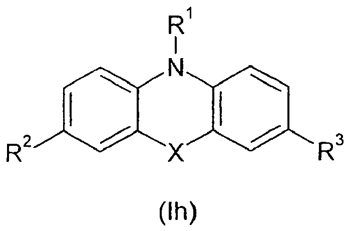 worin bedeuten:
X, Z, Q SO₂; und
R¹ H, Methyl, Ethyl oder unsubstituiertes Phenyl oder mit einer OH, OC(O)Ph, OCH₂Ph oder N-Phenyl-benzimidazolyl-Gruppe, drei CH₃-Gruppen oder zwei F-Resten substituiertes Phenyl, 4-Methoxyphenyl, Thienyl oder Pyridyl;
R², R³ H oder unsubstituiertes Phenyl, wobei mindestens R² oder R³ unsubstituiertes Phenyl bedeutet, oder für den Fall, dass R¹ mit einer OH, OC(O)Ph, OCH₂Ph oder N-Phenyl-benzimidazolyl-Gruppe, drei CH₃-Gruppen oder zwei F-Resten substituiertes Phenyl, 4-Methoxyphenyl, Thienyl oder Pyridyl bedeutet, bedeuten
R² H;
R³ H oder CHO; bevorzugt H.

4. Organische Leuchtdiode umfassend eine Blockschicht für Löcher und Excitonen, **dadurch gekennzeichnet, dass** die Blockschicht für Löcher und Excitonen mindestens eine Verbindung der Formel I gemäß einem der Ansprüche 1, 2 oder 3 enthält.

5. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Digitalkameras, Fahrzeugen sowie Zielanzeigen an Bussen und Bahnen enthaltend eine organische Leuchtdiode gemäß Anspruch 4.

6. Phenothiazin-S,S-dioxid-Derivate ausgewählt aus der Gruppe bestehend aus Verbindungen der Formeln XIV, XVI, XVII, XVIII, XIX, XX und XXII 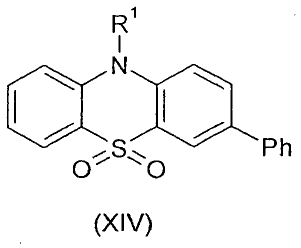 und 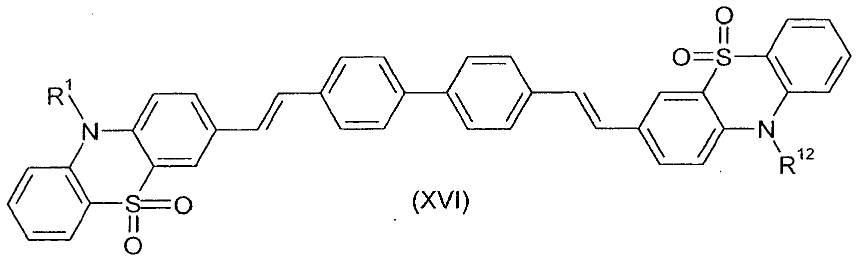 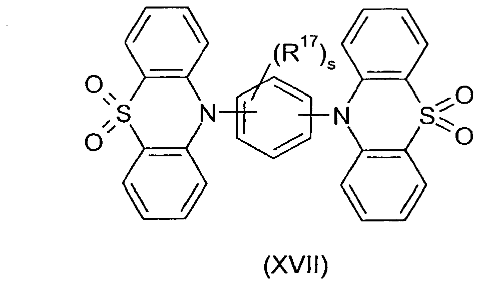 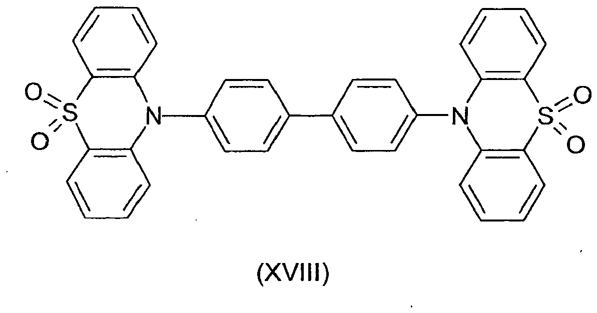 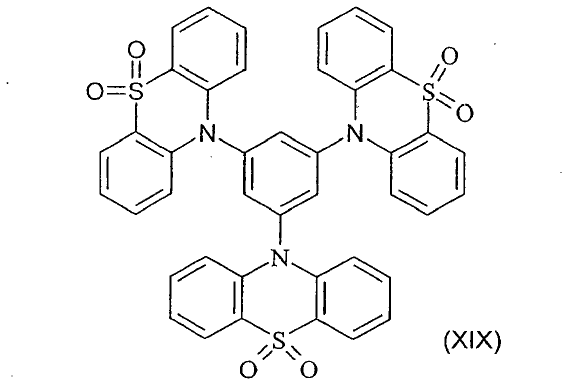 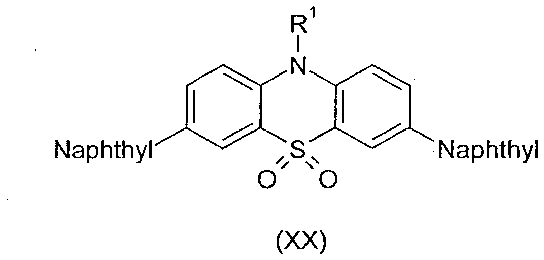 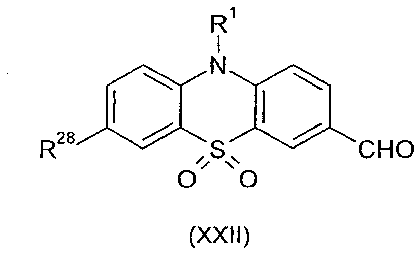 worin bedeuten:
R¹, R¹² H, Me, Et oder unsubstituiertes Phenyl;
R¹⁷ Methoxy;
s 0 oder 2, wobei die Substituenten im Falle von s = 2 bevorzugt in der 2- und der 5-Position angeordnet sind;
Ph unsubstituiertes Phenyl;
Naphthyl 1- oder 2-Naphthyl; und
R²⁸ H oder-CHO.

7. Phenothiazin-S-oxid-Derivate der Formel XXX 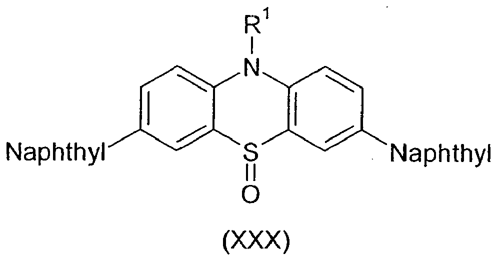 worin bedeuten:
R¹ H, Me, Et oder unsubstituiertes Phenyl; und
Naphthyl 1- oder 2-Naphthyl.

8. Phenothiazin-S,S-dioxid-Derivate ausgewählt aus der Gruppe bestehend aus Verbindungen der Formeln XXXI und XXXII 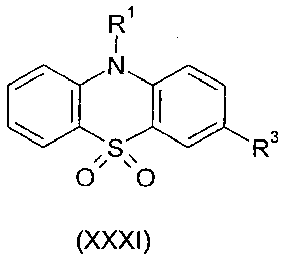 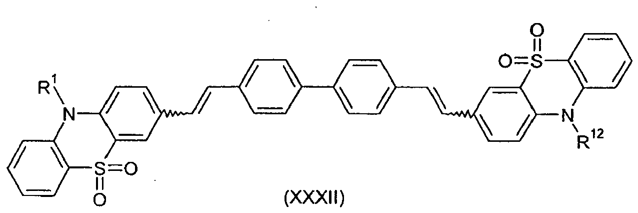 worin bedeuten
R¹ mit einer OH, OC(O)Ph, OCH₂Ph oder N-Phenyl-benzimidazolyl-Gruppe, drei CH₃-Gruppen oder zwei F-Resten substituiertes Phenyl, 4-Methoxyphenyl, 2-Thienyl oder Pyridyl,
R³ CHO,
R¹² ein mit drei CH₃-Gruppen substituiertes Phenyl.

9. Verwendung von Phenothiazin-S,S-dioxid-Derivaten gemäß Anspruch 6 oder 8 und Phenothiazin-S-oxid-Derivaten gemäß Anspruch 7 in organischen Leuchtdioden.

10. OLED enthaltend mindestens ein Phenothiazin-S,S-dioxid-Derivat gemäß Anspruch 6 oder 8 und/oder mindestens ein Phenothiazin-S-oxid-Derivat nach Anspruch 7.

11. Licht-emittierende Schicht enthaltend mindestens ein Phenothiazin.-S,S-dioxid-Derivat gemäß Anspruch 6 oder 8 und/oder mindestens ein Phenothiazin-S-oxid-Derivat nach Anspruch 7.

12. OLED enthaltend eine Licht-emittierende Schicht gemäß Anspruch 11.

13. Loch- und Excitonenblockschicht enthaltend mindestens ein Phenothiazin-S,S-dioxid-Derivat gemäß den Formeln XIV, XVII, XIX, XXII gemäß Anspruch 6 und/oder gemäß den Formeln XXXI, XXXIII gemäß Anpruch 8.

14. OLED enthaltend eine Loch- und Excitonenblockschicht gemäß Anspruch 13.

## Claims

1. The use of compounds of the formula I 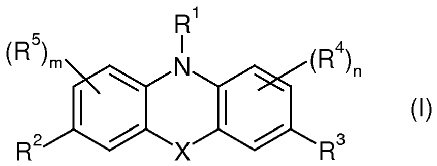 in which:
X is SO₂, SO;
R¹ is H, alkyl, aryl, heteroaryl; or 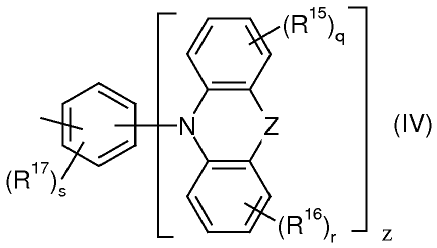 or 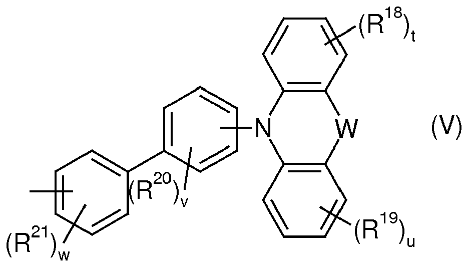
R², R³ are each independently H, alkyl, aryl or COR³¹, where R² and R³ are not at the same time H,
or
at least one of the R² or R³ radicals is a radical of the formula II 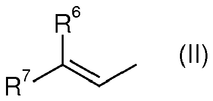 where the further R² or R³ radical has the same definition or is H, alkyl, aryl; the further R² or R³ radical is preferably likewise a radical of the formula II,
or
one of the R² or R³ radicals is a radical of the formula III 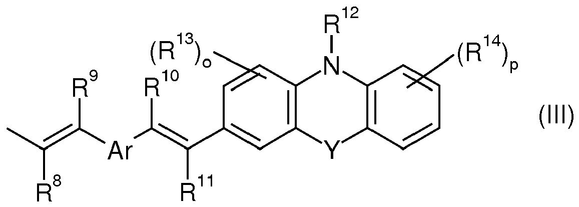 where the further R² or R³ radical is H, alkyl, aryl;
R⁴, R⁵, R¹³, R¹⁴, R¹⁵, R¹⁶, R¹⁸, R¹⁹, R²⁰, R²¹ are each independently alkyl, aryl, alkenyl, alkynyl or alkylaryl;
R¹⁷ is halogen, alkoxy, alkyl, aryl, alkenyl, alkynyl or alkylaryl;
m, n are each 0, 1, 2 or 3, preferably 0 or 1, more preferably 0;
o, p, q, r, s, t, u, v, w are each 0, 1, 2, 3 or 4, preferably 0 or 1, more preferably 0;
z is 1 or 2;
R⁶, R⁷ are each independently H, alkyl, aryl, alkenyl, alkynyl, alkylaryl, alkoxy or aryloxy;
R⁸, R⁹, R¹⁰, R¹¹ are each independently H, alkyl, aryl, alkenyl, alkynyl or alkylaryl;
R¹² is H, alkyl or aryl;
R²², R²³ are each independently H, alkyl, aryl; or R²² and R²³ together form a cyclic radical;
Y, Z, W are each S, SO₂; O, SO; and
Ar is arylene;
R³¹ is H, alkyl or aryl;
as hole and exciton blockers in organic light-emitting diodes.

2. The use according to claim 1, wherein a hole or exciton blocker of the formula I is used, in which the symbols and radicals are each defined as follows:
X is SO₂;
R¹ is H, methyl, ethyl, unsubstituted phenyl or 4-alkoxyphenyl; or phenyl, 4-methoxyphenyl, thienyl or pyridyl substituted by one OH, OC(O)Ph, OCH₂Ph or N-phenylbenzimidazolyl group, two F radicals, three CH₃ groups or or 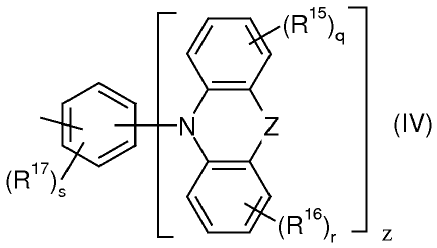 or 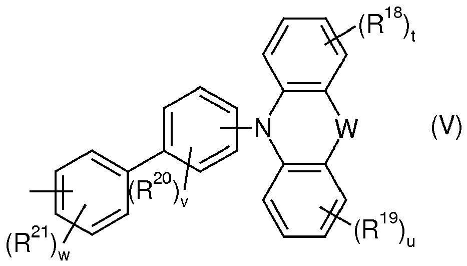
R², R³ are each H, unsubstituted phenyl, 1-naphthyl, 2-naphthyl or CHO, where at least one of the R² or R³ radicals is unsubstituted phenyl, 1-naphthyl or 2-naphthyl,
or
R² and R³ are each 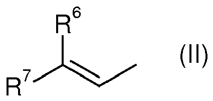 or
R² is H and R³ is 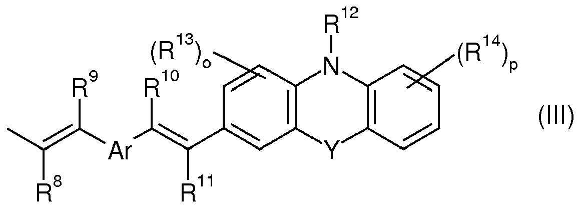
m, n, o, p, q, r, s, t, u, v and w are each 0, 1 or 2, preferably 0;
z is 1 or 2;
R⁶, R⁷ are each unsubstituted phenyl;
R⁸, R⁹, R¹⁰, R¹¹ are each H;
R¹² is H, methyl, ethyl, unsubstituted phenyl or 4-alkoxyphenyl or phenyl substituted by three CH₃ groups;
R¹⁷ is alkyl, alkoxy, preferably methoxy;
R²², R²³ are each aryl;
Y, Z, W are each S or SO₂; and
Ar is phenylene, naphthylene or biphenylene.

3. The use according to claim 1 or 2, wherein the hole or exciton blockers of the formula I are selected from the group consisting of compounds of the formulae Ia, Id, Ie, Ig and Ih 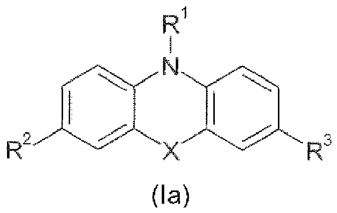 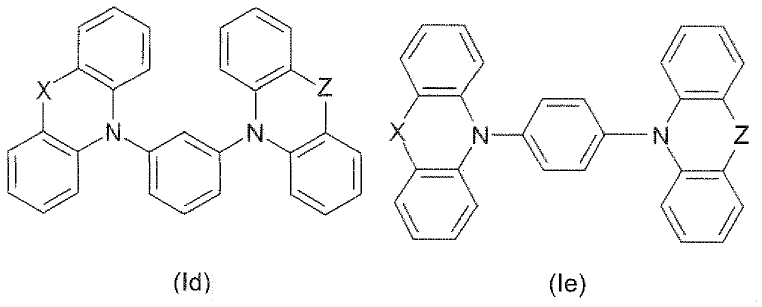 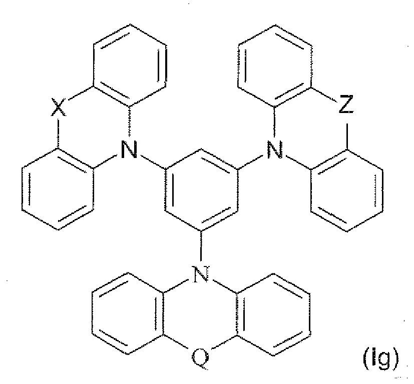 and 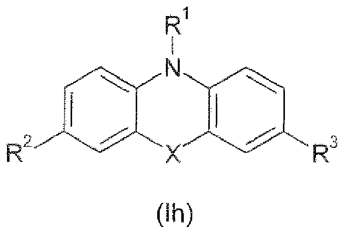 in which:
X, Z, Q are each SO₂; and
R¹ is H, methyl, ethyl or unsubstituted phenyl, or phenyl, 4-methoxyphenyl, thienyl or pyridyl substituted by one OH, OC(O)Ph, OCH₂Ph or N-phenylbenzimidazolyl group, three CH₃ groups or two F radicals;
R², R³ are each H or unsubstituted phenyl, where at least R² or R³ is unsubstituted phenyl, or, in the case that R¹ is phenyl, 4-methoxyphenyl, thienyl or pyridyl substituted by one OH, OC(O)Ph, OCH₂Ph or N-phenylbenzimidazolyl group, three CH₃ groups or two F radicals,
R² is H;
R³ is H or CHO; preferably H.

4. An organic light-emitting diode comprising a blocking layer for holes and excitons, wherein the blocking layer for holes and excitons comprises at least one compound of the formula I according to any of claims 1, 2 and 3.

5. A device selected from the group consisting of stationary visual display units such as visual display units of computers, televisions, visual display units in printers, kitchen appliances and advertising panels, illuminations, information panels and mobile visual display units such as visual display units in mobile telephones, laptops, digital cameras, vehicles and destination displays on buses and trains, comprising an organic light-emitting diode according to claim 4.

6. A phenothiazine S,S-dioxide derivative selected from the group consisting of compounds of the formulae XIV, XVI, XVII, XVIII, XIX, XX and XXII 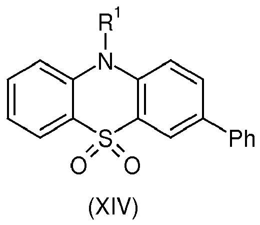 and 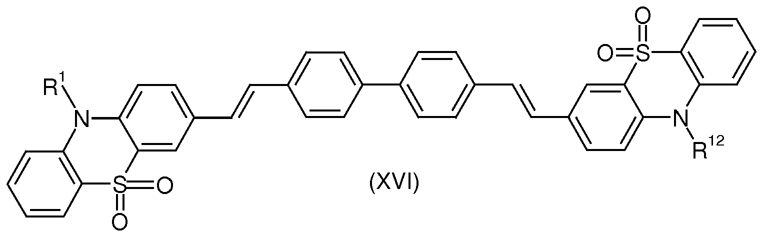 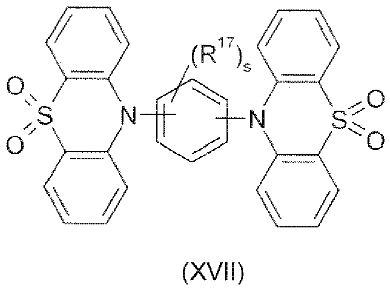 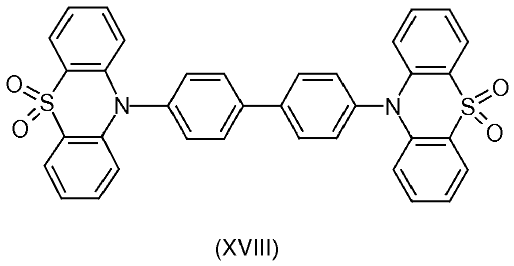 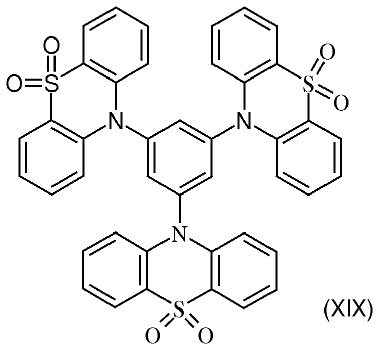 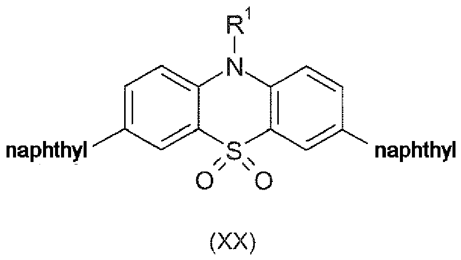 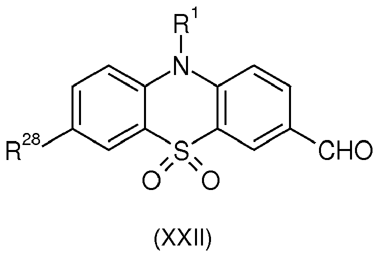 in which:
R¹, R¹² are each H, Me, Et or unsubstituted phenyl;
R¹⁷ is methoxy;
s is 0 or 2, where the substituents, in the case that s = 2, are preferably arranged in the 2- and the 5-position;
Ph is unsubstituted phenyl;
naphthyl is 1- or 2-naphthyl; and
R²⁸ is H or -CHO.

7. A phenothiazine S-oxide derivative of the formula XXX 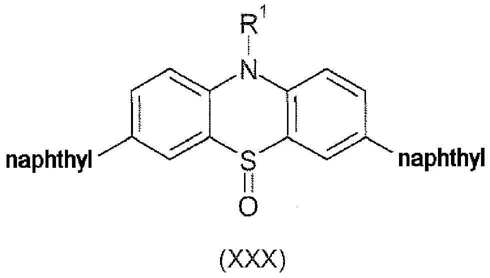 in which:
R¹ is H, Me, Et or unsubstituted phenyl; and
naphthyl is 1- or 2-naphthyl.

8. A phenothiazine S,S-dioxide derivative selected from the group consisting of compounds of the formulae XXXI and XXXII 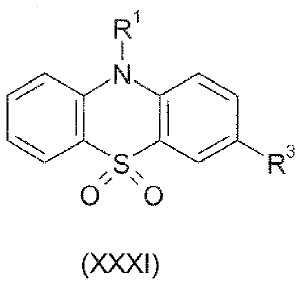 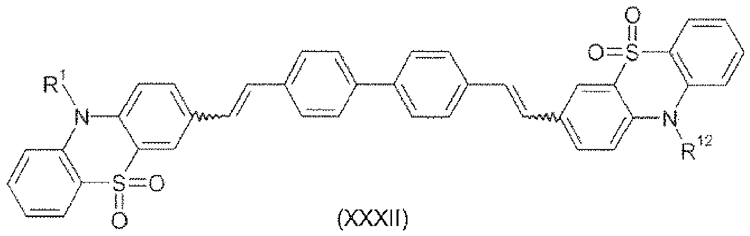 in which
R¹ is phenyl, 4-methoxyphenyl, 2-thienyl or pyridyl substituted by one OH, OC(O)Ph, OCH₂Ph or N-phenylbenzimidazolyl group, three CH₃ groups or two F radicals,
R³ is CHO,
R¹² is a phenyl substituted by three CH₃ groups.

9. The use of phenothiazine S,S-dioxide derivatives according to claim 6 or 8 and phenothiazine S-oxide derivatives according to claim 7 in organic light-emitting diodes.

10. An OLED comprising at least one phenothiazine S,S-dioxide derivative according to claim 6 or 8 and/or at least one phenothiazine S-oxide derivative according to claim 7.

11. A light-emitting layer comprising at least one phenothiazine S,S-dioxide derivative according to claim 6 or 8 and/or at least one phenothiazine S-oxide derivative according to claim 7.

12. An OLED comprising a light-emitting layer according to claim 11.

13. A hole- and exciton-blocking layer comprising at least one phenothiazine S,S-dioxide derivative of the formulae XIV, XVII, XIX, XXII according to claim 6 and/or of the formulae XXXI, XXXIII according to claim 8.

14. An OLED comprising a hole- and exciton-blocking layer according to claim 13.

## Revendications

1. Utilisation de composés de formule I 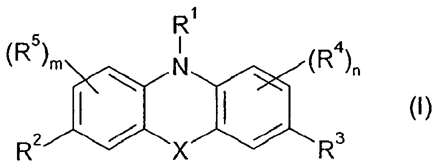 où :
X signifie SO₂, SO ;
R¹ signifie H, alkyle, aryle, hétéroaryle ; ou 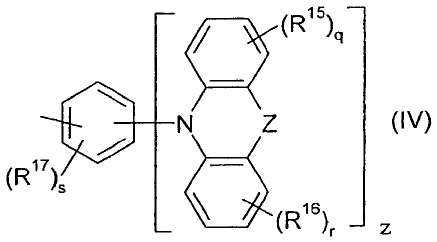 ou 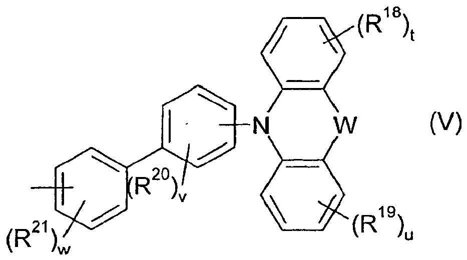
R², R³ signifient, indépendamment l'un de l'autre, H, alkyle, aryle ou COR³¹, R² et R³ ne signifiant pas simultanément H ou
au moins un des radicaux R² ou R³ représente un radical de formule II 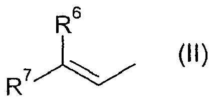 l'autre radical R² ou R³ présentant la même signification ou signifiant H, alkyle, aryle, de préférence l'autre radical R² ou R³ représente également un radical de formule (II), ou
un des radicaux R² ou R³ représente un radical de formule III 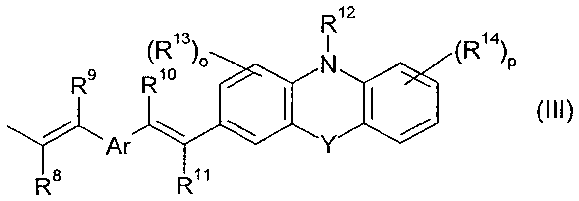 l'autre radical R² ou R³ signifiant H, alkyle, aryle ;
R⁴, R⁵, R¹³, R¹⁴, R¹⁵, R¹⁶, R¹⁸, R¹⁹, R²⁰, R²¹ signifient, indépendamment les uns des autres, alkyle, aryle, alcényle, alcynyle ou alkylaryle ;
R¹⁷ signifie halogène, alcoxy, alkyle, aryle, alcényle, alcynyle ou alkylaryle ;
m, n valent 0, 1, 2 ou 3, de préférence 0 ou 1, de manière particulièrement préférée 0 ;
o, p, q, r, s, t, u, v, w valent 0, 1, 2, 3 ou 4, de préférence 0 ou 1, de manière particulièrement préférée 0 ;
z vaut 1 ou 2 ;
R⁶, R⁷ signifient, indépendamment l'un de l'autre, H, alkyle, aryle, alcényle, alcynyle, alkylaryle, alcoxy ou aryloxy ;
R⁸, R¹⁰, R⁹,R¹¹ signifient, indépendamment les uns des autres, H, alkyle, aryle, alcényle, alcynyle ou alkylaryle ;
R¹² signifie H, alkyle, aryle ;
R²², R²³ signifient, indépendamment l'un de l'autre H, alkyle, aryle ;
ou R²² et R²³ forment ensemble un radical cyclique ;
Y, Z, W signifient S, SO₂, 0, SO et
Ar signifie arylène ;
R³¹ signifie H, alkyle, aryle ;
comme bloquant de trous et d'excitons dans les diodes électroluminescentes organiques.

2. Utilisation selon la revendication 1, **caractérisée en ce qu'**on utilise un bloquant de trous ou d'excitons de formule I, dans laquelle les symboles et les radicaux présentent des significations suivantes :
X signifie SO₂ ;
R¹ signifie H, méthyle, éthyle, phényle non substitué ou 4-alcoxyphényle ; ou phényle, 4-méthoxyphényle, thiényle ou pyridyle substitué par OH, OC(O)Ph, OCH₂Ph ou un groupe N-phénylbenzimidazolyle, deux radicaux F, trois groupes CH₃ ou ou 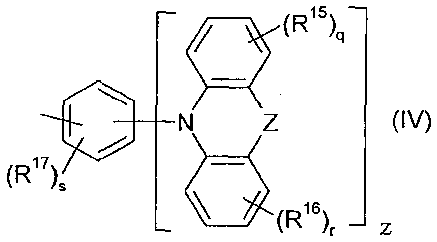 ou 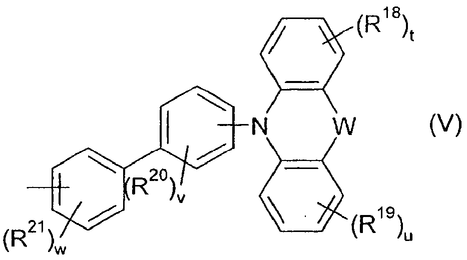
R², R³ signifient H, phényle non substitué, 1-naphtyle, 2-naphtyle ou CHO, au moins un des radicaux R² ou R³ signifiant phényle non substitué, 1-naphtyle ou 2-naphtyle, ou R² et R³ signifient 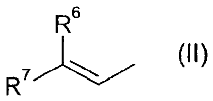 ou
R² signifie H et R³ signifie 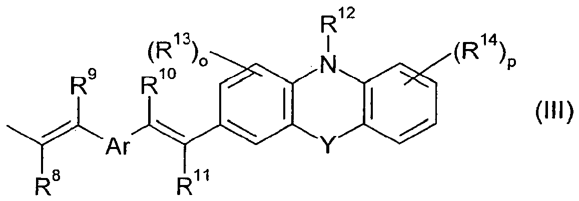
m, n, o, p, q, r, s, t, u, v et w valent 0, 1 ou 2, de préférence 0 ;
z vaut 1 ou 2 ;
R⁶, R⁷ signifient phényle non substitué ;
R⁸, R⁹, R¹⁰, R¹¹ signifient H ;
R¹² signifie H, méthyle, éthyle, phényle non substitué ou 4-alcoxyphényle ou phényle substitué par trois groupes CH₃ ;
R¹⁷ signifie alkyle, alcoxy, de préférence méthoxy ;
R²², R²³ signifient aryle ;
Y, Z, W signifient S ou SO₂ ; et
Ar signifie phénylène, naphtylène ou biphénylène.

3. Utilisation selon la revendication 1 ou 2, **caractérisée en ce que** les bloquants de trous ou d'excitons de formule I sont choisis dans le groupe constitué par les composés des formules Ia, Id, Ie, Ig et Ih 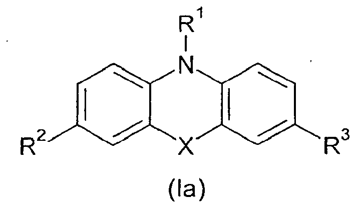 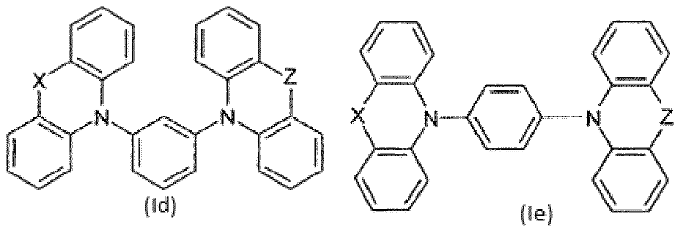 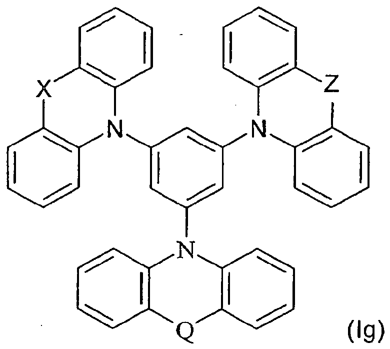 et 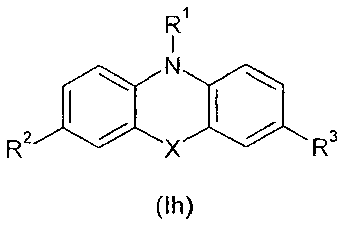 où :
X, Z, Q signifient SO₂ ; et
R¹ signifie H, méthyle, éthyle ou phényle non substitué ou phényle, 4-méthoxyphényle, thiényle ou pyridyle substitué par OH, OC(O)Ph, OCH₂Ph ou un groupe N-phénylbenzimidazolyle, trois groupes CH₃ ou deux radicaux F;
R², R³ signifient H ou phényle non substitué, au moins R² ou R³ signifiant phényle non substitué, ou, pour le cas où R¹ signifie phényle, 4-méthoxyphényle, thiényle ou pyridyle substitué par OH, OC(O)Ph, OCH₂Ph ou un groupe N-phénylbenzimidazolyle, trois groupes CH₃ ou deux radicaux F,
R² signifie H ;
R³ signifie H ou CHO ; de préférence H.

4. Diode électroluminescente organique comprenant une couche de blocage de trous et d'excitons, **caractérisée en ce que** la couche de blocage de trous et d'excitons contient au moins un composé de formule I selon l'une quelconque des revendications 1, 2 ou 3.

5. Dispositif choisi dans le groupe constitué par les écrans stationnaires, tels que les écrans d'ordinateurs, de téléviseurs, les écrans dans les imprimantes, les appareils de cuisine ainsi que les panneaux publicitaires, les éclairages, les panneaux indicateurs et les écrans mobiles, tels que les écrans dans les téléphones portables, les ordinateurs portables, les caméras digitales, les véhicules ainsi que les affichages de destination sur les bus et les trains contenant une diode lumineuse organique selon la revendication 4.

6. Dérivés de phénothiazine-S,S-dioxyde choisis dans le groupe constitué par les composés des formules XIV, XVI, XVII, XVIII, XIX, XX et XXII 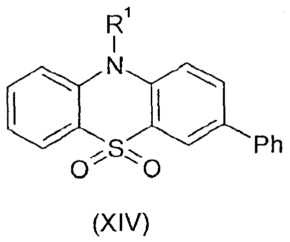 et 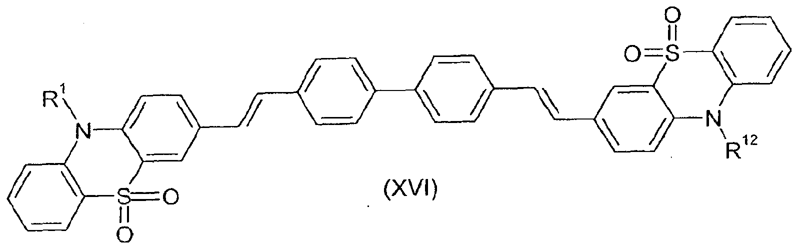 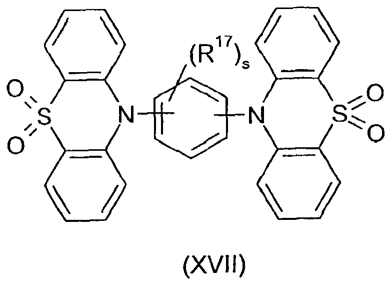 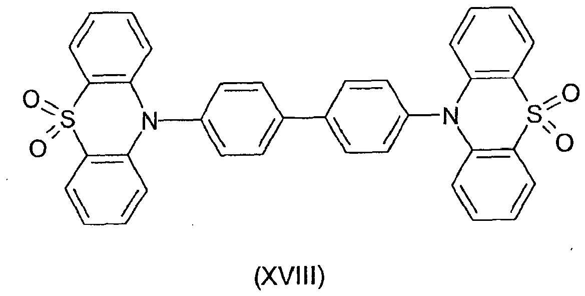 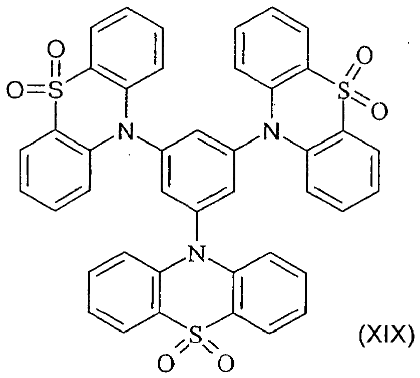 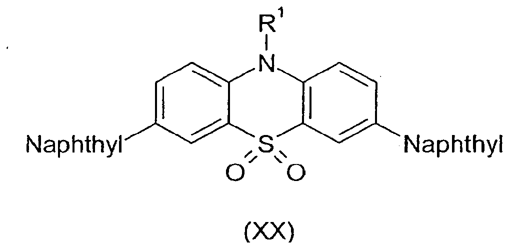 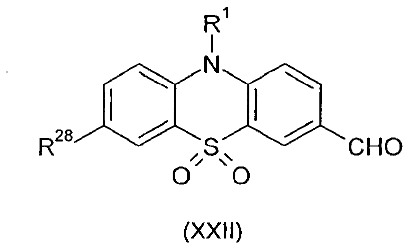 où :
R¹, R¹² signifient H, Me, Et ou phényle non substitué ;
R¹⁷ signifie méthoxy ;
s vaut 0 ou 2, les substituants étant disposés, dans le cas de s = 2, de préférence en position 2 et 5 ;
Ph signifie phényle non substitué ;
Naphthyl signifie 1-naphtyle ou 2-naphtyle ; et
R²⁸ signifie H ou -CHO.

7. Dérivés de phénothiazine-S-oxyde de formule XXX 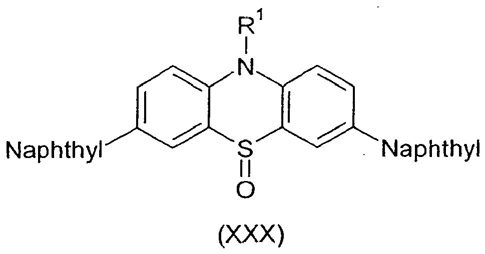 où :
R¹ signifie H, Me, Et ou phényle non substitué ; et
Naphthyl signifie 1-naphtyle ou 2-naphtyle.

8. Dérivés de phénothiazine-S,S-dioxyde choisis dans le groupe constitué par des composés des formules XXXI et XXXII 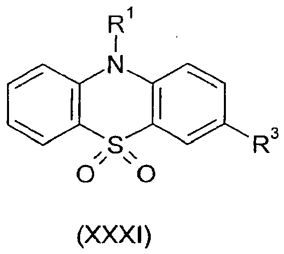 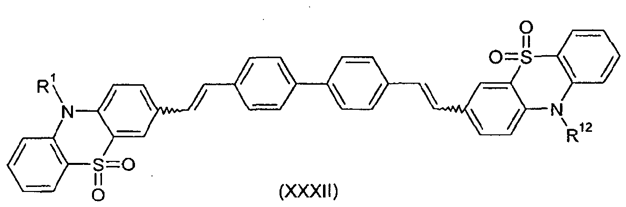 où
R¹ signifie phényle, 4-méthoxyphényle, 2-thiényle ou pyridyle substitué par OH, OC(O)Ph, OCH₂Ph ou un groupe N-phénylbenzimidazolyle, trois groupes CH₃ ou deux radicaux F,
R³ signifie CHO,
R¹² signifie phényle substitué par trois groupes CH₃.

9. Utilisation de dérivés de phénothiazine-S,S-dioxyde selon la revendication 6 ou 8 et dérivés de phénothiazine-S-oxyde selon la revendication 7 dans des diodes électroluminescentes organiques.

10. DELO contenant au moins un dérivé de phénothiazine-S,S-dioxyde selon la revendication 6 ou 8 et/ou au moins un dérivé de phénothiazine-S-oxyde selon la revendication 7.

11. Couche émettrice de lumière contenant au moins un dérivé de phénothiazine-S,S-dioxyde selon la revendication 6 ou 8 et/ou au moins un dérivé de phénothiazine-S-oxyde selon la revendication 7.

12. DELO contenant au moins une couche émettrice de lumière selon la revendication 11.

13. Couche de blocage de trous et d'excitons contenant au moins un dérivé de phénothiazine-S,S-dioxyde selon les revendications XIV, XVII, XIX, XXII selon la revendication 6 et/ou selon les formules XXXI, XXXIII selon la revendication 8.

14. DELO contenant au moins une couche de blocage de trous et d'excitons selon la revendication 13.
